(19) Europäisches Patentamt
European Patent Office
Office européen des brevets

(11) Veröffentlichungsnummer: **0 119 954**
**B1**

(12) **EUROPÄISCHE PATENTSCHRIFT**

(45) Veröffentlichungstag der Patentschrift: **14.03.90**

(51) Int. Cl.⁵: **C 07 D 223/16,** A 61 K 37/02

(21) Anmeldenummer: **84810071.5**

(22) Anmeldetag: **06.02.84**

# (54) Gewisse 3-Amino-1-benzazepin-2-on-1-alkansäuren, Verfahren zu ihrer Herstellung, pharmazeutische Präparate enthaltend diese Verbindungen, sowie ihre therapeutische Verwendung.

(30) Priorität: **10.02.83 US 465695**

(43) Veröffentlichungstag der Anmeldung:
**26.09.84 Patentblatt 84/39**

(45) Bekanntmachung des Hinweises auf die Patenterteilung:
**14.03.90 Patentblatt 90/11**

(84) Benannte Vertragsstaaten:
**AT BE CH DE FR GB IT LI LU NL SE**

(56) Entgegenhaltungen:
**EP-A-0 046 291**
**EP-A-0 072 352**
**EP-A-0 107 095**

**Chemical Abstracts, Band 100, 1984, Seite 19, Ref.Nr.167718k; Columbus, Ohio, US. W H Parsons et al.: "Benzolactams. A new class of converting enzymeinhibitors." & Biochem. Biophys. Res. Commun. 1983, 117(1), 108-13**

(73) Patentinhaber: **CIBA-GEIGY AG**
**Klybeckstrasse 141**
**CH-4002 Basel (CH)**

(72) Erfinder: **Watthey, Jeffrey W.H., Dr.**
**61 Deepwood Drive**
**Chappaqua New York 10514 (US)**

**EP 0 119 954 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft neue 3-Amino-1-benzazepin-2-on-1-alkansäuren der allgemeinen Formel

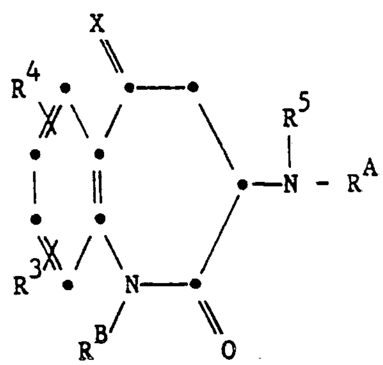

(I),

worin $R^A$ und $R^B$ Reste der Formel

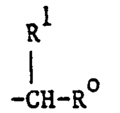
bzw.
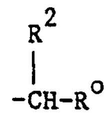

darstellen, worin $R^o$ Carboxy oder ein funktionell modifiziertes Carboxy darstellt; $R^1$ Wasserstoff, Niederalkyl, Amino-niederalkyl, Aryl, Aryl-niederalkyl, Cycloalkyl, Cycloalkyl-niederalkyl, Acylamino-niederalkyl, Mono- oder Di-niederalkylamino-niederalkyl, Niederalkylthio-niederalkyl, Carboxy-niederalkyl, verestertes Carboxy-niederalkyl, Carbamoyl-niederalkyl, N-substituiertes Carbamoyl-niederalkyl, Hydroxy-niederalkyl, verethertes oder acyliertes Hydroxy-niederalkyl, Aryloxy-niederalkyl, Aryl-(thio-, sulfinyl- oder sulfonyl-)-niederalkyl, Aryl-N-niederalkylaminoniederalkyl oder Arylamino-niederalkyl bedeutet; $R^2$ für Wasserstoff oder Niederalkyl steht; $R^3$ und $R^4$ unabhängig voneinander Wasserstoff, Niederalkyl, Niederalkoxy, Niederalkanoyloxy, Hydroxy, Halogen oder Trifluormethyl bedeuten, oder $R^3$ und $R^4$ gemeinsam für Niederalkylendioxy stehen; $R^5$ Wasserstoff oder Niederalkyl bedeutet; und X Oxo, zwei Wasserstoffatome oder Hydroxy oder acyliertes Hydroxy zusammen mit einem Wasserstoffatom bedeutet; und worin der carbocyclische Ring auch Hexahydro oder 6,7,8,9-Tetrahydro sein kann; mit der Massgabe, dass, wenn $R^1$ Wasserstoff, Niederalkyl, Amino-niederalkyl, Aryl, Aryl-niederalkyl, Cycloalkyl oder Cycloalkyl-niederalkyl ist, X acyliertes Hydroxy zusammen mit einem Wasserstoffatom bedeutet; Salze, insbesondere pharmazeutisch verträgliche Salze davon Stereoisomere von allen diesen Verbindungen; Verfahren zu ihrer Herstellung, pharmazeutische Präparate enthaltend diese Verbindungen, sowie ihre therapeutische Verwendung.

Eine funktionell modifizierte Carboxylgruppe $R^o$ ist beispielsweise eine veresterte Carboxylgruppe oder eine Carbamoylgruppe, die gegebenenfalls N-substituiert ist. Insbesondere bedeuten einer oder beide Reste $R^o$ unabhängig voneinander Carboxy, verestertes Carboxy, Carbamoyl oder substituiertes Carbamoyl.

Eine Gruppe $R^o$ wird durch $COR^6$ im Radikal $R^A$ und durch $COR^7$ Radikal $R^B$ dargestellt.

Eine Carboxlygruppe $R^o$ wird durch $COR^6$ worin $R^6$ Hydroxy ist, oder durch $COR^7$, worin $R^7$ Hydroxy ist, dargestellt.

Eine veresterte Carboxylgruppe $R^o$ ist insbesondere eine solche worin der veresternde Rest gegebenenfalls substituiertes Niederalkyl oder gegebenenfalls substituiertes Phthalidyl darstellt, und wird dargestellt durch $COR^6$ oder $COR^7$, worin einer der Reste $R^6$ und $R^7$ oder beide Reste Niederalkoxy bedeuten, welches gegebenenfalls durch Amino, Mono- oder Di-niederalkylamino, Carboxy, z.B. in der α-Stellung, Niederalkoxycarbonyl, z.B. in der α-Stellung, Aryl, Hydroxy, Niederalkanoyloxy, Niederalkoxy, Bicycloalkoxycarbonyl substituiert sein kann, oder für 3-Phthalidoxy oder (Niederalkyl, Niederalkoxy, Halogen)-substituiertes 3-Phthalidoxy stehen kann.

Eine gegebenenfalls N-substituierte Carbamoylgruppe $R^o$ wird durch $COR^6$ oder $COR^7$ dargestellt, worin einer der Reste $R^6$ und $R^7$ oder beide insbesondere Amino; Niederalkylamino; Di-niederalkylamino; Di-niederalkylamino, worin beide Alkylgruppen durch eine Kohlenstoff-Kohlenstoff-Bindung verbunden sind und gemeinsam mit dem Aminostickstoff einen 5-, 6- oder 7-gliedrigen heterocyclischen Ring, z.B. Pyrrolidino, Piperidino oder Perhydroazepino, bilden; (Amino oder Acylamino)-substituiertes Niederalkylamino; α-(Carboxy oder Niederalkoxycarbonyl)-substituiertes Niederalkylamino oder Arylsubstituiertes Niederalkylamino, worin Aryl vorzugsweise Phenyl oder Indolyl bedeutet und das an dem α-Kohlenstoffatom durch Carboxy oder Niederalkoxycarbonyl substituiert sein kann, bedeuten.

Weiter können die Begriffe verestertes Carboxy und N-substituiertes Carbamoyl, sofern sie in der Definition von $R^1$ als verestertes Carboxy-niederalkyl und N-substituiertes Carbamoyl-niederalkyl erscheinen, irgendeine der für diese Begriffe oben angegebenen Bedeutungen haben.

Die vorliegend verwendeten allgemeinen Definitionen besitzen im Bereich der Erfindung die folgenden Bedeutungen:

Die Bezeichnung "nieder", die vorstehend und nachfolgend in Verbindung mit organischen Resten bzw. Verbindungen verwendet wird, definiert solche mit bis zu und einschliesslich 7, vorzugsweise bis zu und einschliesslich 4 und insbesondere 1 oder 2 Kohlenstoffatomen.

Niederalkyl enthält 1—7 Kohlenstoffatome, vorzugsweise 1 bis 4 Kohlenstoffatome und bedeutet z.B. Ethyl, Propyl, Butyl oder inbesondere Methyl.

Aryl bedeutet einen carbocyclischen oder heterocyclischen aromatischen Rest, vorzugsweise unsubstituiertes oder durch Niederalkyl, Niederalkoxy, Niederalkylendioxy, Niederalkanoyloxy, Hydroxy, Halogen oder Trifluormethyl mono- oder disubstituiertes Phenyl; Indolyl, vorzugsweise 3-Indolyl; oder durch Niederalkyl, Niederalkoxy, Niederalkylendioxy, Niederalkanoyloxy, Hydroxy, Halogen oder Trifluormethyl substituiertes Indolyl, vorzugsweise 3-Indolyl.

Cycloalkyl bedeutet einen gesättigten cyclischen Kohlenwasserstoffrest, der vorzugsweise 3 bis 8 Kohlenstoffatome enthält und beispielsweise Cyclopentyl oder Cyclohexyl ist.

Arylniederalkyl bedeutet vorzugsweise Benzyl, 1- oder 2-Phenylethyl, 1-, 2- oder 3-Phenylpropyl, 1-, 2-, 3- oder 4-Phenylbutyl, worin der Phenylring unsubstituiert oder durch Niederalkyl, Hydroxy, Niederalkoxy, Niederalkylendioxy, Niederalkanoyloxy, Halogen oder Tri-fluormethyl mono- oder disustituiert ist; und auch Indolylmethyl, vorzugsweise 3-Indolylmethyl, 1-oder 2-Indolylethyl, vorzugsweise 2-(3-Indolyl)ethyl.

Cycloalkyl-niederalkyl bedeutet vorzugsweise loder 2-(Cyclopentyl oder Cyclohexyl)ethyl, 1-, 2- oder 3-(Cyclopentyl oder Cyclohexyl)propyl oder 1-, 2-, 3- oder 4-(Cyclopentyl oder Cyclohexyl)butyl.

Niederalkoxy enthält vorzugsweise 1 bis 4 Kohlenstoffatome und ist beispielsweise Methoxy, Propoxy, Isopropoxy oder insbesondere Ethoxy.

Mono-niederalkylamino enthält vorzugsweise 1 bis 4 Kohlenstoffatome in dem Niederalkylteil und ist beispielsweise N-Methylamino, N-Propylamino oder vorzugsweise N-Ethylamino. Di-niederalkylamino enthält vorzugsweise 1 bis 4 Kohlenstoffatome in jedem Niederalkylteil und bedeutet z.B. N,N-Dimethylamino, N-Methyl-N-ethylamino und vorzugsweise N,N-Diethylamino.

Niederalkylthio-niederalkyl bedeutet vorzugsweise (Methyl, Ethyl oder Propyl)-thio-(methyl, ethyl, propyl oder butyl), vorzugsweise 2-(Methylthio)ethyl.

Niederalkanoyloxy bedeutet vorzugsweise Acetoxy, Propionyloxy oder Pivaloyloxy.

Alkylendioxy bedeutet vorzugsweise Ethylendioxy und vorzugsweise Methylendioxy.

Aryl-niederalkoxy bedeutet vorzugsweise Benzyloxy, Benzyloxy substituiert durch Methyl, Methoxy oder Chlor, und Pyridylmethoxy.

Carboxy-niederkalkoxy bedeutet vorzugsweise 1-Carboxyethoxy.

Niederalkoxycarbonyl-niederalkoxy bedeutet vorzugsweise 1-(Ethoxycarbonyl)ethoxy.

Amino-niederalkoxy, Mono-niederalkylamino-niederalkoxy und Di-niederalkylamino-niederalkoxy bedeuten vorzugsweise Aminoethoxy, Ethylaminoethoxy bzw. Diethylaminoethoxy.

Niederalkanoyloxyalkoxy bedeutet vorzugsweise Pivaloyloxymethoxy.

Bicycloalkyloxycarbonyl-niederalkoxy bedeutet vorzugsweise Bicyclo [2.2.1] heptyloxycarbonylniederalkoxy, unsustituiert oder substituiert durch Niederalkyl, vorzugsweise Bicyclo [2.2.1] heptyloxycarbonyl-methoxy, z.B. Bornyloxycarbonyl-methoxy.

Amino-niederalkyl und ω-Amino-niederalkyl bedeuten vorzugsweise Amino(ethyl, propyl oder butyl) bzw. ω-Amino(ethyl, propyl oder butyl).

Halogen bedeutet vorzugsweise Chlor, kann jedoch auch Brom, Fluor oder Iod sein.

Acyliertes Hydroxy bedeutet vorzugsweise Niederalkanoyloxy, z.B.

Acetyloxy, Benzoyloxy, am Phenylring durch Niederalkyl, Halogen oder Niederalkoxy, z.B. wie durch Methyl, Chlor bzw. Methoxy sustituiertes Benzoyloxy, oder Nicotinoyloxy.

Verethertes Hydroxy bedeutet vorzugsweise Niederalkoxy, z.B. Methoxy, Ethoxy oder t-Butoxy, oder Benzyloxy.

Aryloxy bedeutet vorzugsweise Phenoxy oder Phenoxy substituiert durch Niederalkyl, Niederalkoxy oder Halogen, wie Methyl, Methoxy bzw. Chlor.

Arylthio bedeutet vorzugsweise phenylthio oder durch Niederalkyl, Niederalkoxy oder Halogen, wie Methyl, Methoxy bzw. Chlor substituiertes Phenylthio.

Arylsulfinyl und -sulfonyl bedeutet vorzugsweise Phenylsulfinyl und Phenylsulfonyl oder durch Niederalkyl, Niederalkoxy oder Halogen, wie Methyl, Methoxy bzw. Chlor substituiertes Phenylsulfinyl und Phenylsulfonyl.

Arylamino bedeutet vorzugsweise Anilino; Aryl-N-niederalkylamino bedeutet vorzugsweise N-Methyl-anilino.

Acylamino-niederalkyl und ω-Acylamino-niederalkyl bedeuten vorzugsweise Acylamino(ethyl, propyl oder butyl) bzw. ω-Acylamino(ethyl, propyl oder butyl).

Acylamino bedeutet Niederalkanoylamino, Niederalkoxycarbonylamino, Cycloalkylcarbonylamino, Cycloalkyloxycarbonylamino, Cycloalkylniederalkoxycarbonylamino; auch Aryl-niederalkanoylamino, Aryl-niederalkoxycarbonylamino, Arylsulfonamido, worin Aryl vorzugsweise Phenyl oder vorzugsweise durch Niederalkyl, Niederalkoxy oder Halogen substituiertes Phenyl bedeutet. Acylamino steht weiter für Aroylamino, worin Aroyl vorzugsweise Benzoyl oder vorzugsweise durch Niederalkyl, Niederalkoxy oder Halogen substituiertes Benzoyl bedeutet; oder für Nicotinoyl.

Aryl-niederalkoxycarbonylamino bedeutet vorzugsweise Aryl-methoxycarbonylamino, insbesondere Benzyloxycarbonylamino, Benzyloxycarbonylamino, welches am Phenylring durch Niederalkyl, Niederalkoxy oder Halogen, wie Methyl, Methoxy bzw. Chlor substituiert ist, oder Pyridylmethoxycarbonylamino.

Die Salze von Verbindungen der Formel I sind von solchen Verbindungen abgeleitet, die salzbildende Eigenschaften haben. Bevorzugt sind die pharmazeutisch verträglichen Salze.

Pharmazeutisch verträgliche Salze sind vorzugsweise Metall- oder Ammoniumsalze der Verbindungen der Formel I, worin $R^o$ Carboxy bedeutet, oder der Formeln IA—IC, worin $COR^6$ und/oder $COR^7$ Carboxy bedeuten, insbesondere Alkali- oder Erdalkalimetallsalze, z.B. Natrium-, Kalium-, Magnesium- oder Calciumsalze; oder vorteilhafterweise leicht kristallisierende Ammoniumsalze, abgeleitet von Ammoniak oder organischen Aminen, wie Mono-, Di- oder Tri-nieder(alkyl, cycloalkyl oder hydroxyalkyl)aminen, Niederalkylendiaminen oder (Hydroxyniederalkyl oder Arylniederalkyl)alkylammonium-Basen, z.B. Methylamin, Diethylamin, Triethylamin, Dicyclohexylamin, Triethanolamin, Ethylendiamin, Tris-(hydroxymethyl)aminomethan oder Benzyltrimethylammoniumhydroxid. Diese Verbindungen der Formel I bilden Säureadditionssalze, bei denen es sich vorzugsweise um solche von therapeutisch verträglichen anorganischen oder organischen Säuren handelt, wie starken Mineralsäuren, z.B. Halogenwasserstoffsäuren, z.B. Chlorwasserstoff- oder Bromwasserstoffsäure; Schwefel-, Phosphor-, Salpeter- oder Perchlorsäure; aliphatischen oder aromatischen Carbon- oder Sulfonsäuren, z.B. Ameisen-, Essig-, Propion-, Bernstein-, Glykol-, Milch-, Aepfel-, Wein-, Glucon-, Citronen-, Ascorbin-, Malein-, Fumar-, Hydroxymalein-, Brenztrauben-, Phenylessig-, Benzoe-, 4-Aminobenzoe-, Anthranil-, 4-Hydroxybenzoe-, Salicyl-, 4-Aminosalicyl-, Pamoa-, Nicotin-, Methansulfon-, Ethansulfon-, Hydroxyethansulfon-, Benzolsulfon-, p-Toluolsulfon-, Naphthalinsulfon-, Sulfanil- oder Cyclohexylsulfaminsäure.

Die Verbindungen der Formel I zeigen wertvolle pharmakologische Eigenschaften, z.B. kardiovaskuläre Effekte, welche sie unter anderem aufgrund ihrer Hemmwirkung auf die Bildung von Angiotensin II hervorrufen. Diese Wirkung entsteht durch selektive Hemmung des Enzyms, welches das Angiotensin in Säugern umwandelt. Die Verbindungen sind daher nützlich für die Behandlung von Krankheiten, welche auf die Hemmung des Enzyms, das Angiotensin in Säugern, einschliesslich Menschen, umwandelt, ansprechen.

Die Verbindungen der Erfindung zeigen in erster Linie hypotensive/antihypertensive Wirkungen und beeinflussen die Herztätigkeit, u.a. aufgrund ihrer hemmenden wirkung auf das Enzym, welches das Angiotensin umwandelt. Diese Eigenschaften können durch in-vitro-oder in-vivo-Tierversuche, vorzugsweise an Säugetieren, z.B. Ratten, Katzen, Hunden oder deren isolierten Organen, als Testobjekte nachgewiesen werden. Die Tiere können entweder normotensiv oder hypertensiv, z.B. genetisch spontan hypertensive Ratten, oder renal hypertensive Ratten oder Hunde, und Hunde, denen Natrium entzogen ist, sein. Die Verbindungen können den Versuchstieren enteral oder parenteral, vorzugsweise oral oder intravenös, z.B. in Gelatine-Kapseln oder in Form von Stärke enthaltenden Suspensionen bzw. wässrigen Lösungen, verabreicht werden. Die verwendete Dosis kann in einem Bereich von ungefähr zwischen 0,01 und 100 mg/kg/Tag, vorzugsweise zwischen ungefähr 0,05 und 50 mg/kg/Tag, insbesondere zwischen ungefähr 0,1 und 25 mg/kg/Tag liegen.

Die blutdrucksenkende Wirkung in vivo wird entweder direkt mit einem Katheter, der in die femurale Arterie des Versuchstieres eingeführt ist, oder indirekt durch Sphygmomanometrie am Rattenschwanz, und einem Uebertragungsinstrument registriert. Der Blutdruck wird vor und nach der Verabreichung des Wirkstoffes in mm Hg bestimmt.

So sind die antihypertensiven Wirkungen in spontan hypertensiven Ratten durch indirekte Messung des systolischen Blutdrucks nachweisbar. Wache Ratten werden einzeln in sehr engen Käfigen in eine leicht geheizte Kammer gebracht. Ein Puls-Sensor wird innerhalb einer aufblasbaren Manschette am Schwanz jeder Ratte angebracht. Die Manschette wird periodisch aufgeblasen, um die Schwanz-Arterie zu schliessen. Der Druck in der Manschette wird kontinuierlich reduziert, und der systolische Druck entspricht demjenigen Druck in der Manschette, bei dem die Pulswellen wieder auftreten. Nach der Registrierung von Kontrollwerten des Blutdrucks und der Herzfrequenz werden die Testverbindungen einmal täglich an vier aufeinander folgenden Tagen oral verabreicht. Zusätzliche Blutdruckmessungen werden üblicherweise 2,0, 4,0 und 23,5 Stunden nach einer täglichen Dosis vorgenommen. Die werte werden mit denjenigen von Ratten, welche lediglich mit der Trägersubstanz behandelt worden sind, verglichen.

Die Verbindungen der Erfindung zeigen nach intravenöser oder oraler Verabreichung eine hemmende wirkung gegen die durch Angiotensin I hervorgerufene Blutdrucksteigerung auch an normotensiven Ratten. Das Angiotensin I wird durch das genannte Umwandlungsenzym zu der stark blutdrucksteigernden Substanz Angiotensin II hydrolysiert. Die Hemmung des genannten Enzyms blockiert die Bildung von Angiotensin II aus Angiotensin I. Auf diese Weise wird die durch Angiotensin I hervorgerufene Blutdrucksteigerung abgeschwächt.

Der entsprechende Test in vivo wird mit männlichen normotensiven Ratten, welche mit dem Natriumsalz der 5-Ethyl-5-(1-methyl-propyl)-2-thiobarbitursäure narkotisiert sind, durchgeführt. Eine femurale Arterie und eine Wadenvene werden mit je einer Hohlnadel für die direkte Messung des Blutdrucks und für die intravenöse Verabreichung des Angiotensins I und einer erfindungsgemässen Verbindung versehen. Nach der Stabilisierung des basalen Blutdrucks wird die Druckänderung nach drei, in 5-minütigen Intervallen, i.v. durchgeführten Reizungen mit 333 ng/kg Angiotensin I bestimmt. Solche Druckänderungen werden wieder 5, 10, 15, 30 und 60 Minuten nach intravenöser Verabreichung oder 1, 2, 3

und 4 Stunden nach peroraler Verabreichung der zu prüfenden Verbindung bestimmt und mit den Anfangswerten verglichen. Jede durch die erfindunsgemässen Verbindungen hervorgerufene festgestellte Abnahme vom Ansprechen des Blutdrucks ist ein Hinweis auf die Hemmung des Enzyms, welches das Angiotensin I umwandelt.

Die Hemmung des Enzyms, welches das Angiotensin umwandelt, durch die Verbindungen dieser Erfindung in vitro kann analog zu Biochim. Biophys. Acta *293*, 451 (1973) nachgewiesen werden. Gemäss dieser. Methode werden die genannten Verbindungen in ungefähr 1 mMol-Konzentrationen in phosphatpuffer gelöst. Zu 100 µl von Lösungen der Testverbindung in Phosphatpuffer, welche man auf die gewünschte Konzentration verdünnt, gibt man zuerst 100 µl von 5 millimolarem Hippuryl-histidyl-leucin in Phosphatpuffer und dann 50 µl des Angiotensin-umwandelnden Enzyms. Dieses Enzym wird aus Lungen von erwachsenen männlichen Kaninchen in Tris-puffer, der Kalium- und Magnesiumchlorid und auch Rohrzucker enthält, hergestellt. Die genannten Lösungen werden 30 Minuten bei 37°C inkubiert. Durch Hinzufügen von 0,75 ml einer 0,6-normalen wässerigen Natriumhydroxidlösung wird die Reaktion dann abgebrochen. Man gibt bei Zimmertemperatur 100 µl einer 0,2 %igen Lösung von o-Phthalaldehyd in Methanol und 10 Minuten danach 100 µl 6n-Chlorwasserstoffsäure dazu. Diese proben werden in einem Spektrophotometer bei 360 nm mit Wasser verglichen, und ihre optische Dichte wird bestimmt. Diese Werte werden durch einen Konversionsfaktor einer Standard-Kurve angepasst, wobei man die während der genannten Inkubation von 30 Minuten gebildete Histidyl-leucin-Menge in Nanomolen erhält. Die Ergebnisse werden, um den $IC_{50}$-Wert zu bestimmen, gegenüber den Wirkstoffkonzentrationen graphisch als Kurve dargestellt. Der $IC_{50}$-Wert bedeutet diejenige Wirkstoff-Konzentration, welche die Hälfte der Aktivität einer Kontrollprobe, die keinen Wirkstoff enthält, ergibt.

Das Angiotensin-umwandelnde Enzym nimmt nicht nur teil an der Umwandlung von Angiotensin I in Angiotensin II, sondern spielt auch eine Rolle bei der Kontrolle der Bradykinin- und Aldesteron-Spiegel. Der Einfluss der Verbindungen dieser Erfindung auf diese Faktoren kann auch zu dem antihypertensiven und herzwirksamen Effekt der Verbindungen dieser Erfindung beitragen.

Wegen der vorgenannten vorteilhaften Eigenschaften sind die erfindungsgemässen Verbindungen als spezifische therapeutische Mittel für Säuger, inklusive Menschen, sehr nützlich.

Dementsprechend sind die Verbindungen der Erfindung wertvolle antihypertensive Mittel, insbesondere für die Herabsetzung von hohem Blutdruck (ohne Rücksicht auf die Aetiologie) und/oder bei Herzerkrankungen wie Herzinsuffizienz, und/oder anderen Zuständen, die mit Oedemen oder Ascites einhergehen. Sie können auch zur Herstellung von anderen wertvollen Produkten, insbesondere von entsprechenden pharmazeutischen Präparaten verwendet werden.

Erfindungsgemäss können eine oder beide der Carboxylgruppen der Dicarbonsäuren, d.h. der Verbindungen der Formel IA, IB oder IC, worin $R^6$ und $R^7$ Hydroxy sind, in Form der funktionellen Derivate als Ester oder Amide vorliegen. Diese funktionellen Derivate sind vorzugsweise die Mono- oder Bisniederalkylester, z.B. Methyl-, Ethyl-, n- oder i-propyl-, Butyl- oder Benzylester; die Mono- oder Bis-amide, die mono- oder di-N-alkylierten Amide, z.B. Mono- oder Diethylamide; die mono- oder di-substituierten Niederalkylester, z.B. die ω-(Amino, Mono- oder Dimethylamino, Carboxy oder Carbethoxy)-(ethyl, propyl oder butyl)-ester. Stark bevorzugte funktionelle Derivate sind die Monoester der Formel IA, z.B. solche, worin einer der Reste $R^6$ und $R^7$ Hydroxy und der andere Niederalkoxy bedeutet.

Derivate der erfindungsgemässen Verbindungen, die Wirkstoff-Vorstufen darstellen, z.B. jegliche pharmazeutisch verträglichen Ester und Amide der erfindungsgemässen Mono- oder Dicarbonsäuren, die durch Solvolyse oder unter physiologischen Bedingungen in die vorliegenden Carbonsäuren übergeführt werden können, z.B. die vorstehend genannten Ester und Amide, stellen einen speziellen Gegenstand der Erfindung dar.

Diese Ester sind vorzugsweise z.B. geradkettige oder verzweigte unsubstituierte oder in geeigneter Weise substituierte Niederalkylester, wie die Pivaloyloxymethyl-, Bornyloxycarbonylmethyl-, Benzyl-, Pyridylmethyl-, α-Carboxyethyl- oder in geeigneter Weise veresterte α-Carboxyethylester, und dergleichen.

Diese Amide sind vorzugsweise z.B. einfache primäre und sekundäre Amide und Amide, abgeleitet von Aminosäuren oder deren Derivatem wie die von Alanin, Phenylalanin und dergleichen abgeleiteten Amide.

Insbesondere betrifft die Erfindung die Verbindungen der Formel

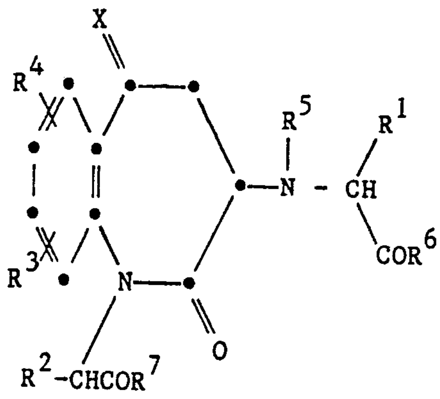

(IA),

worin der carbocyclische Ring auch Hexahydro sein kann; $R^1$ Wasserstoff, Niederalkyl, Amino-niederalkyl, Aryl, Aryl-niederalkyl, Cycloalkyl, Cycloalkyl-niederalkyl, Acylamino-niederalkyl, Mono-oder Di-niederalkylamino-niederalkyl, Niederalkylthio-niederalkyl, Carboxy-niederalkyl, verestertes Carboxy-niederalkyl, Carbamoyl-niederalkyl, N-substituiertes Carbamoyl-niederalkyl, Hydroxy-niederalkyl, verethertes oder acyliertes Hydroxy-niederalkyl, Aryloxyniederalkyl, Arylthio-niederalkyl, Aryl-N-niederalkylamino-niederalkyl oder Arylamino-niederalkyl bedeutet; $R^2$ und $R^5$ Wasserstoff oder Niederalkyl bedeuten; $R^3$ und $R^4$ Wasserstoff, Niederalkyl, Niederalkoxy, Niederalkanoyloxy, Hydroxy, Halogen oder Trifluormethyl bedeuten oder $R^3$ und $R^4$ gemeinsam Niederalkylendioxy darstellen; X Oxo, zwei Wasserstoffatome, oder eine Hydroxygruppe oder acylierte Hydroxygruppe und ein wasserstoff (unter Berücksichtigung der unter Formel I angegebenen Massgabe) bedeutet; $R^6$ und $R^7$ unabhängig voneinander Hydroxy, Amino, Mono-oder Di-niederalkylamino, Niederalkoxy, Aryl-niederalkoxy, Niederalkanoyloxymethoxy, (Amino, Mono- oder Di-niederalkylamino, Carboxy oder Niederalkoxycarbonyl)-niederalkoxy bedeuten; oder deren pharmazeutisch verträgliche Salze.

Bevorzugte Ausführungsformen der Erfindung betreffen Verbindungen der Formel IA, worin $R^1$ Wasserstoff, Niederalkyl, Amino-niederalkyl, Aryl, Aryl-niederalkyl, Cycloalkyl-niederalkyl ist; und worin innerhalb der obigen Definitionen Aryl unsubstituiertes oder durch Niederalkyl, Niederalkoxy, Niederalkylendioxy, Niederalkanoyloxy, Hydroxy, Halogen oder Trifluormethyl mono- oder disubstituiertes Phenyl bedeutet; und Cycloalkyl 3 bis 8 Kohlenstoffatome enthält; X (unter Berücksichtigung der unter Formel I angegebenen Massgabe) und $R^2$ bis $R^7$ die obigen Bedeutungen haben; oder ihre pharmazeutisch verträglichen Salze; oder die genannten Verbindungen, worin der carbocyclische Ring Hexahydro ist.

Weitere bevorzugte Verbindungen der Erfindung sind diejenigen der Formel IA, worin $R^1$ Aryl-niederalkyl ist, worin Aryl Indolyl bedeutet, und auch für Carboxy-niederalkyl, Niederalkoxycarbonylniederalkyl, Hydroxy-niederalkyl, Niederalkylthio-niederalkyl, Acylamino-niederalkyl; Aryloxy-niederalkyl oder Arylthio-niederalkyl steht; X (unter Berücksichtigung der unter Formel I angegebenen Massgabe) und $R^2$ bis $R^7$ die obigen Bedeutungen haben; oder ihre pharmazeutisch verträglichen Salze; oder die genannten Verbindungen, worin der carbocyclische Ring Hexahydro ist.

Besonders bevorzugte Verbindungen der Erfindung sind diejenigen der Formel IA, worin $R^1$ Wasserstoff, Niederalkyl, Amino-niederalkyl, Acylamino-niederalkyl, Aryl-niederalkyl, worin Aryl unsubstituiertes Phenyl oder durch Niederalkyl, Hydroxy, Niederalkoxy, Niederalkylendioxy, Niederalkanoyloxy, Halogen oder Trifluormethyl mono- oder disubstituiertes Phenyl ist, bedeutet; $R^3$ und $R^4$ Wasserstoff, Niederalkyl, Niederalkoxy, Halogen oder Trifluormethyl bedeuten oder $R^3$ und $R^4$ gemeinsam für Alkylendioxy stehen; $R^6$ und $R^7$ unabhängig voneinander Hydroxy, Amino, Niederalkoxy, Phenyl-niederalkoxy, Niederalkoxycarbonyl-niederalkoxy bedeuten; $R^2$, $R^5$ und X (unter Berücksichtigung der unter Formel I angegebenen Massgabe) die obigen Bedeutungen haben, oder ihre pharmazeutisch verträglichen Salze; oder die genannten Verbindungen, worin der carbocyclische Ring Hexahydro ist.

Besonders wertvoll sind Verbindungen der Formel IA, worin $R^1$ Wasserstoff, Niederalkyl, Amino-niederalkyl, Aryl-niederalkoxycarbonylamino-niederalkyl oder Aryl-niederalkyl bedeutet, worin Aryl für unsubstituiertes Phenyl oder durch Niederalkyl, Hydroxy, Niederalkoxy, Niederalkanoyloxy, Halogen oder Trifluormethyl monosubstituiertes Phenyl steht; $R^2$ und $R^5$ Wasserstoff oder Niederalkyl bedeuten; $R^3$ und $R^4$ für Wasserstoff, Niederalkyl, Niederalkoxy, Halogen oder Trifluormethyl stehen, oder $R^3$ und $R^4$ gemeinsam Niederalkylendioxy bedeuten; X für Oxo, zwei Wasserstoffatome, oder eine Hydroxy- oder Niederalkanoyloxygruppe und ein Wasserstoffatom steht (unter Berücksichtigung der unter Formel I angegebenen Massgabe in einer Weise, die der hier für X genannten Bedeutung angepasst ist); $R^6$ und $R^7$ unabhängig voneinander für Hydroxy, Amino, Niederalkoxy, Phenyl-niederalkoxy oder Niederalkoxycarbonylniederalkoxy stehen; oder ihre pharmazeutisch verträglichen Salze; oder die genannten Verbindungen, worin der carbocyclische Ring Hexahydro ist.

Insbesondere bevorzugt sind Verbindungen der Formel IA, worin $R^1$ wasserstoff, Niederalkyl, ω-Amino-niederalkyl, ω-Arylmethoxycarbonylamino-niederalkyl oder Aryl-niederalkyl bedeutet, worin Aryl für unsubstituiertes Phenyl oder durch Niederalkyl, Hydroxy, Niederalkoxy, Niederalkanoyloxy, Halogen oder Trifluormethyl monosubstituiertes Phenyl steht; $R^2$ und $R^5$ Wasserstoff oder Niederalkyl bedeuten; $R^3$ für Wasserstoff steht; $R^4$ Wasserstoff, Niederalkyl, Niederalkoxy, Halogen oder Trifluormethyl bedeutet; X für Oxo, zwei Wasserstoffatome, oder eine Hydroxy- oder Niederalkanoyloxygruppe und ein Wasserstoffatom steht (unter Berücksichtigung der unter Formel I angegebenen Massgabe in einer Weise, die der hier für X genannten Bedeutung angepasst ist); $R^6$ und $R^7$ unabhängig voneinander für Hydroxy, Amino, Niederalkoxy, Phenyl-niederalkoxy, Niederalkoxycarbonyl-niederalkoxy stehen; oder ihre pharmazeutisch verträglichen Salze; oder die genannten Verbindungen, worin der carbocyclische Ring Hexahydro ist.

Vor allem wertvoll sind Verbindungen der Formel IA, worin $R^1$ die Bedeutung hat von Wasserstoff, Methyl, Ethyl, Isopropyl, ω-Aminopropyl, ω-Aminobutyl, ω-(Benzyloxycarbonylamino)propyl, ω-(Benzyloxycarbonylamino)butyl, Aryl-(methyl, ethyl, propyl), worin Aryl unsubstituiertes Phenyl oder durch Methyl, Hydroxy, Methoxy, Methylendioxy, Acetoxy, Chlor oder Trifluormethyl monosubstituiertes Phenyl bedeutet; $R^2$ und $R^5$ für Wasserstoff oder Methyl stehen; $R^3$ und $R^4$ Wasserstoff, Methoxy, Methyl, Chlor oder Trifluormethyl bedeuten; X für Oxo, zwei Wasserstoffatome, oder eine Hydroxy- oder Acetoxygruppe und ein Wasserstoffatom steht (unter Berücksichtigung der unter Formel I angegebenen Massgabe in einer Weise, die der hier für X genannten Bedeutung angepasst ist); $R^6$ und $R^7$ unabhängig

voneinander Hydroxy, Amino, Ethoxy, Methoxy, Benzyloxy, Ethoxycarbonylmethoxy oder Pivaloyloxymethoxy bedeuten; oder ihre pharmazeutisch verträglichen Salze; oder die genannten Verbindungen, worin der carbocyclische Ring Hexahydro ist.

Ueberragend wertvoll sind Verbindungen der Formel

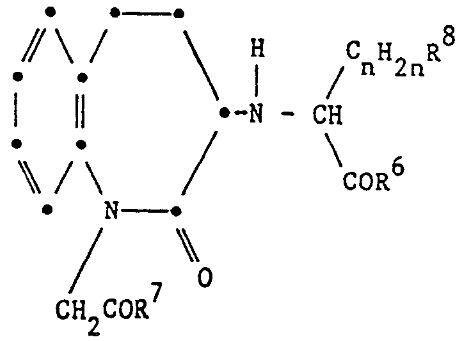

(IB),

worin n eine ganze Zahl von 1 bis 4 bedeutet, $R^8$ Benzyloxycarbonyl-amino bedeutet; $R^6$ und $R^7$ unabhängig voneinander Hydroxy, Niederalkoxy mit bis zu 4 Kohlenstoffatomen, Benzyloxy oder Amino bedeuten, oder ihre pharmazeutisch verträglichen Salze; oder die genannten Verbindungen, worin der carbocyclische Ring Hexahydro ist.

Weitere bevorzugte Verbindungen sind solche der Formel IB, worin $C_nH_{n2}$ Ethylen, n-Propylen oder n-Butylen bedeutet; $R^8$ für Benzyloxycarbonylamino steht; $R^6$ und $R^7$ unabhängig voneinander Hydroxy oder Niederalkoxy mit bis zu 4 Kohlenstoffatomen bedeuten; oder ihre pharmazeutisch verträglichen Salze; oder die genannten Verbindungen, worin der carbocyclische Ring Hexahydro ist.

Die vorliegende Erfindung betrifft auch die Stereoisomeren der Verbindungen der Formel I. Eine Anzahl Racemate ist erhältlich, wenn z.B. in der Formel IA zumindest einer der Reste $R^1$ und $R^2$ nicht Wasserstoff und/oder X H(OH) oder H(acyliertes OH) bedeutet. Weiter können die Verbindungen der Erfindung, in welchen der carbocyclische Ring Hexahydro ist, je nach der Ringverknüpfung als cis- oder trans-Isomere vorliegen.

Die einzelnen Enantiomeren dieser Racemate können ihrerseits erhalten werden. Bestimmte spezifische Vertreter dieser Isomere sind als Inhibitoren für das Angiotensin-umwandelnde Enzym bevorzugt.

Bevorzugt sind die oben genannten Verbindungen, in welchen das asymmetrische Ringkohlenstoffatom (in 3-Stellung), welches die substituierte Aminogruppe trägt, die (S)-Konfiguration aufweist. Weiter bevorzugte Verbindungen sind solche, in welchen das asymmetrische Kohlenstoffatom der Seitenkette, welches die Gruppe $COR^6$ trägt, die (S)-Konfiguration hat.

Herausragend sind Verbindungen der Formel

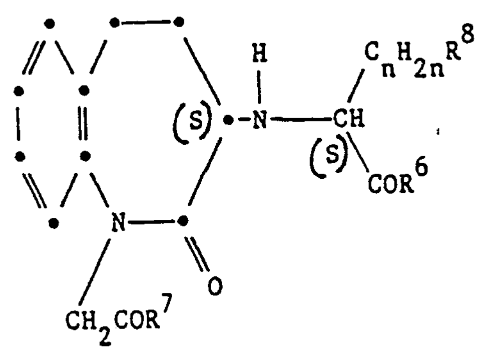

(IC),

worin S die Chiralität bedeutet, n eine ganze Zahl von 1 bis 4 bedeutet; $R^8$ Benzyloxycarbonylamino bedeutet; $R^6$ und $R^7$ unabhängig voneinander Hydroxy, Niederalkoxy mit bis zu 4 Kohlenstoffatomen, Benzyloxy oder Amino bedeuten; oder ihre pharmazeutisch verträglichen Salze.

Die Verbindungen der Formel I gemäss der Erfindung können in an sich bekannter Weise hergestellt werden, indem man z.B.

a) in eine Verbindung der Formel

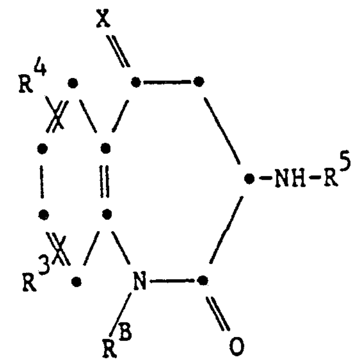

(II),

worin der carbocyclische Ring auch Hexahydro oder 6,7,8,9-Tetrahydro sein kann und worin X, $R^B$, $R^3$, $R^4$ und $R^5$ die vorstehend angegebenen Bedeutungen besitzen, $R^A$ durch Alkylierung mit einer Verbindung der Formel

$$R^A—Z \qquad (IIIA),$$

worin Z eine reaktive veresterte Hydroxygruppe bedeutet und $R^A$ die vorstehend angegebenen Bedeutungen besitzt, oder mit einer Verbindung der Formel

$$R^1—CO—R^o \qquad (IV),$$

worin $R^1$ und $R^o$ die oben angegebenen Bedeutungen haben, in Gegenwart eines Reduktionsmittels, unter vorübergehendem Schotz etwaiger primärer und sekundärer Aminogruppem und/oder gegebenenfalls Hydroxy- und/oder Oxogruppen, die in irgendeinem der Reste X, $R^B$, $R^3$ und $R^4$ und/oder im Alkylierungsmittel anwesend sein können, einführt oder

b) eine Verbindung der Formel

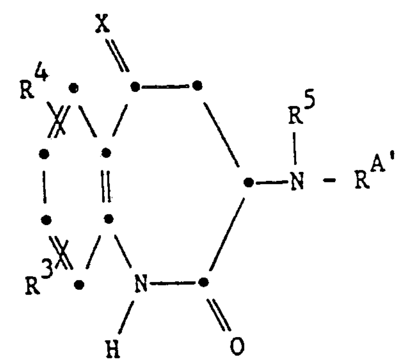

(V),

worin der carbocyclische Ring auch Hexahydro oder 6,7,8,9-Tetrahydro sein kann und worin X, $R^3$, $R^4$ und $R^5$ die vorstehend angegebenen Bedeutungen besitzen und $R^{A'}$ Wasserstoff oder $R^A$ wie vorstehend definiert ist, mit einer Verbindung der Formel

$$R^B—Z \qquad (IIIB),$$

worin Z eine reaktive veresterte Hydroxygruppe bedeutet und $R^B$ die vorstehend angegebenen Bedeutungen besitzt, alkyliert, wobei man vorübergehend etwaige primäre und sekundäre Aminogruppen und/oder gegebenenfalls Hydroxy- und/oder Oxogruppen, die in irgendeinem der Reste X, $R^{A'}$ $R^B$, $R^3$ und $R^4$ anwesend sein können, schützt oder

c) eine Verbindung der Formel

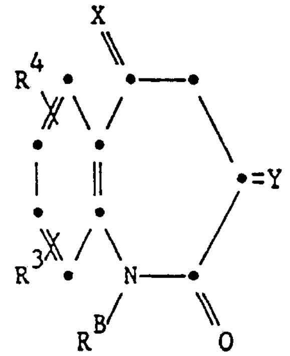

(VI),

worin der carbocyclische Ring auch Hexahydro oder 6,7,8,9-Tetrahydro sein kann und worin Y Oxo, Dichlor, oder eine reaktive veresterte Hydroxygruppe Z gemeinsam mit Wasserstoff bedeutet und X, $R^B$, $R^3$ und $R^4$ die vorstehend angegebenen Bedeutungen besitzen, mit einem Amin der Formel

$$R^A—NH—R^5 \qquad (VII),$$

worin $R^A$ und $R^5$ die vorstehend angegebenen Bedeutungen besitzen, kondensiert, wobei, wenn Y Oxo oder Dichlor bedeutet, die Kondensation in Gegenwart eines Reduktionsmittels und unter vorübergehendem Schutz der Oxogruppe, die als Substituent X anwesend sein kann, durchgeführt wird, oder

d) in einer Verbindung der Formel

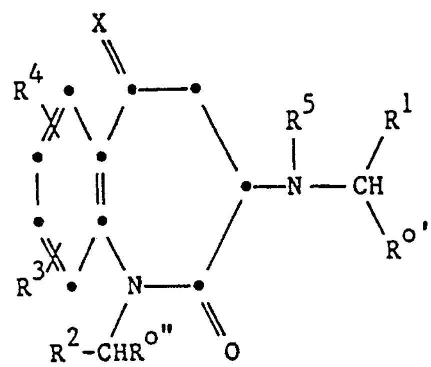

(VIII),

worin der carbocyclische Ring auch Hexahydro oder 6,7,8,9-Tetrahydro sein kann und worin X und $R^1$ bis $R^5$ die vorstehend angegebenen Bedeutungen besitzen, eines der Symbole $R^{o\prime}$ und $R^{o\prime\prime}$ Cyano ist und das andere Cyano oder $R^o$ wie vorstehend definiert ist, die Cyanogruppe(n) einer Solvolyse unterzieht oder

e) eine Verbindung der Formel

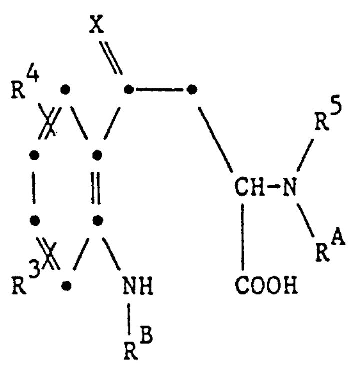

(IX),

worin der carbocyclische Ring auch Hexahydro oder 3,4,5,6-Tetrahydro sein kann und worin X, $R^A$, $R^B$, $R^3$, $R^4$ und $R^5$ die vorstehend angegebenen Bedeutungen besitzen, oder einen Ester hiervon cyclisiert oder

f) eine Verbindung, die strukturell identisch ist mit einer Verbindung der Formel I, wie oben definiert, ausser dass sie eine weitere Doppelbindung am C—3 oder zwischen dem Stickstoffatom und dem benachbarten Kohlenstoffatom innerhalb der Gruppe $R^A$ aufweist, mit einem Reduktionsmittel behandelt, um diese Doppelbindung zu sättigen, oder

g) um eine Verbindung der Formel I, wie vorstehend definiert, in der X Oxo bedeutet, herzustellen, eine Verbindung der Formel

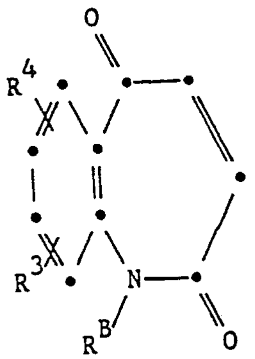

(X),

in der der Carbocyclus auch Hexahydro oder 6,7,8,9-Tetrahydro sein kann, und $R^B$, $R^3$ und $R^4$ die vorstehend angegebenen Bedeutungen haben, mit einem Amin der Formel

$$R^A—NH—R^5 \quad (VII),$$

worin $R^A$ und $R^5$ die vorstehend angegebenen Bedeutungen haben, kondensiert und gewünschtenfalls eine erhaltene Verbindung der Formel I in eine andere Verbindung der Formel I umwandelt und/oder gewünschtenfalls eine erhaltene Verbindung der Formel I, die salzbildende Eigenschaften aufweist, in ihr Salz oder ein erhaltenes Salz in ein anderes Salz überführt oder eine freie Verbindung aus einem derartigen Salz freisetzt und/oder gewünschtenfalls ein optisches Isomeres, das eine spezifische Konfiguration im Hinblick auf zumindest ein Chiralitätszentrum besitzt, aus einer Mischung der stereoisomeren Formen einer erhaltenen Verbindung der Formel I anreichert.

Eine reaktive veresterte Hydroxygruppe Z ist eine mit einer starken organischen Säure veresterte Hydroxygruppe, z.B. einer aliphatischen oder aromatischen Sulfonsäure (wie einer Niederalkansulfonsäure, insbesondere Methansulfonsäure, Trifluormethansulfonsäure, insbesondere Benzolsulfonsäure, p-Toluolsulfonsäure, p-Brombenzolsulfonsäure und p-Nitrobenzolsulfonsäure) oder mit einer starken anorganischen Säure, wie insbesondere Schwefelsäure, oder einer Halogenwasserstoffsäure, wie Chlorwasserstoffsäure oder am meisten bevorzugt Iodwasserstoffsäure oder Bromwasserstoffsäure, veresterte Hydroxygruppe.

Eine substitutive Alkylierung gemäss der vorliegenden Erfindung wird unter herkömmlichen allgemeinen Bedingungen bei Temperaturen im Bereich von etwa 0°C bis zur Siedetemperatur der Reaktionsmischung, vorzugsweise bei Temperaturen zwischen Raumtemperatur und etwa 100°C, durchgeführt. Die Umsetzung findet vorteilhaft in Gegenwart eines Lösungsmittels, das im Hinblick auf die Reaktanten inert ist, statt, wie in Gegenwart eines chlorierten Niederalkans (z.B. Chloroform oder Methylenchlorid), eines acyclischen oder cyclischen Ethers (z.B. Diethylether, 1,2-Dimethoxyethan, Dioxan oder Tetrahydrofuran) und insbesondere eines tertiären Amids mit niedrigem Molekulargewicht (z.B. N,N-Dimethylformamid, N,N-Dimethylacetamid, N-Methylpyrrolidon, N-Ethylpiperidon und Hexamethylphosphorsäure-trisamid). Vorteilhafterweise wird die während der Reaktion freigesetzte starke Säure HZ durch Zugabe eines säurebindenden Mittels gebunden, wie vorzugsweise eines anorganischen Säurefängers, wie eines Alkalimetallbicarbonats, -carbonats oder -hydroxids, eines organischen quaternären Ammoniumsalzes (z.B. eines Tetrabutylammoniumsalzes) oder einer organischen tertiären Base wie Triethylamin, N-Ethylpiperidin, Pyridin oder Chinolin.

Eine erfindungsgemässe Alkylierung kann auch unter an sich bekannten und im Stand der Technik verwendeten Bedingungen einer reduktiven Alkylierung durchgeführt werden. Bei Durchführung dieser Alkylierung werden die Ausgangsstoffe gleichzeitig oder in einem nachfolgenden Schritt mit einem Reduktionsmittel umgesetzt. Unter Reduktionsmitteln, die gleichzeitig mit dem Alkylierungsmittel verwendet werden, sollen Ameisensäure und komplexe Metallhydride, wie Natriumcyanborhydrid, erwähnt werden; unter den Reduktionsmitteln, die überwiegend in einer getrennten nachfolgenden Arbeitsstufe, d.h. zur Reduktion eines vorher gebildeten Imins (Schiff'sche Base) verwendet werden, sollen Diboran und komplexe Metallhydride, wie Natriumborhydrid und Natriumcyanborhydrid, erwähnt werden, die vorteilhaft dem primären Reaktionsgemisch ohne Isolierung eines Zwischenprodukts, z.B. des Imins, zugegeben werden. In diesem Fall wird die Alkylierung vorteilhaft in einem gegenüber dem Reduktionsmittel inerten organischen Lösungsmittel durchgeführt, wie in einem aliphatischen oder cyclischen Ether (wie Diethylether, Diisopropylether, 1,2-Dimethoxyethan, Dioxan oder Tetrahydrofuran) oder in einem aliphatischen Alkohol (wie Methanol, Ethanol, Isopropylalkohol, Glykol, Glykolmonomethylether oder Diethylenglykol), vorzugsweise bei etwa 0 bis 80°C. Ein wesentliches Reduktionsmittel, das sowohl gleichzeitig als auch anschliessend verwendet werden kann, ist Wasserstoff, insbesondere katalytisch aktivierter Wasserstoff. Die Katalysatoren sind diejenigen, die herkömmlich als Hydrierungskatalysatoren eingesetzt werden, vorzugsweise diejenigen der Klasse der Edelmetalle (wie Palladium,

Platin und Rhodium) auf einem Träger (wie Calciumcarbonat, Aluminiumoxid oder Bariumsulfat), in feindisperser Suspension ohne Träger oder in Form von Komplexen in homogener Phase. Auch feindispergierte Uebergangsmetalle, z.B. Raney-Metalle, insbesondere Raney-Nickel, sind sehr geeignete Katalysatoren für die reduktive Alkylierung. Die speziellen Reaktionsbedingungen hängen in grossem Ausmass von dem jeweiligen Hydrierungskatalysator und dessen exakter Aktivität ab und weichen von den allgemein für die Hydrierung bekannten nicht ab. Temperaturen im Bereich von Raumtemperatur bis zu etwa 150°C und Wasserstoffdrücke im Bereich von Atmosphärendruck bis zu etwa 300 Atmosphären sind gemäss den Standardverfahren des Standes der Technik anwendbar. Zusätzlich zu den vorstehend im Zusammenhang mit der Hydridreduktion erwähnten inerten Lösungsmitteln können auch niedermolekulare Amide, insbesondere tertiäre Amide (wie N,N-Dimethylformamid, N,N-Dimethylacetamid, N-Methylpyrrolidon, N-Ethylpiperidon, Hexamethylphosphorsäure-trisamid), auch Formamid und Acetamid als geeignete Lösungsmittel verwendet werden. Spezielle Massnahmen müssen bei Ausgangsmaterialien angewandt werden, die eine leicht reduzierbare funktionelle Gruppe, wie die 5-Oxogruppe, aufweisen; um diese Gruppen zu erhalten, müssen selektive Reduktionsbedingungen, wie sie aus dem Stand der Technik bekannt sind, angewandt werden, oder wenn eine gleichzeitige Reduktion dieser Gruppen erwünscht oder erforderlich ist, werden dementsprechend energische Reagentien und/ oder Bedingungen angewandt.

Die oben genannten im voraus gebildeten Imine werden vorzugsweise durch Kondensation entsprechender Ausgangsstoffe in einem inerten Lösungsmittel, z.B. Toluol oder Methylenchlorid, in erster Linie in Gegenwart von Dehydratisierungskatalysatoren, z.B. Bortrifluoridetherat, p-Toluolsulfonsäure oder Molekularsieben, hergestellt.

Im Falle der Reaktanten der Formeln IIIA, IIIB, IV und VII, welche eine freie Carboxylgruppe $R^o$ enthalten, kann ein geeignetes Carboxylatsalz, vorzugsweise in situ, vor der Kondensation mit den nachfolgend ausführlich genannten, gewünschten Zwischenprodukten hergestellt werden.

In jedem der Alkylierungsverfahren müssen primäre und sekundäre Aminogruppen in den Ausgangsmaterialien, mit Ausnahme der zu alkylierenden Aminogruppe, in einer vorübergehend geschützten Form während der Alkylierung vorliegen. Geeignete Schutzgruppen sowie Verfahren zu deren Einführung und Abspaltung sind aus dem Stand der Technik, der bis in die Einzelheiten ausgearbeitet ist, insbesondere als allgemeine Methoden für die Synthese von Peptiden, bekannt, vergl. Houben-Weyl: Methoden der Organischen Chemie, 4. Ausgabe, Band 15/I und II, E. Wünsch (Herausgeber): Synthese von Peptiden (Georg Thieme-Verlag, Stuttgart, 1974). Die nähere Auswahl der Schutzgruppen hängt von dem speziellen Zweck ab, wobei es erforderlich ist, insbesondere die speziellen Eigenschaften der jeweiligen Ausgangsmaterialien und die Reaktionsbedingungen des jeweiligen Verfahrens mit zu berücksichtigen. Im Fall von verschiedenen zu schützenden funktionellen Gruppen können vorteilhafte Kombinationen ausgewählt werden. Vorzugsweise werden z.B. ähnliche oder besser identische Aminoschutzgruppen sowohl in den Resten $R^o$ als auch im Rest $R^1$ verwendet und gleichzeitig im Anschluss an die Alkylierung abgespalten.

Geeignet als Aminoschutzgruppen sind insbesondere Aminoschutzgruppen, die durch Reduktion entfernt werden können, wie z.B. insbesondere diejenigen vom Benzyloxycarbonyl-Typ, bei denen die Benzyloxycarbonylgruppe an dem aromatischen Teil durch Halogenatome, Niederalkoxygruppen und/oder Niederalkylreste und insbesondere durch Nitrogruppen, substituiert sein kann, wie die p-Chlor- und p-Brombenzyloxycarbonyl-, p-Methoxybenzyloxycarbonyl-, p-Methylbenzyloxycarbonyl- und insbesondere p-Nitrobenzyloxycarbonylgruppe, oder alternativ die Isonicotinyloxycarbonylgruppe. Eine vorteilhafte Aminoschutzgruppe ist eine Ethoxycarbonylgruppe, die in der β-Stellung eine durch drei Kohlenwasserstoffreste substituierte Silylgruppe enthält, wie Triphenylsilyl, Dimethyl-tert.-butylsilyl oder insbesondere Trimethylsilyl. Eine β-(Trihydrocarbylsilyl)-ethoxycarbonylgruppe dieses Typs, wie eine β-(Triniederalkylsilyl)-ethoxycarbonylgruppe, z.B. insbesondere β-(Trimethylsilyl)-ethoxycarbonyl, bildet mit der zu schützenden Aminogruppe eine entsprechende β-Trihydrocarbylsilylethoxycarbonylaminogruppe (z.B. die β-Trimethylsilylethoxycarbonylaminogruppe), die unter sehr speziellen, sehr milden Bedingungen durch Einwirken von Fluorid-Ionen abgespalten werden kann.

Es ist auch möglich, Gruppen zu verwenden, die durch Acidolyse abgespalten werden können, wie die tert.-Butoxycarbonylgruppen und analoge Gruppen, ebenso diejenigen des Aralkyl-Typs, wie Benzhydryl, Di-(4-Methoxy)-benzhydryl und Triphenylmethyl (Trityl), oder bestimmte Aralkoxycarbonylgruppen vom 2-(p-Biphenylyl)-2-propoxycarbonyl-Typ, die in der CH—PS 509 266 beschrieben werden. Es sei bemerkt, dass sich von Estern der Kohlensäure ableitende Schutzgruppen in den meisten Fällen auch durch basische Hydrolyse abspaltbar sind.

Für den fakultativen vorübergehenden Schutz der Hydroxygruppen können mit Vorteil Schutzgruppen verwendet werden, die durch Reduktion abgespalten werden können, vgl. den vorstehend zitierten Text (Houben-Weyl), und auch Gruppen, die durch Acidolyse entfernt werden können, wie 2-Tetrahydropyranyl, tert.-Butoxycarbonyl und tert.-Butyl. Bevorzugte Hydroxyschutzgruppen, die durch Reduktion abgespalten werden können, sind beispielsweise Benzylgruppen, die an dem aromatischen Teil durch Halogen, Niederalkyl, Niederalkoxy und/oder insbesondere Nitro, -insbesondere die 4-Nitrobenzylgruppe-, substituiert sein können. Es ist auch möglich, Acylgruppen, die unter schwach basischen Bedingungen abgespalten werden können, wie Formyl oder Trifluoracetyl, zu verwenden.

Für den fakultativen Schutz der Oxogruppen werden diese vorzugsweise als Ketale, insbesondere als

Ketale abgeleitet von Niederalkanolen, wie Methanol oder Ethanol, oder vorteilhaft von Ethylenglykol, oder als entsprechende Thioketale, vorzugsweise als solche von 1,2-Ethandithiol, geschützt. Alle diese Gruppen können unter den nachstehend angegebenen Bedingungen Oxogruppen freisetzen.

Die anschliessende Abspaltung der Schutzgruppen gemäss der Erfindung hängt von deren Natur ab und wird in jedem Fall in an sich bekannter herkömmlicher Weise durchungeführt, wobei die allgemeinen Eigenschaften des sich ergebenden Produkts in Betracht gezogen werden. Wenn die Schutzgruppen für Amino, Hydroxy und Oxo so ausgewählt worden sind, dass sie unter ähnlichen Bedingungen abgespalten werden können (insbesondere bevorzugt sind die durch Acidolyse oder bei Amino und Hydroxy durch Reduktion abspaltbaren Gruppen, die bereits im einzelnen erwähnt worden sind), werden alle diese Schutzgruppen mit Vorteil in einem einzigen Arbeitsgang abgespalten; in speziellen Fällen ist es jedoch möglich, verschiedene Typen von Gruppen zu verwenden und jede von ihnen einzeln abzuspalten.

Die Gruppen, die durch Reduktion abgespalten werden können, insbesondere diejenigen, die halogenierte Niederalkylreste (z.B. 2,2,2-Trichlorethylreste), Isonicotinylreste (z.B. Isonicotinyloxycarbonyl), und insbesondere substituierte Benzylreste, vor allem 4-Nitrobenzylreste jeglicher Art enthalten, werden vorzugsweise durch Reduktion mit Zink, gewöhnlich in Gegenwart einer Säure, vorzugsweise Essigsäure, und mit oder ohne Zugabe eines inerten organischen Lösungsmittels, gewöhnlich bei Raumtemperatur abgespalten. Die Abspaltung einer Schutzgruppe durch saure Hydrolyse (Acidolyse) wird im Fall von Gruppen des tert.-Butyl-Typs mit Hilfe von Chlorwasserstoff, Fluorwasserstoff oder Trifluoressigsäure und im Fall von säureempfindlichen Schutzgruppen hauptsächlich mit Hilfe einer niederen aliphatischen Carbonsäure, wie Ameisensäure und/oder Essigsäure, in Gegenwart von Wasser und gegebenenfalls eines polyhalogenierten Niederalkanols oder Niederalkanons, wie 1,1,1,3,3,3-Hexafluorpropan-2-ol oder Hexafluoraceton, durchgeführt. Auf diese Weise ist es beispielsweise möglich, eine N-Tritylgruppe durch eine organische Säure, wie Ameisensäure, Essigsäure, Chlor-essigsäure oder Trifluoressigsäure, in wässrigem oder absolutem Trifluorethanol als Lösungsmittel (vgl. DE—OS 23 46 147) oder durch wässrige Essigsäure zu spalten, die tert.-Butoxycarbonylgruppe durch Trifluoressigsäure oder Chlorwasserstoffsäure abzuspalten und die 2-(p-Biphenylyl)-isopropoxycarbonylgruppe durch wässrige Essigsäure oder beispielsweise durch ein Gemisch von Eisessig, Ameisensäure (Konzentration von 82,8%) und Wasser (7:1:2) oder gemäss dem Verfahren in der DE—OS 23 46 147 zu entfernen. Die β-Silylethylestergruppen werden vorzugsweise durch Fluorid-Ionen-abgebende Reagentien, beispielsweise Fluoride von quaternären organischen Basen, wie Tetraethylammoniumfluorid, abgespalten.

Ketalisierte und thioketalisierte Oxogruppen werden durch Acidolyse mit üblichen starken anorganischen Säuren oder mit Oxalsäure in Gegenwart von Wasser, die letztgenannten vorteilhaft durch Behandlung mit einem schwefelbindenden Mittel, z.B. einem Quecksilber(II)Salz und/oder Cadmiumcarbonat in freie Oxogruppen übergeführt. Schutzgruppen, die gegenüber basischen Bedingungen instabil sind, beispielsweise Formyl, Trifluoracetyl und Kohlensäureestergruppen, können vorsichtig durch Einwirken einer wässrigen Natrium- oder Kaliumbicarbonat- oder -carbonatlösung oder auch durch Einwirken von wässrigem Ammoniak in einem organischen Lösungsmittel, gewöhnlich bei Raumtemperatur, entfernt werden. Die Schutzgruppen werden vorzugsweise unter den Reaktionsbedingungen der Beispiele oder unter analogen Bedingungen abgespalten.

Verfahren a) Die Kondensation der Amine der Formel II mit den bekannten α-Ketosäure-Derivaten der Formel IV (vgl. Chem. Ber. *31*, 551, 3133) durch reduktive N-Alkylierung wird unter aus dem Stand der Technik bekannten Bedingungen, z.B. durch katalytische Hydrierung mit Wasserstoff in Gegenwart von Platin-, Palladium- oder Nickel-Katalysatoren oder mit chemischen Reduktionsmitteln, wie einfachen oder komplexen Leichtmetallhydriden, vorteilhaft einem Alkalimetallcyanborhydrid, wie Natriumcyanborhydrid, durchgeführt. Die reduktive Aminierung mit einem Alkalimetallcyanborhydrid wird vorzugsweise in einem inerten Lösungsmittel, z.B. Methanol oder Acetonitril, vorteilhaft in Gegenwart einer Säure, z.B. Chlorwasserstoffsäure oder Essigsäure, bei einer Temperatur zwischen etwa 0 und 50°C, vorzugsweise Raumtemperatur, durchgeführt.

Die Alkylierung der Amine der Formel II mit einem Reagens der Formel IIIA, wie es aus dem Stand der Technik gut bekannt ist, wird mit oder ohne basische Katalysatoren, wie Triethylamin oder Kaliumcarbonat in einem inerten Lösungsmittel durchgeführt.

Die Ausgangsmaterialien der Formeln IIIA und IV sind bekannt, oder wenn sie unbekannt sind, können sie auf einfache Weise nach herkömmlichen Syntheseverfahren hergestellt werden. Die Ausgangsmaterialien der Formel II können nach herkömmlichen Syntheseverfahren erhalten werden und mit Vorteil in der Weise, die nachstehend eingehender für die speziellen Zwischenprodukte beschrieben und veranschaulicht ist.

Verbindungen der Formel II können durch Kondensation, unter basischer Katalyse, einer Verbindung der Formel

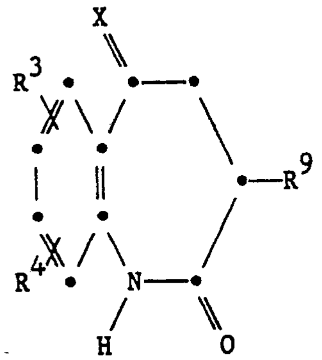

(XI)

oder eines Hexahydro-Derivates davon, worin $R^3$ und $R^4$ Wasserstoff, Niederalkyl, Niederalkoxy, Niederalkanoyloxy, Halogen oder Trifluormethyl, oder $R^3$ und $R^4$ zusammen Niederalkylendioxy bedeuten, X zwei Wasserstoffatome, ein Wasserstoffatom und ein verethertes oder verestertes Hydroxy, Oxo oder als Ketal oder Thioketal geschütztes Oxo bedeutet, und $R^9$ Amino, Niederalkylamino, Azido oder Acylamino, z.B. Niederalkanoylamino oder -alkoxycarbonylamino, bedeutet, mit einer Verbindung der Formel

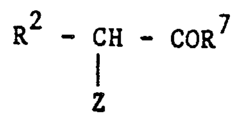

(III'B),

worin $R^2$ Wasserstoff oder Niederalkyl, $R^7$ Hydroxy, Diniederalkylamino, Niederalkoxy, Arylniederalkoxy, Niederalkanoyloxymethoxy oder Niederalkoxycarbonylniederalkoxy und Z eine reaktive veresterte Hydroxygruppe bedeutet; und gegebenenfalls Reduktion, Hydrogenolyse, Hydrolyse oder Alkylierung des erhaltenen Zwischenprodukts, erhalten werden.

Verbindungen der Formel XI, werden aus den entsprechenden gegebenenfalls substituierten und/oder derivatisierten 2,3,4,5-Tetrahydro-1H-1-benzazepin-2-onen [J. Chem. Soc. *1937*, 456, British Patent 1,359,285, Liebigs Ann. Chem. *574*, 171 (1951)] erhalten. Neue, in geeigneter Weise derivatisierte 1-Benzazepin-2-on-Ausgangsverbindungen werden vorteilhaft durch Beckmann-Umlagerung der entsprechend derivatisierten Naphthalen-1-one unter Verwendung von wohlbekannten und hierin beschriebenen Verfahren hergestellt.

Solche Tetrahydro-1-benzazpin-2-one werden in die 3-Halogen-, z.B. die 3-Chlor-2,3,4,5-tetrahydro-1H-1-benzazepin-2-one übergeführt, wobei hierin beschriebene Verfahren angewendet werden, z.B. Behandlung mit Phosphorpentachlorid mit anschliessender Hydrierung. Substitution solcher Halogenderivate mit einem Metallazid, z.B. Natriumazid, und gegebenenfalls Reduktion, oder Substitution mit Ammoniak oder einem Niederalkylamin, und gegebenenfalls Acylierung ergibt Verbindungen der Formel XI.

Alternativ können Verbindungen der Formel XI, worin $R^9$ Amino, Alkylamino oder Acylamino bedeutet, durch Reduktion und Cyclisierung einer in geeigneter Weise substituierten und/oder derivatisierten 4-(2-Nitrophenyl)-2-aminobuttersäure und gegebenenfalls nachfolgende N-Alkylierung oder N-Acylierung hergestellt werden.

Eine alternative Synthese für die optisch aktiven Verbindungen dieser Erfindung geht aus von der natürlichen Aminosäure Tryptophan. So wird L-4-(2-Aminophenyl)-4-oxo-2-aminobuttersäure (L-Kynurenin, J. Am. Chem. Soc. *76*, 1708 (1954), erhalten aus L-Tryptophan) in ein optisch aktives Ausgangsmaterial der Formel XI überführt, worin $R^9$ Acylamino bedeutet, z.B. 3-(S)-t-Butoxycarbonylamino-2,3,4,5-tetrahydro-1H-1-benzazepin-2,5-dion, wie in Australian Journal of Chemistry 33, 633—40 (1980) beschrieben. Die Lactam-Alkylierung einer Verbindung der Formel XI mit einem Reagens der Formel IIIB, wird auf bekannte Weise, vorzugsweise in Gegenwart von Basen, wie Alkalimetallhydriden, z.B. Natrium- oder Kaliumhydrid, Alkalimetallalkoxiden, wie Kalium-tert.-butylat oder Natriummethanolat, organo-metallischen Reagentien, z.B. Lithiumdiisopropylamid, oder unter Bedingungen der Phasen-Transfer-Katalyse, z.B. in Gegenwart eines Tetrabutylammoniumsalzes, vorzugsweise in einem Lösungsmittel, z.B. Tetrahydrofuran, Dimethylformamid, bei einer Temperatur vorzugsweise zwischen 0° und 75°C, durchgeführt.

Das Verfahren b) wird in üblicher Weise, unter den Bedingungen der vorher beschriebenen substitutiven Alkylierung, vorzugsweise in Gegenwart von sehr starken Basen, wie Alkalimetallhydriden (z.B. Natrium- oder Kaliumhydrid), -alkoholaten (z.B. Natrium-methylat oder -ethylat, Kalium-tert.-butylat) oder -amiden (z.B. Lithiumdiisopropylamid) durchgeführt, wobei die vorstehend erwähnten Ether und Amide als Lösungsmittel bevorzugt sind. Bei einer speziellen Ausführungsform des Verfahrens b) werden Ausgangsmaterialien verwendet, worin $R^{A'}$ Wasserstoff bedeutet, und es werden zumindest zwei Aequivalente der Reaktante IIIB verwendet. In dem erhaltenen Produkt sind beide Reste $R^A$ und $R^B$ identisch und liegen im Bereich der Bedeutungen von $R^B$.

Die Ausgangsmaterialien der Formel IIIB sind bekannt, oder wenn sie unbekannt sind, können sie auf einfache Weise nach herkömmlichen Syntheseverfahrem hergestellt werden. Die Ausgangsmaterialien der Formel V können nach herkömmlichen Syntheseverfahren erhalten werden und mit Vorteil in der Weise, die nachstehend eingehender für die speziellen Zwischenprodukte beschrieben und veranschaulicht ist.

Verbindungen der Formel V können durch Kondensation unter den Bedingungen der reduktiven Alkylierung einer Verbindung der Formel,

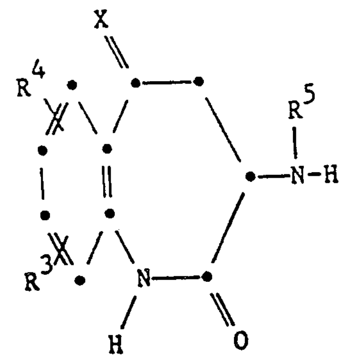

(XII)

oder ihres Hexahydro-Derivates, worin $R^3$, $R^4$ und X die für Formel XI angegebenen Bedeutungen haben, und $R^5$ Wasserstoff oder Niederalkyl bedeutet, mit einer Verbindung der Formel

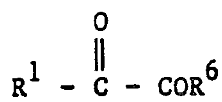

(IV'),

worin $R^1$ und $R^6$ die vorher definierten Bedeutungen haben oder unter Alkylierungsbedingungen mit einer Verbindung der Formel

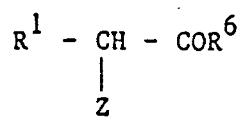

(III'A),

worin $R^1$, $R^6$ und Z die vorher angegebenen Bedeutungen haben, erhalten werden.

Das Verfahren c), das auch eine Alkylierungsreaktion darstellt, wird entsprechend den gleichen allgemeinen Gesichtspunkten und unter den gleichen experimentellen Bedingungen durchgeführt, wie im einzelnen vorstehend beschrieben (substitutive oder reduktive Alkylierung). Die Ausgangsmaterialien der Formel VI können mit Hilfe an sich bekannter herkömmlicher Verfahren hergestellt werden, z.B. in der nachstehend eingehender beschriebenen Weise.

Die Ausgangsmaterialien der Formel VII oder VII' sind Aminosäuren und deren Derivate, die aus dem Stand der Technik bekannt sind oder nach bekannten Methoden hergestellt werden können. Es ist erwähnenswert, dass die optisch aktiven erfindungsgemässen Verbindungen ausgehend von einer optisch aktiven Verbindung der Formel VII oder VII', z.B. von L-α-Aminophenylbuttersäure, L-Phenylalanin, L-Tryptophan, L-Methionin, L-Asparaginsäure, L-Threonin, L-Glutaminsäure, L-Lysin, L-Ornithin, und deren Derivaten, hergestellt werden können.

Das Verfahren d) wird auch in herkömmlicher Weise unter den allgemeinen Bedingungen der Solvolyse durchgeführt, die dafür bekannt sind, dass sie Cyanide (Nitrile) in die freien Carbonsäuren oder ihre Salze, Ester oder Amide überführen. Für die Umwandlung in eine freie Säure wird die Hydrolyse mit Wasser vorteilhaft in einem inerten organischen Lösungsmittel, das zumnindest teilweise mit Wasser mischbar ist, wie Ether (z.B. Diethyl- oder Diisopropylether, 1,2-Dimethoxyethan oder insbesondere Dioxan oder Tetrahydrofuran), oder einem Niederalkanol (z.B. Methanol, Ethanol, Isopropylalkohol, einem Butylalkohol, insbesondere tert.-Butylalkohol) durchgeführt, wobei eine grössere Menge Wasser in den letztgenannten Fällen erforderlich ist, um eine Alkoholyse zu verhindern. Die Hydrolyse kann sowohl durch starke Säuren, insbesondere anorganische Säuren, wie Schwefelsäure, oder vorzugsweise Halogenwasserstoffsäuren (z.B. Bromwasserstoffsäure oder als erste wahl Chlorwasserstoffsäure), als auch durch Basen, insbesondere anorganische Basen, wie Hydroxide und Carbonate der Alkalimetalle, z.B. Natrium- und Kaliumhydroxid, katalysiert werden. Die Basen werden gewöhnlich in zumindest stöchiometrischen Anteilen verwendet, wobei Carbonsäuresalze als primäre Produkte entstehen. Die sauren Katalysatoren werden zur Erzielung des besten Resultats vorteilhaft in Form einer verdünnten wässrigen Lösung angewandt. Die Endprodukte der Formel I, worin R° eine veresterte Carboxylgruppe bedeutet, können erhalten werden, indem man die Solvolyse des Nitrils mit dem entsprechenden Alkohol (Alkoholyse) in Gegenwart einer katalytische Menge einer wasserfreien starken Säure, vorteilhaft von gasförmigem Chlorwasserstoff, durchführt. Gewöhnlich wird ein Ueberschuss an Alkohol als Lösungsmittel verwendet, jedoch können inerte organische Lösungsmittel, wie acyclische und cyclische Ether (insbesondere die vorstehend erwähnten), und/oder halogenierte Niederalkane (insbesondere

Chloroform und Dichlormethan) zugegeben werden. Wird die Alkoholyse unter streng wasserfreien Bedingungen durchgeführt, muss das primäre Produkt (Imidoester), vorteilhaft durch Zugabe von Wasser, hydrolysiert werden. Andererseits wird, wenn man die Alkoholyse in Gegenwart eines annähernd stöchiometrischen Aequivalents von Wasser durchführt, der gewünschte Ester direkt erhalten. Um ein entsprechendes Amid (eine Verbindung der Formel I, worin R° Carbamoyl bedeutet) zu erhalten, kann ein entsprechendes Nitril der Formel VIII vorzugsweise einer alkalischen Hydrolyse in Gegenwart von Wasserstoffperoxid unterzogen werden.

Die Ausgangsmaterialien der Formel VIII können mit Hilfe an sich bekannter herkömmlicher Methoden, z.B. durch eine Kondensation analog derjenigen des Verfahrens c), erhalten werden, wobei ein Ausgangsmaterial der Formel VI mit einem Amin der Formel

$$R^5\text{-NH-}\underset{}{\overset{R^1}{\overset{|}{\text{CH}}}}\text{-CN} \qquad (\text{VII}'),$$

worin $R^1$ und $R^5$ die vorstehend angegebenen Bedeutungen besitzen, behandelt wird. Auch die Verfahren a) und b) können in analoger Weise zur Herstellung von Nitrilen der Formel VIII verwendet werden.

Die Cyclisierung entsprechend der Verfahrensvariante e) kann auch in an sich bekanter Weise, z.B. durch Dehydratation, durchgeführt werden. Besonders geeignete allgemeine Methoden für diesen Zweck sind die im Zusammenhnang mit der Bildung der Amidbindung in Peptiden entwickelten, wie sie in Sammelwerken, z.B. Houben-Weyl, Band 15/I und Band 15/II, wie vorstehend zitiert, angegeben werden. Gemäss einer bevorzugten Abwandlung wird die zu cyclisierende Aminogruppe durch Protonierung (d.h. in Form eines Säureadditionssalzes) inaktiv gemacht und die Carboxylgruppe in einen aktivierten Ester, wie einen solchen mit 2,4,5-Trichlorphenol, Pentachlorphenol, Pentafluorphenol, 2-Nitrophenol oder insbesondere 4-Nitrophenol, oder mit einer N-Hydroxyverbindung, wie N-Hydroxysuccinimid, 1-Hydroxybenztriazol oder N-Hydroxypiperidin, oder alternativ mit einem N,N'-disubstituierten Isohnarnstoff, wie insbesondere N,N'-Dicyclohexylisoharnstoff, oder einem analogen allgemein bekannten aktivierenden Mittel, übergeführt. Die Cyclisierung wird durchgeführt, indem man vorzugsweise durch Zugabe einer organischen Base, z.B. eines quaternären Ammoniumsalzes oder insbesondere eines tertiären Amins, wie Triethylamin, N-Ethylmorpholin oder N-Methylpiperidin, basisch macht, um die zu cyclisierende Aminogruppe durch Umwandlung in die unprotonierte Form zu reaktivieren. Die Reaktionstemperatur beträgt gewöhnlich von −20 bis +50°C, vorzugsweise annähernd Raumtemperatur, und es werden übliche Lösungsmittel verwendet, z.B. Dioxan, Tetrahydrofuran, Acetonitril, Pyridin, Dimethylformamid, Dimethylacetamid, Dimethylsulfoxid, N-Methylpyrrolidon, Hexamethylphosphorsäuretrisamid, sowie Chloroform und Methylenchlorid und bewährte Mischungen derselben. Bei einer speziellen Variante des Verfahrens kann die Carboxygruppe direkt in situ durch Einwirken der freien Säure mit einem Carbodiimid, wie N,N'-Dicyclohexylcarbodiimid (gegebenenfalls unter Zusatz von N-Hydroxysuccinimid, eines unsubstituierten oder beispielsweise Halogen-, Methyl- oder Methoxysubstituierten 1-Hydroxybenztriazols oder von 4-Hydroxybenzo-1,2,3-triazin-3-oxid oder N-Hydroxy-5-norbornen-2,3-dicarboximid) oder mit N,N'-Carbonyldiimidazol aktiviert werden.

Die Ausgangsmaterialien der Formel IX können nach an sich bekannten allgemeinen Methoden, z.B. wie sie nachstehend anhand spezieller Beispiele erörtert werden, hergestellt werden.

Auch die Reduktion entsprechend dem Verfahren f) kann in einer Weise durchgeführt werden, wie sie an sich allgemein für die Sättigung derartiger Doppelbindungen bekannt ist. Im einzelnen kann sich die Doppelbindung in dem ungesättigten Ausgangsmaterial, das der Formel I entspricht, zwischen C—3 und C—4, zwischen C—3 und dem benachbarten Stickstoffatom, oder zwischen diesem Stickstoffatom und dem benachbarten Kohlenstoffatom innerhalb der Gruppe $R^A$ befinden. Die Sättigung der Doppelbindungen wird vorteilhaft durch katalytische Hydrierung, beispielsweise unter den im einzelnen vorstehend erörterten bevorzugten Bedingungen, sowie auch durch Metallreduktion, wie Zinkreduktion, in neutralem oder saurem Medium oder insbesondere im Fall der C-N-Doppelbindung durch Diboran oder komplexe Hydride, wie Natriumborhydrid, wie vorstehend erwähnt, durchgeführt. Die ungesättigten Ausgangsmaterialien für diese Verfahrensvariante werden nach allgemein bekannten Methoden, z.B. den bei den Verfahren a) und c) und/oder in einer speziellen Form, wie nachstehend erörtert, erhalten.

Die Kondensation gemäss Verfahren g) wird unter üblichen allgemeinen Bedingungen, bei Temperaturen zwischen 0° und 100°C, in einem gegenüber den Reaktanten inerten Lösungsmittel, z.B. Methylenchlorid, 1,2-Dimethoxyethan, N,N-Dimethylformamid, vorzugsweise in Anwesenheit einer Base, z.B. eines tertiären Amines, wie Triethylamin, oder eines Alkalimetallhydrides, wie Natriumhydrid, durchgeführt.

Bei der Durchführung der fakultativen Umwandlung eines erhaltenen Endprodukts der Formel I in eine andere Verbindung der Formel I werden Umwandlungen wie die folgenden durchgeführt: eine Aminogruppe wird alkyliert, und/oder eine Oxogruppe, insbesondere diejenige des Symbols X, wird in eine Hydroxygruppe plus Wasserstoff oder in zwei Wasserstoffatome durch Reduktion umgewandelt, und/oder Hydroxy wird in Oxo umgewandelt durch Oxidation oder in Wasserstoff durch Reduktion, und/oder eine freie Hydroxy- oder Carboxygruppe wird aus ihrer veresterten Form durch Hydrolyse oder Hydrogenolyse freigesetzt, und/oder eine Hydroxy- oder Aminogruppe wird acyliert, und/oder ein freies Carboxyl wird

, verestert, und/oder der aromatische carbocyclische Ring in der Formel I wird hydriert zu Hexahydro oder zu 6,7,8,9-Tetrahydro, und/oder der Hexahydro-carbocyclische Ring wird dehydriert zu dem 6,7,8,9-Tetrahydro- oder dem aromatischen carbocyclischen Ring. Alle diese fakultativen Umwandlungen werden nach gut bekannten herkömmlichen Methoden durchgeführt.

Durch die Alkylierungsreaktion kann z.B. das durch $R^5$ dargestellte Niederalkyl in das Endprodukt der Formel I eingeführt werden, worin $R^5$ Wasserstoff bedeutet, wobei sämtliche der im einzelnen vorher erörterten Abwandlungen angewandt werden können. Sowohl die substitutive als auch die reduktive Alkylierung können angewandt werden, die erstgenannte mit Alkylhalogeniden, die letztgenannte mit niedrigen aliphatischen Aldehyden und Ketonen und katalytisch aktiviertem Wasserstoff oder im Fall von Formaldehyd vorteilhaft mit Ameisensäure als Reduktionsmittel. Durch die substitutive Alkylierung können Niederalkylgruppen auch in eine durch das Symbol $R^o$ dargestellte Carbamoylgruppe eingeführt werden.

Die Verbindungen der Formel I oder IA und die Zwischenprodukte hierfür, worin X Oxo bedeutet, können in die entsprechenden Verbindungen, worin X ein Wasserstoffatom und eine Hydroxygruppe bedeutet, durch Reduktion, z.B. katalytische Hydrierung, z.B. mit Wasserstoff in Gegenwart eines Platinkatalysators, oder mit einem Metallhydrid-Reduktionsmittel, wie Natriumborhydrid, oder gemäss der Meerwein-Ponndorf-Methode oder einer Abwandlung derselben unter Verwendung eines Alkanols, insbesondere Isopropylalkohol, sowohl als Lösungsmittel als auch als Reduktionsmittel und eines Metallalkoholats, vorzugsweise eines dem reduzierenden Alkohol entsprechenden, wie Aluminiumisopropylat, als Katalysator, übergeführt werden. Die Reduktion der Oxogruppe zu zwei Wasserstoffatomen kann vorteilhaft z.B. durch Behandlung mit amalgamiertem Zink und Chlorwasserstoffsäure oder durch Raney-Nickel-Desulfurierung eines entsprechenden Dithioketals bewirkt werden. Erhaltene Verbindungen, worin X ein Wasserstoffatom und eine Hydroxygruppe bedeutet, können in die Verbindungen, worin X zwei Wasserstoffatome bedeutet, z.B. durch katalytische Hydrierung des Addukts eines Carbodiimids, beispielsweise des Addukts durch Kondensation einer Verbindung, worin X ein Wasserstoffatom und eine Hydroxygruppe bedeutet, mit Dicyclohexylcarbodiimid in Gegenwart von Cuprochlorid gemäss der allgemeinen, in Chem. Ber. *107*, 1353 (1974) beschriebenen Methode übergeführt werden.

Alternativ können die Verbindungen, worin X ein Wasserstoffatom und eine Hydroxygruppe bedeutet, zuerst in die entsprechenden Verbindungen, worin X ein Wasserstoffatom und eine acylierte Hydroxygruppe (oder Acyloxy z.B. Acetoxy) bedeutet, übergeführt und anschliessend z.B. durch katalytische Hydrierung in Gegenwart eines Palladiumkatalysators, zu Verbindungen reduziert werden, worin X zwei Wasserstoffatome bedeutet. Die Oxidation von Hydroxy zur Bildung von Oxo kann vorzugsweise mit einem Derivat des sechswertigen Chroms, wie Chromsäure und deren Salze, mit einem Permanganatsalz (insbesondere Kaliumpermanganat) oder unter den Bedingungen der Oppenauer-Oxidation mit Aceton oder Cyclohexanon als Oxidationsmittel und Aluminiumisopropylat als Katalysator durchgeführt werden. Veresterte Hydroxygruppen werden insbesondere mit Hilfe der vorstehend im einzelnen im Zusammenhang mit der Abspaltung der Hydroxyschutzgruppen erörterten Methoden freigesetzt. Die Acylierung von Hydroxygruppen wird auf übliche Weise, vorzugsweise unter Verwendung eines entsprechenden Säureanhydrids oder Halogenids, durchgeführt.

Der aromatische carbocyclische Ring in Verbindungen der Formel I oder in den Zwischenprodukten für die Herstellung von Verbindungen der Formel I wird in die Hexahydro-Form z.B. durch Hydrierung bei atmosphärischem oder höherem Druck, in Gegenwart eines Katalysators (wie Platin oder Rhodium), bei Zimmertemperatur oder erhöhter Temperatur, in einem polaren Lösungsmittel, z.B. Ethanol, umgewandelt.

Verbindungen der Formel I oder IA, worin $R^1$ Amino-niederalkyl bedeutet, können in die Verbindungen, worin $R^1$ für Acylaminoniederalkyl steht, oder umgekehrt, gemäss den aus dem Stand der Technik bekannten und vorher im Zusammenhang mit den Schutzgruppen beschriebenen Methoden, übergeführt werden.

Die freien Carbonsäuren der Formel I oder IA, worin $R^6$ und/oder $R^7$ Hydroxy bedeuten oder deren Salze, können mit geeigneten aus dem Stand der Technik gut bekannten Alkoholen oder deren reaktiven Derivaten, oder mit einem Diazoalkan, insbesondere Diazomethan, verestert werden, um die entsprechenden Mono- oder Di-ester, nämlich die Verbindungen der Formel I oder IA zu ergeben, worin $R^6$ und/oder $R^7$ Niederalkoxy, Aryl-niederalkoxy, Niederalkanoyloxymethoxy oder Niederalkoxycarbonyl-niederalkoxy bedeuten. Alternativ kann die Carboxylgruppe in ein reaktives Derivat derselben, wie einen aktivierten Ester, z.B. einen solchen mit 2,4,5-Trichlorphenol, Pentachlorphenol, Pentafluorphenol, 2-Nitrophenol oder insbesondere 4-Nitrophenol, oder mit einer N-Hydroxy-Verbindung, z.B. N-Hydroxysuccinimid, 1-Hydroxybenztriazol oder N-Hydroxypiperidin, oder alternativ mit einem N,N'-disubstituierten Isoharnstoff, wie insbesondere N,N'-Dicyclohexylisoharnstoff, oder in ein gemischtes Anhydrid, z.B. ein Säurehalogenid (z.B. insbesondere Säurechlorid) umgewandelt werden. Dieses aktivierte Zwischenprodukt wird dann mit dem gewünschten Alkohol umgesetzt. Weiter kann die freie Carbonsäure über reaktionsfähige Zwischenprodukte in die Mono- oder Di-amide der Formel I, worin $R^6$ und/oder $R^7$ Amino, Mono- oder Di-niederalkylamino bedeuten, umgewandelt werden.

Die freie Carboxylgruppe kann aus einem veresterten Carboxyl in allgemein bekannter Weise, insbesondere durch Basen-katalysierte Hydrolyse, freigesetzt werden. Von speziellem Interesse sind jedoch Methoden, nach denen es möglich ist, selektiv eine spezielle durch die Symbole $—COR^6$ und $—COR^7$

wiedergegebene Carboxylgruppe freizusetzen. In einem derartigen Fall kann von einer geeigneten Kombination von Estergruppen Gebrauch gemacht werden, die im Stand der Technik insbesondere als Carboxylschutzgruppen bekannt und in grosser Vielfalt insbesondere für die Synthese von peptiden entwickelt worden sind, vgl. Houben-Weyl, Band 15/I und Band 15/II wie vorstehend zitiert. Für die selektive Abspaltung unter Freisetzung der Carboxylgruppe geeignete Reste sind Ester, die sich z.B. von Alkoholen, die durch Acidolyse entfernbare Reste ergeben, ableiten, wie Cyanmethylalkohol, Benzoylmethylalkohol oder tert.-Butylalkohol, jedoch insbesondere von Alkoholen, die Reste ergeben, die abgespalten werden können durch Reduktion, wie 2,2,2-Trichlorethanol, Benzylalkohol, und insbesondere 4-Nitrobenzylalkohol, oder alternativ Isonicotinalkohol. Eine besonders bevorzugte Klasse von substituierten Alkanolen sind Ethylalkohole, die in β-Stellung eine trisubstituierte Silylgruppe enthalten, wie Triphenylsilyl, Dimethyl-tert.-butylsilyl oder insbesondere Trimethylsilyl. Wie beispielsweise in der BE—PS 851 576 beschrieben, sind diese Alkohole besonders zur selektiven, Entfernung geeignet, da die entsprechenden β-Silylethylester, beispielsweise β-(Trimethylsilyl)-ethylester, die Stabilität herkömmlicher Alkylester besitzen, jedoch selektiv unter milden Bedingungen durch Einwirken von Fluorid-Ionen unter Erhalt anderer veresterter Carboxylgruppen, z.B. von Alkoxycarbonylgruppen, abgespalten werden können.

Die Abspaltung der veresternden Gruppen hängt von ihrer Natur ab und wird in jedem Fall in herkömmlicher, an sich bekannter weise durchgeführt, wobei man die Eigenschaften der anderen beteiligten Reste mit berücksichtigt. Die Gruppen, die durch Reduktion abgespalten werden können, insbesondere diejenigen, die halogenierte Niederalkylreste (beispielsweise 2,2,2-Trichlorethylreste), Isonicotinylreste (z.B. Isonicotinyloxycarbonyl) und gegebenenfalls substituierte Benzylreste, vor allem 4-Nitrobenzylreste jeglicher Art enthalten, werden bevorzugt durch Reduktion mit Zink, gewöhnlich in Gegenwart einer Säure, vorzugsweise Essigsäure, und mit oder ohne Zusatz eines inerten organischen Lösungsmittels, gewöhnlich bei Raumtemperatur abgespalten, diejenigen des Benzyl-Typs, insbesondere unsubstituierte Benzylester, werden auch durch Hydrogenolyse-Techniken, wie sie üblicherweise für Benzylgruppen angewandt werden, z.B. mit Wasserstoff in Gegenwart einer Katalysators, z.B. Palladium, entfernt.

Die Umwandlung der Verbindungen der Formel I oder IA, worin $R^6$ und/oder $R^7$ Niederalkoxy, Aryl-niederalkoxy, Amino, Mono- oder Di-(niederalkyl)-amino bedeuten, in die Verbindungen der Formel I oder IA, worin $R^6$ und/oder $R^7$ Hydroxy bedeuten, wird vorteilhaft durchgeführt durch Hydrolyse mit anorganischen Säuren, wie Halogenwasserstoffsäuren, Trifluoressigsäure oder Schwefelsäure. Die β-Silylethylestergruppen werden vorzugsweise durch Fluorid-Ionen erzeugende Reagentien, z.B. Fluoride von quaternären organischen Basen, wie Tetraethylammoniumfluorid, abgespalten. Gruppen $COR^6$ und $COR^7$, die Basen-instabil sind, können vorsichtig durch rasches Einwirken einer wässrigen Natriumoder Kaliumbicarbonatlösung oder vorzugsweise von wässrigem Ammoniak in einem organischen Lösungsmittel, gewöhnlich bei Raumtemperatur, und/oder mit wässrigen Alkalien, vorzugsweise Alkalimetallhydroxiden, wie Lithium- oder Natriumhydroxid, entfernt werden. Die Estergruppen werden vorzugsweise unter den Reaktionsbedingungen der Beispiele oder unter analogen Bedingungen abgespalten.

Eine geeignete Kombination der Estergruppen kann bei den früheren Synthesestufen oder durch eine geeignete Wahl an Ausgangsmaterialien und Reaktanten ausgewählt werden, wobei z.B. eine selektiv abspaltbare Estergruppe mit einem Carboxyl eingeführt wird, das in der letzten Stufe freigesetzt wird.

Die Verbindungen der Formel I oder IA, worin $R^6$ und/oder $R^7$ Niederalkoxy bedeuten, können mit Ammoniak, Mono- oder Di-niederalkylaminen amidiert werden, um Verbindungen der Formel I oder IA zu ergeben, worin $R^6$ und/oder $R^7$ unsubstituiertes oder Mono- oder Di-niederalkyl-Amino bedeuten.

Die Verbindungen der Formel I oder IA, worin weder $R^6$ noch $R^7$ Hydroxy bedeuten, können in Monocarbonsäuren der Formel I oder IA übergeführt werden, worin einer der Reste $R^6$ und $R^7$ Hydroxy bedeutet. Eine derartige Umwandlung wird durch selektive hydrolytische oder hydrogenolytische Verfahren, die aus dem Stand der Technik gut bekannt sind und sich nach dem chemischen Charakter der $R^6$- und $R^7$-Substituenten richten, durchgeführt.

Die vorstehenden Reaktionen werden nach Standard-Methoden in Gegenwart oder Abwesenheit von Verdünnungsmitteln, vorzugsweise solchen, die gegenüber den Reagentien inert sind und deren Lösungsmittel darstellen, von Katalysatoren, Kondensationsmitteln bzw. den anderen Mitteln und/oder in inerter Atmosphäre, bei niedrigen Temperaturen, Raumtemperatur oder erhöhten Temperaturen, vorzugsweise beim Siedepunkt der verwendeten Lösungsmittel, bei atmosphärischem oder überatmosphärischem Druck, durchgeführt.

Die Erfindung umfasst weiterhin jegliche Variante der vorliegenden Verfahren, bei der ein bei irgendeiner Stufe derselben erhältliches Zwischenprodukt als Ausgangsmaterial verwendet wird und die verbliebenen Stufen ausgeführt werden, oder das Verfahren bei irgendeiner Stufe desse ben unterbrochen wird oder bei der die Ausgangsmaterialien unter den Reaktionsbedingungen gebildet werden oder bei der die Reaktionskomponenten in Form ihrer Salze oder optisch reinen Antipoden verwendet werden. Hauptsächlich diejenien Ausgangsmaterialien sollten bei diesen Reaktionen verwendet werden, die zur Bildung der vorstehend als besonders wertvoll angegebenen Verbindungen führen.

Die Erfindung betrifft auch neue Ausgangsmaterialien und Verfahren zu deren Herstellung.

In Abhängigkeit von der Wahl der Ausgangsmaterialien und Methoden können die neuen Verbindungen in Form eines der möglichen Isomeren oder von deren Gemischen vorliegen, beispielsweise

in Abhängigkeit von der Anzahl der asymmetrischen Kohlenstoffatome, als reine optische Isomere, wie als Antipoden, oder als Gemische von optischen Isomeren, wie als Racemate, oder als Gemische von Diastereoisomeren.

Erhaltene Gemische der Diastereoisomeren und Gemische der Racemate könnenwegen der physikochemischen Unterschiede der Bestand-teile in bekannter Weise in die reinen Isomeren, Diastereoisomeren oder Racemate getrennt werden, z.B. durch Chromatographie und/oder fraktionierte Kristallisation.

Die erhaltenen Racemate können weiterhin in die optischen Antipoden nach an sich bekannten Methoden, beispielsweise durch Umkristallisieren aus einem optisch aktiven Lösungsmittel, mit Hilfe von Mikroorganismen oder durch Umsetzung eines sauren Endprodukts mit einer optisch aktiven Base, die Salze mit der racemischen Säure bildet, und Trennung der auf diese Weise erhaltenen Salze, beispielsweise aufgrund ihrer unterschiedlichen Löslichkeiten, in die Diastereoisomeren, aus denen die Antipoden durch Einwirken geeigneter Mittel freigesetzt werden können, aufgetrennt werden. Basische racemische Produkte können gleichfalls in die Antipoden, z.B. durch Trennung von deren diastereoisomeren Salzen, z.B. durch fraktionierte Kristallisation von deren d- oder l-Tartraten, getrennt werden. Irgendwelche racemischen Zwischenprodukte oder Ausgangsmaterialien können in ähnlicher Weise getrennt werden.

Zweckmässigerweise wird der aktivere der beiden Antipoden isoliert.

Schliesslich werden die erfindungsgemässen Verbindungen entweder in freier Form oder in Form von deren Salzen erhalten. Irgendeine erhaltene Base kann in ein entsprechendes Säureadditionssalz, vorzugsweise unter Verwendung einer pharmazeutisch verträglichen Säure oder eines Anionenaustausch-Präparates, übergeführt werden, oder erhaltene Salze können in die entsprechenden freien Basen, beispielsweise unter Verwendung einer stärkeren Base, wie eines Metall- oder Ammoniumhydroxids oder eines basischen Salzes, z.B. eines Alkalimetallhydroxids oder -carbonats, oder eines Kationenaustausch-Präparats, übergeführt werden. Eine Verbindung der Formel I, worin $R^o$ Carboxy bedeutet, oder der Formel IA, worin $COR^6$ und/oder $COR^7$ Carboxy bedeuten, kann somit auch in die entsprechenden Metalloder Ammoniumsalze übergeführt werden. Diese oder andere Salze, beispielsweise die Pikrate, können auch für die Reinigung der erhaltenen Basen verwendet werden. Die Basen werden in die Salze übergeführt, die Salze werden getrennt, und die Basen werden aus den Salzen freigesetzt. Im Hinblick auf die enge Verwandtschaft zwischen den freien Verbindungen und den Verbindungen in Form ihrer Salze ist, wenn immer in diesem Zusammenhang auf eine Verbindung Bezug genommen wird, auch ein entsprechendes Salz mit umfasst, vorausgesetzt, dass dies unter den gegebenen Umständen möglich oder sinnvoll ist.

Die Verbindungen einschliesslich ihrer Salze können auch in Form ihrer Hydrate erhalten werden oder andere für die Kristallisation verwendete Lösungsmittel enthalten.

Die erfindungsgemässen pharmazeutischen präparate sind solche, die für die enterale, wie die orale oder rektale, und die parenterale Verabreichung an Säuger, einschliesslich des Menschen, zur Behandlung oder Verhinderung von Erkrankungen geeignet sind, die auf die Inhibierung des Angiotensin-umwandelnden Enzyms ansprechen, z.B.von kardiovaskulären Erkrankungen, wie der Hypertension, und von kongestiver Herzinsuffizienz und umfassen eine wirksame Menge einer pharmakologisch aktiven Verbindung der Formel I oder eines pharmazeutisch verträglichen Salzes derselben, allein oder in Kombination mit einem oder mehreren pharmazeutisch verträglichen Trägern.

Die pharmakologisch aktiven Verbindungen der Erfindung sind bei der Herstellung von pharmazeutischen Zusammensetzungen verwendbar, die eine wirksame Menge derselben in Verbindung oder Beimischung mit Excipienten oder Trägern, die für die enterale oder parenterale Anwendung geeignet sind, umfassen. Bevorzugt sind Tabletten und Gelatinekapseln, die den aktiven Bestandteil zusammen mit a) Verdünnungsmitteln, z.B. Lactose, Dextrose, Sucrose, Mannit, Sorbit, Cellulose und/oder Glycin, b) Gleitmitteln, z.B. Siliciumdioxid, Talk, Stearinsäure, deren Magnesium- oder Calciumsalz und/oder Polyethylenglykol, für Tabletten auch c) Bindemitteln, z.B. Magnesiumaluminiumsilikat, Stärkepaste, Gelatine, Tragant, Methylcellulose, Natriumcarboxymethylcellulose und/oder Polyvinylpyrrolidon, gewünschtenfalls d) Zerteilungs- bzw. Desintegrationsmitteln, z.B. Stärken, Agar, Alginsäure oder deren Natriumsalz, oder schäumenden Mischungen und/oder e) Absorbentien, farbgebenden Mitteln, Geschmacksstoffen und süssenden Mitteln umfassen. Injizierbare Präparate sind vorzugsweise wässrige isotonische Lösungen oder Suspensionen, und Suppositorien werden vorteilhaft aus Fettemulsionen oder -suspensionen hergestellt. Diese Zusammensetzungen können sterilisiert werden und/oder Adjuvantien, wie Konservierungs-, Stabilisierungs-, Netz- oder Emulgiermittel, Lösungspromotoren, Salze für die Regulierung des osmotischen Drucks und/oder Puffer, enthalten. Zusätzlich können sie auch andere therapeutisch wertvolle Substanzen enthalten. Diese präparate werden nach herkömmlichen Misch-, Granulier- bzw. Ueberzugsmethoden hergestellt und enthalten etwa 0,1 bis 75%, vorzugsweise etwa 1 bis 50%, des aktiven Bestandteils. Eine Einheitsdosis für einen Säuger von etwa 50 bis 70 kg kann zwischen etwa 10 und 200 mg aktiven Bestandteil enthalten.

Die folgenden Beispiele sollen die Erfindung erläutern und keine Einschränkungen darstellen. Die Temperaturen sind in Celsiusgraden angegeben, und sämtliche Teile sind in Form von Gewichtsteilen angegeben. Wenn nicht anders erwähnt, werden sämtliche Verdampfungen unter vermindertem Druck vorzugsweise, zwischen etwa $20 \cdot 10^{-3}$ und $133 \cdot 10^{-3}$ bar (zwischen etwa 15 und 100 mg Hg), durchgeführt.

Im Fall der Verbindungen der Formel I oder IA, worin mehr als ein Asymmetrie-Zentrum vorliegt, werden die erhaltenen diastereoisomeren Verbindungen als A, B etc. in den betreffenden Beispielen

bezeichnet. Die jeweiligen diastereoisomeren Verbindungen werden durch die physikalischen Eigenschaften, z.B. den Schmelzpunkt oder den Drehwert, charakterisiert.

Im Fall der Verbindungen der Formel I oder IA, worin X zwei Wasserstoffatome bedeutet und ein Asymmetrie-Zentrum in der Seitenkette an dem das Stickstoff tragenden Kohlenstoffatom vorliegt, wurden die Symbole A und B wie folgt den jeweiligen Isomeren auf Basis ihrer relativen Wanderung bei der Chromatographie zugeordnet. Auf Basis der Wanderung bei der Dünnschichtchromatographie und Normalphasen-Hochdruck-Flüssigkeits-Chromatographie unter Verwendung von Siliciumdioxidgel als stationäre Phase wird das sich schnell bewegende Isomere als Isomer A und das sich langsam bewegende Isomere als Isomer B bezeichnet. Auf Basis der Wanderung bei der Hochdruck-Flüssigkeits-Chromatographie mit reverser phase wird das sich langsam bewegende Isomere als Isomer A und das sich schnell bewegende Isomere als Isomer B bezeichnet.

Beispiel 1

5-Acetoxy-1-carboxymethyl-3-(S)-(1-ethoxycarbonyl-3-phenylpropylamino)-2,3,4,5-tetrahydro-1H-1-benzazepin-2-on Hydrochlorid

Eine Lösung von 4 g 5-Acetoxy-3-(S)-amino-1-carboxymethyl-2,3,4,5-tetrahydro-1H-1-benzazepin-2-on Trifluoracetat und 6,1 g Benzylbrenztraubensäureethylester in 30 ml Methanol und 30 ml Essigsäure wird 1 Stunde bei Raumtemperatur gerührt. Eine Lösung von 0,75 g Natriumcyanborhydrid in 10 ml Methanol wird dann tropfenweise während 7 Stunden zugegeben. Der Ansatz wird für weitere 18 Stunden bei Raumtemperatur gerührt. Nach Zugabe von 4 ml konzentrierter Salzsäure wird eine weitere Stunde gerührt. Die Lösungsmittel werden unter vermindertem Druck abgezogen und der Rückstand zwischen 200 ml Eis/Wasser und 50 ml Ether verteilt. Der pH wird mit 40%iger Natronlauge auf 9,3 eingestellt, und die Etherschicht verworfen. Der pH der wässrigen Lösung wird mit konzentrierter Salzsäure auf 4,3 eingestellt, und die Lösung wird mit 3 × 225 50 ml Essigester extrahiert. Die vereinigten Essigesterlösungen werden über Natriumsulfat getrocknet und unter vermindertem Druck eingeengt. Der Rückstand wird mit 50 ml Essigester gekocht, auf Raumtemperatur abgekühlt, filtriert und ergibt die Titelverbindung, Fp. 215—217°.

Die Ausgangsverbindung wird folgendermassen hergestellt:

Eine Lösung von 5 g 3-(S)-t-Butoxycarbonylamino-1-t-butoxycarbonylmethyl-5-acetoxy-2,3,4,5-tetrahydro-1H-1-benzazepin-2-on in 60 ml Trifluoressigsäure wird 1 Stunde bei Raumtemperatur unter Stickstoffatmosphäre gerührt. Das Lösungsmittel wird unter vermindertem Druck abgezogen und der Rückstand mit Ether gewaschen. Man erhält das Acetoxyaminosäuretrifluoracetat, das ohne weitere Reinigung weiter verwendet wird.

Beispiel 2

a) Eine Lösung von 5 g 1-Ethoxycarbonylmethyl-2,3,4,5-tetrahydro-1H-1-benzazepin-2,3-dion, 4,4 g S-Benzyl-L-cysteinethylester und 380 mg Dibutylzinndichlorid in 250 ml Chloroform wird in einem Wasserabscheider 18 Stunden unter Rückfluss gekocht. Das Lösungsmittel wird unter vermindertem Druck entfernt, man erhält das rohe Imin. Diese Verbindung wird in 250 ml Methanol gelöst, und 1,4 g Natriumcyanborhydrid und 45 ml Eisessig werden zugegeben. Der Ansatz wird bei Raumtemperatur 18 Stunden unter Stickstoffatmosphäre gerührt. 15 ml konzentrierte Salzsäure werden zugegeben, und der Ansatz wird weitere 30 Minuten bei Raumtemperatur gerührt. Die Lösungsmittel werden unter vermindertem Druck abgezogen, und der Rückstand wird zwischen 500 ml verdünntem wässrigen Ammoniak und 300 ml Ether verteilt. Die wässrige Phase wird weiterhin mit 2 × 150 ml Ether extrahiert und die vereinigten etherischen Lösungen werden mit 200 ml gesättigter Natriumchloridlösung gewaschen, sodann mit 200 ml 2N Salzsäure und 200 ml gesättigtem Natriumchlorid. Die etherische Lösung wird über Natriumsulfat getrocknet, das Lösungsmittel unter vermindertem Druck abgezogen, man erhält das rohe Produkt als Isomerengemisch. Dieses Material wird durch Blitzchromatographie unter Verwendung von Essigester/Toluol (1:4) gereinigt. Man erhält 1-Ethoxycarbonylmethyl-3-[1-ethoxycarbonyl-2-benzylthio-(1R)-ethylamino]-2,3,4,5-tetrahydro-1H-1-(3R,S)-benzazepin-2-on (als Mischung der R,R und R,S Isomeren).

Das α-Ketolactamausgangsmaterial kann folgendermassen hergestellt werden:

5,0 kg 3-Azido-2,3,4,5-tetrahydro-1H-1-benzazepin-2-on werden unter Stickstoff portionsweise zu 3,03 kg Kaliumtert.-butylat in 50 Litern trockenem Tetrahydrofuran so zugegeben, dass die Temperatur unter 5° bleibt, der Ansatz wird eine Stunde gerührt, nachdem die Zugabe beendet ist. Eine Lösung von 4,38 kg Bromessigsäureethylester in 5 Litern Tetrahydrofuran wird dann langsam so zugegeben, dass die Temperatur unter 5° bleibt. Der Ansatz wird dann über Nacht bei Raumtemperatur aufbewahrt. 1,5 kg Hiflo-Filteraid wird zugegeben, und der Ansatz wird filtriert. Der Filterkuchen wird mit Tetrahydrofuran gewaschen und die vereinigten Tetrahydrofuranlösungen werden zur Trockne eingeengt. Man erhält 3-Azido-1-ethoxycarbonylmethyl-2,3,4,5-tetrahydro-1H-1-benzazepin-2-on, das ohne weitere Reinigung für die nächste Stufe verwendet wird. Eine Mischung von 13,98 kg 3-Azido-1-ethoxycarbonylmethyl-2,3,4,5-tetrahydro-1H-1-benzazepin-2-on und 1,3 kg 5 %iges Palladium-auf-Kohle in 57 Litern wasserfreiem Ethanol wird 5 Stunden unter einem Druck von 3 Atmosphären Wasserstoff hydriert. Das Druckgefäss wird in stündlichen Intervallen belüftet, um den angesammelten Stickstoff entweichen zu lassen. Der Katalysator wird abfiltriert und mit Ethanol gewaschen. Die Lösung wird zur Trockne eingeengt. Man erhält 3-Amino-1-ethoxycarbonylmethyl-2,3,4,5-tetrahydro-1H-1-benzazepin-2-on.

31 ml t-Butylnitrit werden unter Rühren zu einer Lösung von 55 g 3-Amino-1-ethoxycarbonylmethyl-2,3,4,5-tetrahydro-1H-1-benzazepin-2-on in 1000 ml Chloroform und 2,8 ml Essigsäure gegeben. Der Ansatz wird 3,5 Stunden unter Stickstoff unter Rückfluss gekocht, und dann auf 0° abgekühlt. Unter Rühren werden während einer halben Stunde 43,5 g *m*-Chlorperbenzoesäure in 5 Portionen zugegeben. Man lässt den Ansatz auf Raumtemperatur erwärmen und rührt für weitere 1,5 Stunden. Der Ansatz wird mit 500 ml wässrigem Natriumbicarbonat, 2 × 250 ml konzentrierter wässeriger Ammoniaklösung und 250 ml gesättigter Natriumchloridlösung gewaschen. Die organische Lösung wird über Natriumsulfat getrocknet, mit Aktivkohle behandelt und unter vermindertem Druck eingeengt. Man erhält ein Oel, das nach Behandlung mit Ether das 1-Ethoxycarbonylmethyl-2,3,4,5-tetrahydro-1H-1-benzazepin-2,3-dion ergibt, Fp. 112—114°.

b) Auf ähnliche Weise wird 1-Ethoxycarbonylmethyl-3-[1-ethoxycarbonyl-2-phenethylthio-(1R)-ethylamino]-2,3,4,5-tetrahydro-1H-1-(3R,S)-benzazepin-2-on hergestellt, wobei man von (S)-S-phenethylcysteine (U.S. Patent 3,950,542) ausgeht.

c) Auf ähnliche Weise wird 1-Ethoxycarbonylmethyl-3-[1-ethoxycarbonyl-2-phenylthio-(1R)-ethylamino]-2,3,4,5-tetrahydro-1H-1-benzazepin-2-on ausgehend von (S)-S-Phenylcystein [S. H. Zbarsky und L. Young, J. Biol. Chem. *151*, 211 (1943)] hergestellt.

d) Auf ähnliche Weise wird 1-Ethoxycarbonylmethyl-3-[1-ethoxycarbonyl-3-methylthio-(1S)-propylamino]-2,3,4,5-tetrahydro-1H-1-benzazepin-2-on ausgehend von L-Methioninethylester hergestellt.

e) Auf ähnliche Weise wird 1-Ethoxycarbonylmethyl-3-[1-ethoxycarbonyl-2-benzyloxy-(1S)-ethylamino]-2,3,4,5-tetrahydro-1H-1-(3S,R)-benzazepin-2-on ausgehend von O-Benzyl-L-serin hergestellt.

Beispiel 3

a) Zu einer Lösung von 2,1 g 1-Ethoxycarbonylmethyl-3-[1-ethoxycarbonyl-2-benzylthio-(1R)-ethylamino]-2,3,4,5-tetrahydro-1H-1-benzazepin-2-on in 30 ml Methanol wird eine wässrige Natriumhydroxidlösung gegeben (2N, 4,8 ml, 2,1 Mol-Aequivalente), und die Lösung wird bei Raumtemperatur 18 Stunden gerührt. Das Lösungsmittel wird unter vermindertem Druck abgezogen, und der Rückstand wird zwischen 100 ml Wasser und 100 ml Essigester verteilt. Die wässrige Phase wird mit 2 × 50 ml Essigester extrahiert und dann mit konzentrierter Salzsäure auf pH 3 eingestellt. Ein Oel scheidet sich ab. 50 ml Essigester werden zugegeben und die Mischung wird 30 Minuten bei Raumtemperatur gerührt. Man erhält kristallines 1-Carboxymethyl-3-[1-carboxy-2-benzylthio-(1R)-ethylamino]-2,3,4,5-tetrahydro-1H-1-(3S)-benzazepin-2-on, Fp. 213—215° (Zersetzung), $[\alpha]_D = -207,2°$ (c = 1.0, DMF).

b) Auf ähnliche Weise hergestellt wird 1-Carboxymethyl-3-[1-carboxy-2-phenylethylthio-(1R)-ethylamino]-2,3,4,5-tetrahydro-1H-1-(3S)-benzazepin-2-on, Fp. 197—199° (Zersetzung), $[\alpha]_D = -46,1°$ (c = 0.83, DMF).

c) Auf ähnliche Weise hergestellt wird 1-Carboxymethyl-3-[1-carboxy-2-phenylthio-(1R)-ethylamino]-2,3,4,5-tetrahydro-1H-1-(3S)-benzazepin-2-on, Fp. 206—208° (Zersetzung), $[\alpha]_D = -110,8°$ (c = 0.72, DMF).

d) Auf ähnliche Weise hergestellt wird 1-Carboxymethyl-3-[1-carboxy-3-methylthio-(1S)-propylamino]-2,3,4,5-tetrahydro-1H-1-(3S)-benzazepin-2-on, Fp. 236°, $[\alpha]_D = -169°$ (c = 1.1, Ethanol).

e) Auf ähnliche Weise hergestellt wird 1-Carboxymethyl-3-[1-carboxy-2-benzyloxy-(1S)-ethylamino]-2,3,4,5-tetrahydro-1H-1-(3S,R)-benzazepin-2-on Hydrochlorid, Fp. 68—70° (Zersetzung), $[\alpha]_D = 42°$ (c = 1, Methanol).

Beispiel 4

a) Zu einer Lösung von 1,0 g 1-Ethoxycarbonylmethyl-3-[1-ethoxycarbonyl-2-benzylthio-(1R)-ethylamino]-2,3,4,5-tetrahydro-1H-1-(3R,S)-benzazepin-2-on in 30 ml Ethanol wird eine wässrige Kaliumhydroxydlösung (2N, 1 ml, 0,95 Mol-Aequivalente) gegeben, und die Mischung wird bei Raumtemperatur 18 Stunden gerührt. Die Lösungsmittel werden unter vermindertem Druck abgezogen, und 30 ml Wasser wird zu dem Rückstand gegeben. Die Lösung wird mit 2 × 30 ml Ether extrahiert, die wässrige Phase mit konzentrierter Salzsäure angesäuert und mit 3 × 20 ml Essigester extrahiert. Die vereinigten Essigesterlösungen werden über Magnesiumsulfat getrocknet, eingeengt, und der Rückstand wird in 35 ml Dichlormethan aufgenommen. Diese Lösung wird mit trockenem Chlorwasserstoffgas gesättigt und das Lösungsmittel unter vermindertem Druck abgezogen. Der Rückstand wird mit Ether behandelt. Man erhält das 1-Carboxymethyl-3-[1-ethoxycarbonyl-2-benzylthio-(1R)-ethylamino]-2,3,4,5-tetrahydro-1H-1-(3R,S)-benzazepin-2-on Hydrochlorid, Fp. 188—185°, $[\alpha]_D = -13,2°$ (c = 1.1, Ethanol), als eine Mischung zweier Diastereoisomerer.

b) Auf ähnliche Weise ergibt die Hydrolyse von 1-Ethoxycarbonylmethyl-3-[1-ethoxycarbonyl-2-phenylthio-(1R)-ethylamino]-2,3,4,5-tetrahydro-1H-1-(3R,S)-benzazepin-2-on das 1-Carboxymethyl-3-[1-ethoxycarbonyl-2-phenylthio-(1R)-ethylamino]-2,3,4,5-tetrahydro-1H-1-(3S)-benzazepin-2-on Hydrochlorid, Fp. 120—126° (Zersetzung), $[\alpha]_D = 58,6°$, (c = 0.81, Ethanol).

c) Auf ähnliche Weise ergibt die Hydrolyse von 1-Ethoxycarbonylmethyl-3-[1-ethoxycarbonyl-3-methylthio - (1S) - propylamino] - 2,3,4,5 - tetrahydro - 1H - 1 - benzazepin - 2 -on das 1 - Carboxymethyl - 3 - [1 - ethoxycarbonyl - 3 - methylthio - (1S) - propylamino] - 2,3,4,5 - tetrahydro - 1H - 1 - (3S) - benzazepin - 2 - on Hydrochlorid, Fp. 196—197°.

d) Auf ähnliche Weise wird das 1-Carboxymethyl-3-[1-ethoxycarbonyl-2-benzyloxy-(1S)-ethylamino]-2,3,4,5-tetrahydro-1H-1-(3R,S)-benzazepin-2-on Hydrochlorid, Fp. 198—201°, $[\alpha]_D = 3°$ (c = 1, Methanol) erhalten.

Beispiel 5

Unter Verwendung von Verfahren, die den vorstehend beschriebenen entsprechen, mit N-(ε)-Benzoyl-L-lysin als Ausgangsmaterial wird das 1-Carboxymethyl-3-[1-ethoxycarbonyl-5-benzoylamino-(1S)-pentylamino]-2,3,4,5-tetrahydro-1H-1-(3S)-benzazepin-2-on Hydrochlorid, Fp. 107—109°, $[\alpha]_D = -80°$ (c = 1, Methanol) hergestellt.

Beispiel 6

Die folgenden Verbindungen der Formel IA, in denen X zwei Wasserstoffatome bedeutet, $R^2$ bis $R^5$ Wasserstoff bedeuten, $R^6$ Hydroxy oder Ethoxy und $R^7$ Hydroxy ist, können nach den Verfahren der vorstehenden Beispiele hergestellt werden, insbesondere deren S,S-Isomere.

| Verbindung | $R^1$ | Derivat |
|---|---|---|
| 6a | Ethoxycarbonylmethyl | |
| 6b | Methylthioethyl | |
| 6c | 4-Benzoylaminobutyl | |
| 6d | 4-(Benzyloxycarbonylamino)-butyl | Hexahydro |
| 6e | Phenoxyethyl | |
| 6f | Phenylthioethyl | |
| 6g | Hydroxymethyl | |

Ausgangsmaterialien für:

6a - L-Asparaginsäurediethylester

6b - Methioninethylester

6c - N-Benzoyl-L-lysinethylester

6d - L-N-Benzyloxycarbonyl-L-lysinethylester

6e - 4-Phenoxy-2-aminobuttersäureethylester

6f - Phenylthio-2-aminobuttersäureethylester

6g - L-Serinethylester

Beispiel 7

a) Zu einer Lösung von 1,5 g 3-[(5-Benzyloxycarbonylamino-1-methoxycarbonyl)-pentylamino]-1-ethoxycarbonylmethyl-2,3,4,5-tetrahydro-1H-1-benzazepin-2-on (Isomer A) in 60 ml Methanol werden bei 0° 5 ml 5 %iger Natronlauge zugegeben und der Ansatz wird bei Raumtemperatur 18 Stunden gerührt. Die Lösung wird mit 2N Salzsäure angesäuert und zur Trockne eingeengt. 25 ml Ethanol werden zugegeben und die Lösung wird zur Trockne eingeengt. Der zurückbleibende weisse Festkörper wird mit 30 ml Methylenchlorid gerührt, und der zurückbleibende Feststoff abfiltriert. Dieses Material wird mit 30 ml Methylenchlorid gerührt und abfiltriert. Die vereinigten Methylenchloridlösungen werden eingedampft, der zurückbleibende Festkörper mit Ether behandelt und filtriert. Man erhält das 3-[(5-Benzyloxycarbonylamino-1-carboxy)-pentylamino]-1-carboxymethyl-2,3,4,5-tetrahydro-1H-1-benzazepin-2-on Hydrochlorid (Isomer A), Fp. 123—126°, $[\alpha]_D = 106°$ (c = 1, Methanol).

b) Auf ähnliche Weise ergibt 3-[(5-Benzyloxycarbonylamino-l-methoxycarbonyl)-pentylamino]-1-ethoxycarbonylmethyl-2,3,4,5-tetrahydro-1H-1-benzazepin-2-on (Isomer B) das 3-[(5-Benzyloxycarbonylamino-1-carboxy)-pentylamino]-1-carboxymethyl-2,3,4,5-tetrahydro-1H-l-benzazepin-2-on Hydrochlorid (Isomer B), Fp. 107—110°, $[\alpha]_D = -88°$ (c = 1.26, Methanol), dem die S,S-Stereochemie zuzuordnen ist.

Das Ausgangsmaterial wird folgendermassen hergestellt:

Eine Lösung von 8 g 3-Amino-1-ethoxycarbonylmethyl-2,3,4,5-tetrahydro-1H-1-benzazepin-2-on, 0,4 ml Essigsäure und 4,5 ml t-Butylnitrit in 160 ml Chloroform wird 2 Stunden unter Rückfluss erhitzt und auf Raumtemperatur abgekühlt. 6,0 g *m*-Chlorperbenzoesäure werden portionsweise unter Rühren zugegeben, und es wird während 30 Minuten weitergerührt. Die Lösung wird mit 100 ml gesättigter wässriger Natriumbicarbonatlösung, 50 ml 2N Salzsäure und 50 ml wasser gewaschen und über Magnesiumsulfat getrocknet. Das Lösungsmittel wird unter vermindertem Druck abgezogen und der Rückstand mit Essigester/Petrolether (Kp 60—80°) behandelt. Man erhält als gelben Festkörper das α-Ketolactam 1-Ethoxycarbonylmethyl-2,3,4,5-tetrahydro-1H-1-benzazepin-2,3-dion, Fp. 108—110°, das ohne weitere Reinigung in der nächsten Stufe verwendet wird.

Zu einer Lösung von 11 g 1-Ethoxycarbonylmethyl-2,3,4,5-tetrahydro-1H-1-benzazepin-2,3-dion, 14 g N-ε-Benzyloxycarbonyllysinmethylesterhydrochlorid, 6 ml Triethylamin und 0,7 g Dibutylzinndichlorid in 600 ml Methylenchlorid werden 50 g 4 Å Molekularsieb gegeben. Der Ansatz wird 40 Stunden gerührt und unter Rückfluss gekocht. Nach Abkühlen auf Raumtemperatur wird der Ansatz durch Celite filtriert und das Lösungsmittel unter vermindertem Druck abgezogen. Der Rückstand wird in 750 ml Methanol und 28 ml Essigsäure aufgenommen.

Nach 5 Minuten werden 3,6 g Natriumcyanborhydrid zugegeben und der Ansatz wird 72 Stunden bei Raumtemperatur gerührt. Dann wird durch Zugabe von 10 ml konzentrierter Salzsäure angesäuert. Die Lösung wird zur Trockne eingeengt. Man erhält ein Oel, das durch Blitzchromatographie unter Verwendung von Essigester/Toluol (9:1) aufgetrennt wird. Es werden zwei Fraktionen erhalten:

a) 3-[(5-Benzyloxycarbonylamino-1-methoxycarbonyl)-pentylamino]-1-ethoxycarbonylmethyl-2,3,4,5-tetrahydro-1H-1-benzazepin-2-on (Isomer A); $R_f$ = 0,7, NMR ($CDCl_3$) 1,47 (m, 5H), 3,16 (m, 4H).

b) 3-[5-Benzyloxycarbonylamino-1-methoxycarbonyl)-pentylamino]-1-ethoxycarbonylmethyl-2,3,4,5-tetrahydro-1H-1-benzazepin-2-on (Isomer B); $R_f$ = 0,6, NMR ($CDCl_3$) 1,30 (m, 5H), 3,06 (m, 4H).

## Beispiel 8

Eine Lösung von 22,6 g 3-[(5-Benzyloxycarbonylamino-1-methoxycarbonyl)-(1S)-pentylamino]-1-ethoxycarbonylmethyl-2,3,4,5-tetrahydro-1H-1-(3S)-benzazepin-2-on (Isomer B) und 84 ml 1N Natronlauge in 580 ml Methanol wird 19 Stunden unter Stickstoffatmosphäre bei Raumtemperatur gerührt. Die Lösung wird eingeengt und der Rückstand zwischen 300 ml Wasser und 300 ml Ether verteilt. Die organische Schicht wird abgetrennt und die wässrige Schicht einmal mit 300 ml Ether gewaschen. Die wässrige Schicht wird dann mit konzentrierter Salzsäure auf pH 2 eingestellt, wonach das produkt langsam auskristallisiert. Dieser Festkörper wird durch Vakuumfiltration gesammelt, intensiv mit Wasser gewaschen und getrocknet. Man erhält 3-[(5-Benzyloxycarbonylamino-l-carboxy)-(1S)-pentylamino]-1-carboxymethyl-2,3,4,5-tetrahydro-1H-1-(3S)-benzazepin-2-on, Fp. 216—218° (Zersetzung).

## Beispiel 9

Trockenes Chlorwasserstoffgas wird durch eine Lösung von 2,8 g 3-[(5-Benzyloxycarbonylamino-1-t-butyloxycarbonyl)-(1S)-pentylamino]-1-ethoxycarbonylmethyl-2,3,4,5-tetrahydro-1H-1-(3S)-benzazepin-2-on in 150 ml Essigester eingeleitet, bis die dünnschichtchromatographische Analyse zeigt, dass kein Ausgangsmaterial zurückgeblieben ist. Das Lösungsmittel wird unter vermindertem Druck abgezogen und der Rückstand mit Ether behandelt. Man erhält das 3-[(5-Benzyloxycarbonylamino-1-carboxy)-(1S)-pentylamino]-1-ethoxycarbonylmethyl-2,3,4,5-tetrahydro-1H-1-(3S)-benzazepin-2-on Hydrochlorid, Fp. 124—126° (Zersetzung), $[\alpha]_D = -116°$ (c = 1, Methanol).

Das Ausgangsmaterial wird wie folgt hergestellt:

Das für die Herstellung verwendete 3-[(5-Benzyloxycarbonylamino-1-t-butoxycarbonyl)-(1S)-pentylamino]-1-ethoxycarbonylmethyl-2,3,4,5-tetrahydro-1H-l-(3S)-benzazepin-2-on wird entsprechend der in Beispiel 7 beschriebenen Methode durch Kondensation von 1-Ethoxycarbonylmethyl-2,3,4,5-tetrahydro-1H-1-benzazepin-2,3-dion und N-ε-Benzyloxycarbonyllysin-t-butylester hergestellt.

Das dazu verwendete 1-Ethoxycarbonylmethyl-2,3,4,5-tetrahydro-1H-1-benzazepin-2,3-dion kann auch folgendermassen hergestellt werden:

5,0 kg 3-Azido-2,3,4,5-tetrahydro-1H-1-benzazepin-2-on werden portionsweise unter Stickstoff zu 3,03 kg Kaliumtert.-butylat in 50 Litern trockenem Tetrahydrofuran so zugegeben, dass die Temperatur unter 5° bleibt. Der Ansatz wird nach beendeter Zugabe noch eine Stunde gerührt. Eine Lösung von 4,38 kg Bromessigsäureethylester in 5 Litern Tetrahydrofuran wird dann langsam so zugegeben, dass die Temperatur unter 5° bleibt. Der Ansatz wird dann über Nacht bei Raumtemperatur gehalten. 1,5 kg Hiflo-Filteraid wird hinzugegeben und der Ansatz wird filtriert. Der Filterkuchen wird mit Tetrahydrofuran gewaschen, die vereinigten Tetrahydrofuranlösungen werden zur Trockne eingeengt. Man erhält 3-Azido-1-ethoxycarbonylmethyl-2,3,4,5-tetrahydro-1H-1-benzazepin-2-on, das ohne weitere Reinigung für die nächste Stufe eingesetzt wird.

Eine Mischung von 13,98 kg 3-Azido-1-ethoxycarbonylmethyl-2,3,4,5-tetrahydro-1H-1-benzazepin-2-on und 1,3 kg 5% Palladium-auf-Kohle in 57 Litern wasserfreiem Ethanol wird 5 Stunden bei einem Druck von 3 Atmosphären Wasserstoff hydriert. Das Druckgefäss wird stündlich belüftet, um angesammelten

Stickstoff entweichen zu lassen. Der Katalysator wird abfiltriert und mit Ethanol gewaschen. Die Lösung wird zur Trockne eingeengt. Man erhält 3-Amino-1-ethoxycarbonylmethyl-2,3,4,5-tetrahydro-1H-1-benzazepin-2-on.

31 ml t-Butylnitrit werden unter Rühren zu einer Lösung von 55 g 3-Amino-1-ethoxycarbonylmethyl-2,3,4,5-tetrahydro-1H-1-benzazepin-2-on in 1000 ml Chloroform und 2,8 ml Essigsäure gegeben. Der Ansatz wird unter Stickstoff 3,5 Stunden unter Rückfluss gekocht und dann auf 0° abgekühlt. Unter Rühren werden während einer halben Stunde 43,5 g *m*-Chlorperbenzoesäure in 5 Portionen zugegeben. Man lässt den Ansatz auf Raumtemperatur kommen und weitere 1,5 Stunden rühren. Der Ansatz wird mit 500 ml gesättigter wässriger Natriumbicarbonatlösung, 2 × 250 ml konzentrierter wässriger Ammoniaklösung und 250 ml gesättigter wässriger Kochsalzlösung gewaschen. Die organische Lösung wird über Natriumsulfat getrocknet, mit Aktivkohle behandelt und unter vermindertem Druck eingeengt. Man erhält ein Oel, das mit Ether behandelt wird und so 1-Ethoxycarbonylmethyl-2,3,4,5-tetrahydro-1H-1-benzazepin-2,3-dion Fp. 112—114° ergibt.

Beispiel 10

3 - [(5 - Benzyloxycarbonylamino - 1 - methoxycarbonyl) - (1S) - pentylamino] - 1 - benzyloxycarbonylmethyl-2,3,4,5 - tetrahydro - 1H - 1 - (3S) - benzazepin - 2 - on Hydrochlorid, Fp. 61—63°, $[\alpha]_D = -58°$ (c = 1, Methanol) wird nach den in den vorangegangenen Beispielen beschriebenen Methoden durch Kondensation von N-ε-Benzyloxycarbonyllysinmethylester und 1-Benzyloxycarbonylmethyl-2,3,4,5-tetrahydro-1H-1-benzazepin-2,3-dion, Fp. 106—108 hergestellt. Das α-Ketolactam wird unter Anwendung von vorstehend beschriebenen Verfahren durch Oxidation von 3-Amino-1-benzyloxycarbonylmethyl-2,3,4,5-tetrahydro-1H-1-benzazepin-2-on hergestellt.

Beispiel 11

Das folgende Verfahren wird für die selektive Hydrolyse von 3-[(5-Benzyloxycarbonylamino)-pentylamino]-diestern zu der entsprechenden 1-Carboxymethylverbindung verwendet:

Der Diester wird in Ethanol gelöst und mit einem Mol Aequivalent Kalilauge behandelt, der Ansatz wird bei Raumtemperatur 1 Stunde gerührt. Das Lösungsmittel wird unter vermindertem Druck abgezogen, und Wasser wird zu dem Rückstand gegeben. Die wässrige Lösung wird mit Ether gewaschen, mit Salzsäure angesäuert, um die freie Aminosäure herzustellen, die durch Extraktion isoliert und in das Hydrochlorid überführt wird.

Die folgenden Verbindungen werden hergestellt:

a) 3-[(5-Benzyloxycarbonylamino-1-methoxycarbonyl)-(1S)-pentylamino]-1-carboxymethyl-2,3,4,5-tetrahydro-1H-1-(3S)-benzazepin-2-on Hydrochlorid, Fp. 102—104°, $[\alpha]_D = -116°$ (c = 1, Methanol) durch Hydrolyse des entsprechenden 1-Ethoxycarbonylmethylderivates.

b) 3-[(5-Benzyloxycarbonylamino-1-ethoxycarbonyl)-(1S)-pentylamino]-1-carboxymethyl-2,3,4,5-tetrahydro-1H-l-(3S)-benzazepin-2-on Hydrochlorid, Fp. 104—106°, $[\alpha]_D = -113°$ (c = 1, Methanol), durch Hydrolyse des entsprechenden 1-Ethoxycarbonylmethylderivates.

Beispiel 12

Herstellung von 10,000 Tabletten mit einem Gehalt von je 10 mg der aktiven Substanz des Beispiels 1:

| Zusammensetzung | |
|---|---|
| 5-Acetoxy-1-carboxymethyl-3-(S)-(1-ethoxycarbonyl-3-phenylpropylamino)-2,3,4,5-tetrahydro-1H-1-benzazepin-2-on Hydrochlorid | 100,00 g |
| Milchzucker | 1157,00 g |
| Maisstärke | 75,00 g |
| Polyethylenglykol 6000 | 75,00 g |
| Talkpulver | 75,00 g |
| Magnesiumstearat | 18,00 g |
| Gereinigtes Wasser | q.s |

Verfahren

Sämtliche pulvrigen Bestandteile werden mit einem Sieb von 0,6 mm Maschenweite gesiebt. Dann wird der Wirkstoff mit Milchzucker, Talk, Magnesiumstearat und mit der Hälfte der Stärke in einem geeigneten Mischer vermischt. Die andere Hälfte der Stärke wird in 40 ml Wasser suspendiert, und die

Suspension zur siedenden Lösung von Polyethylenglykol in 150 ml Wasser gegeben. Die erhaltene Paste wird zu den Pulvern gegeben und gegebenenfalls unter Zugabe einer weiteren Wassermenge granuliert. Das Granulat wird über Nacht bei 35° getrocknet, durch ein Sieb von 1,2 mm Maschenweite getrieben und zu Tabletten mit 6,4 mm Durchmesser, welche eine Bruchrille aufweisen, gepresst.

**Patentansprüche für die Vertragsstaaten: BE CH DE FR GB IT LI LU NL SE**

1. Verbindungen der allgemeinen Formel

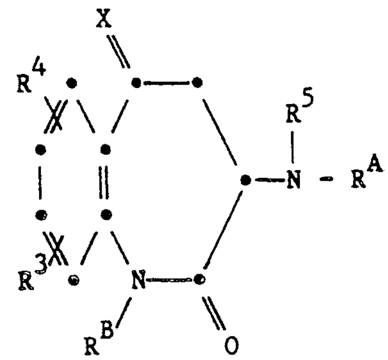

(I),

worin $R^A$ und $R^B$ Reste der Formel

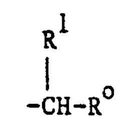

bzw.

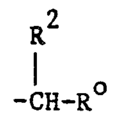

darstellen, worin R° Carboxy oder ein funktionell modifiziertes Carboxy darstellt; $R^1$ Wasserstoff, Niederalkyl, Amino-niederalkyl, Aryl, Aryl-niederalkyl, Cycloalkyl, Cycloalkyl-niederalkyl, Acylamino-niederalkyl, Mono- oder Di-niederalkylamino-niederalkyl, Niederalkylthio-niederalkyl, Carboxy-niederalkyl, verestertes Carboxy-niederalkyl, Carbamoyl-niederalkyl, N-substituiertes Carbamoyl-niederalkyl, Hydroxy-niederalkyl, verethertes oder acyliertes Hydroxy-niederalkyl, Aryloxy-niederalkyl, Aryl-(thio-, sulfinyl- oder sulfonyl-)-niederalkyl, Aryl-N-niederalkylamino-niederalkyl oder Arylamino-niederalkyl bedeutet; $R^2$ für Wasserstoff oder Niederalkyl steht; $R^3$ und $R^4$ unabhängig voneinander Wasserstoff, Niederalkyl, Niederalkoxy, Niederalkanoyloxy, Hydroxy, Halogen oder Trifluormethyl bedeuten, oder $R^3$ und $R^4$ gemeinsam für Niederalkylendioxy stehen; $R^5$ Wasserstoff oder Niederalkyl bedeutet; und X Oxo, zwei Wasserstoffatome oder Hydroxy oder acyliertes Hydroxy zusammen mit einem Wasserstoffatom bedeutet; und worin der carbocyclische Ring auch Hexahydro oder 6,7,8,9-Tetrahydro sein kann; mit der Massgabe, dass, wenn $R^1$ Wasserstoff, Niederalkyl, Amino-niederalkyl, Aryl, Aryl-niederalkyl, Cycloalkyl oder Cycloalkyl-niederalkyl ist, X acyliertes Hydroxy zusammen mit einem Wasserstoffatom bedeutet; Salze und Stereoisomere von allen diesen Verbindungen.

2. Verbindungen der Formel

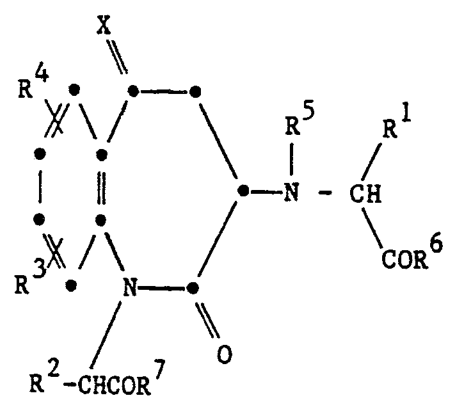

(IA),

worin der carbocyclische Ring auch Hexahydro sein kann; $R^1$ Wasserstoff, Niederalkyl, Amino-niederalkyl, Aryl, Aryl-niederalkyl, Cycloalkyl, Cycloalkyl-niederalkyl, Acylamino-niederalkyl, Mono- oder Di-niederalkylamino-niederalkyl, Niederalkylthio-niederalkyl, Carboxy-niederalkyl, verestertes Carboxy-niederalkyl, Carbamoyl-niederalkyl, N-substituiertes Carbamoyl-niederalkyl, Hydroxy-niederalkyl,

verethertes oder acyliertes Hydroxy-niederalkyl, Aryloxy-niederalkyl, Arylthio-niederalkyl, Aryl-N-niederalkylamino-niederalkyl oder Arylamino-niederalkyl bedeutet; $R^2$ und $R^5$ Wasserstoff oder Niederalkyl bedeuten; $R^3$ und $R^4$ Wasserstoff, Niederalkyl, Niederalkoxy, Niederalkanoyloxy, Hydroxy, Halogen oder Trifluormethyl bedeuten oder $R^3$ und $R^4$ gemeinsam Niederalkylendioxy darstellen; X Oxo, zwei Wasserstoffatome, oder eine Hydroxygruppe oder acylierte Hydroxygruppe und ein Wasserstoff (unter Berücksichtigung der in Anspruch 1 angegebenen Massgabe) bedeutet; $R^6$ und $R^7$ unabhängig voneinander Hydroxy, Amino, Mono-oder Di-niederalkylamino, Niederalkoxy, Aryl-niederalkoxy, Niederalkanoyloxymethoxy, (Amino, Mono- oder Di-niederalkylamino, Carboxy oder Niederalkoxy-carbonyl)-niederalkoxy bedeuten; oder Salze davon.

3. Verbindungen der im Anspruch 2 gezeigten Formel IA, worin $R^1$ Wasserstoff, Niederalkyl, Amino-niederalkyl, Aryl-niederalkoxy-carbonylamino-niederalkyl oder Aryl-niederalkyl bedeutet, worin Aryl für unsubstituiertes Phenyl oder durch Niederalkyl, Hydroxy, Niederalkoxy, Niederalkanoyloxy, Halogen oder Trifluormethyl monosubstituiertes Phenyl steht; $R^2$ und $R^5$ Wasserstoff oder Niederalkyl bedeuten; $R^3$ und $R^4$ für Wasserstoff, Niederalkyl, Niederalkoxy, Halogen oder Trifluormethyl stehen, oder $R^3$ und $R^4$ gemeinsam Niederalkylendioxy bedeuten; X für Oxo, zwei Wasserstoffatome, oder eine Hydroxy- oder Niederalkanoyloxygruppe und ein Wasserstoffatom steht (unter Berücksichtigung der in Anspruch 1 angegebenen Massgabe in einer Weise, die der hier für X genannten Bedeutung angepasst ist); $R^6$ und $R^7$ unabhängig voneinander für Hydroxy, Amino, Niederalkoxy, Phenyl-niederalkoxy oder Niederalkoxy-carbonyl-niederalkoxy stehen; oder ihre Salze; oder die genannten Verbindungen, worin der carbocyclische Ring Hexahydro ist.

4. Verbindungen der im Anspruch 2 gezeigten Formel IA, worin $R^1$ Wasserstoff, Niederalkyl, ω-Amino-niederalkyl, ω-Arylmethoxycarbonylamino-niederalkyl oder Aryl-niederalkyl bedeutet, worin Aryl für unsubstituiertes Phenyl oder durch Niederalkyl, Hydroxy, Niederalkoxy, Niederalkanoyloxy, Halogen oder Trifluormethyl monosubstituiertes Phenyl steht; $R^2$ und $R^5$ Wasserstoff oder Niederalkyl bedeuten; $R^3$ für Wasserstoff steht; $R^4$ Wasserstoff, Niederalkyl, Niederalkoxy, Halogen oder Trifluormethyl bedeutet; X für Oxo, zwei Wasserstoffatome, oder eine Hydroxy- oder Niederalkanoyloxygruppe und ein Wasserstoffatom steht (unter Berücksichtigung der in Anspruch 1 angegebenen Massgabe in einer Weise, die der hier für X genannten Bedeutung angepasst ist); $R^6$ und $R^7$ unabhängig voneinander für Hydroxy, Amino, Niederalkoxy, Phenyl-niederalkoxy, Niederalkoxy-carbonyl-niederalkoxy stehen; oder ihre Salze; oder die genannten Verbindungen, worin der carbocyclische Ring Hexahydro ist.

5. Verbindungen der im Anspruch 2 gezeigten Formel IA, worin $R^1$ die Bedeutung hat von Wasserstoff, Methyl, Ethyl, Isopropyl, ω-Aminopropyl, ω-Aminobutyl, ω-(Benzyloxycarbonylamino)propyl, ω-(Benzyloxycarbonylamino)butyl, Aryl-(methyl, ethyl, propyl), worin Aryl unsubstituiertes phenyl oder durch Methyl, Hydroxy, Methoxy, Methylendioxy, Acetoxy, Chlor oder Trifluormethyl monosubstituiertes Phenyl bedeutet; $R^2$ und $R^5$ für Wasserstoff oder Methyl stehen; $R^3$ und $R^4$ Wasserstoff, Methoxy, Methyl, Chlor oder Trifluormethyl bedeuten; X für Oxo, zwei Wasserstoffatome, oder eine Hydroxy- oder Acetoxygruppe und ein Wasserstoffatom steht (unter Berücksichtigung der in Anspruch 1 angegebenen Massgabe in einer Weise, die der hier für X genannten Bedeutung angepasst ist); $R^6$ und $R^7$ unabhängig voneinander Hydroxy, Amino, Ethoxy, Methoxy, Benzyloxy, Ethoxycarbonylmethoxy oder Pivaloyloxymethoxy bedeuten; oder ihre Salze; oder die genannten Verbindungen, worin der carbocyclische Ring Hexahydro ist.

6. Verbindungen der Formel

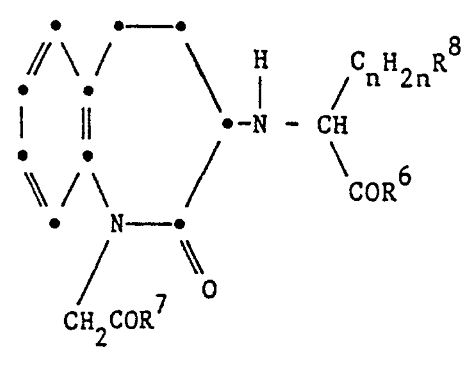

(IB),

worin n eine ganze Zahl von 1 bis 4 bedeutet, $R^8$ Benzyloxycarbonylamino bedeutet; $R^6$ und $R^7$ unabhängig voneinander Hydroxy, Niederalkoxy mit bis zu 4 Kohlenstoffatomen, Benzyloxy oder Amino bedeuten, oder ihre Salze; oder die genannten Verbindungen, worin der carbocyclische Ring Hexahydro ist.

7. Verbindungen der Formel

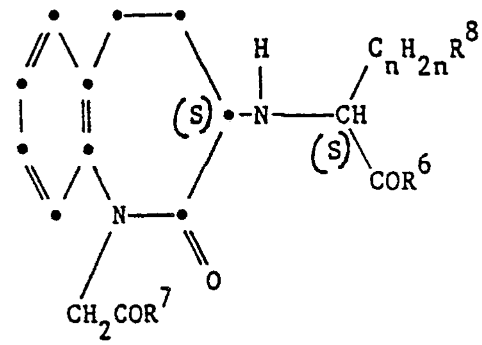

(IC),

worin S die Chiralität bedeutet, n eine ganze Zahl von 1 bis 4 bedeutet; $R^8$ Benzyloxycarbonylamino bedeutet; $R^6$ und $R^7$ unabhängig voneinander Hydroxy, Niederalkoxy mit bis zu 4 Kohlenstoffatomen, Benzyloxy oder Amino bedeuten; oder ihre Salze.

8. 5-Acetoxy-l-carboxymethyl-3-(S)-(1-ethoxycarbonyl-3-phenylpropylamino)-2,3,4,5-tetrahydro-1H-1-benzazepin-2-on oder ein Salz davon.

9. 3-[(5-Benzyloxycarbonylamino-l-carboxy)-pentylamino]-l-carboxymethyl-2,3,4,5-tetrahydro-1H-1-benzazepin-2-on oder ein Salz davon.

10. Pharmazeutische Präparate, enthaltend eine Verbindung gemaßseinen der Ansprüche 1 bis 9 oder ein pharmazeutisch annehmbares Salz davon zusammen mit einem pharmazeutisch geeigneten Trägermaterial.

11. Die in einem der Ansprüche 1 bis 9 genannten Verbindungen oder deren pharmazeutisch annehmbare Salze zur Anwendung in einen Verfahren zur therapeutischen Behandlung des menschlichen oder tierischen Körpers.

12. Die in einem der Ansprüche 1 bis 9 genannten Verbindungen oder deren pharmazeutisch annehmbare Salze als Hemmer des Angiotensin-umwandelnden Enzyms.

13. Verwendung der in einem der Ansprüche 1 bis 9 genannten Verbindungen oder ihrer pharmazeutisch anwendbaren Salze zur Herstellung von pharmazeutischen Präparaten.

14. Verfahren zur Herstellung von den in Anspruch 1 genannten Verbindungen der Formel I, in der alle Symbole die in Anspruch 1 angegebenen Bedeutungen haben, oder deren Salzen, dadurch gekennzeichnet, dass man

a) in eine Verbindung der Formel

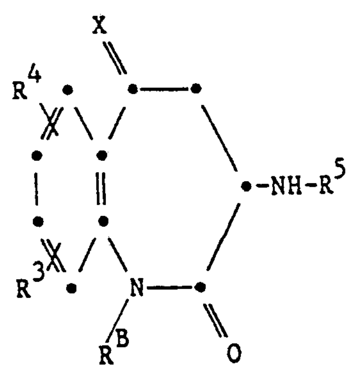

(II),

worin der carbocyclische Ring auch Hexahydro oder 6,7,8,9-Tetrahydro sein kann und worin X, $R^B$, $R^3$, $R^4$ und $R^5$ die vorstehend angegebenen Bedeutungen besitzen, $R^A$ durch Alkylierung mit einer Verbindung der Formel

$R^A—Z$ (IIIA)

worin Z eine reaktive veresterte Hydroxygruppe bedeutet und $R^A$ die vorstehend angegebenen Bedeutungen besitzt, oder mit einer Verbindung der Formel

$R^1—CO—R^o$ (IV),

worin $R^1$ und $R^o$ die oben angegebenen Bedeutungen haben, in Gegenwart eines Reduktionsmittels, unter

vorübergehendem Schutz etwaiger primärer und sekundärer Aminogruppen und/oder gegebenenfalls Hydroxy- und/oder Oxogruppen, die in irgendeinem der Reste X, $R^B$, $R^3$ und $R^4$ und/oder im Alkylierungsmittel anwesend sein können, einführt oder

b) eine Verbindung der Formel

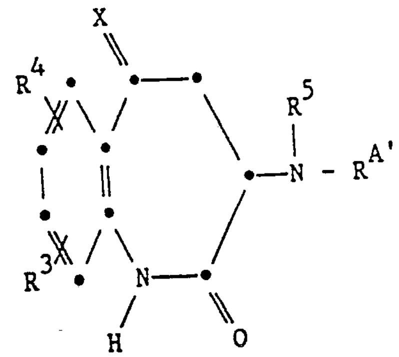

(V),

worin der carbocyclische Ring auch Hexahydro oder 6,7,8,9-Tetrahydro sein kann und worin X, $R^3$, $R^4$ und $R^5$ die vorstehend angegebenen Bedeutungen besitzen und $R^{A'}$ Wasserstoff oder $R^A$ wie vorstehend definiert ist, mit einer Verbindung der Formel

$$R^B—Z \qquad \text{(IIIB)},$$

worin Z eine reaktive veresterte Hydroxygruppe bedeutet und $R^B$ die vorstehend angegebenen Bedeutungen besitzt, alkyliert, wobei man vorübergehend etwaige primäre und sekundäre Aminogruppen und/oder gegebenenfalls Hydroxy- und/oder Oxogruppen, die in irgendeinem der Reste X, $R^{A'}$ $R^B$, $R^3$ und $R^4$ anwesend sein können, schützt oder

c) eine Verbindung der Formel

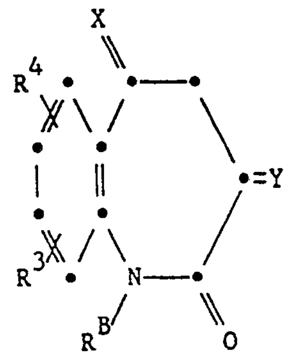

(VI),

worin der carbocyclische Ring auch Hexahydro oder 6,7,8,9-Tetrahydro sein kann und worin Y Oxo, Dichlor, oder eine reaktive veresterte Hydroxygruppe Z gemeinsam mit Wasserstoff bedeutet und X, $R^B$, $R^3$ und $R^4$ die vorstehend angegebenen Bedeutungen besitzen, mit einem Amin der Formel

$$R^A—NH—R^5 \qquad \text{(VII)},$$

worin $R^A$ und $R^5$ die vorstehend angegebenen Bedeutungen besitzen, kondensiert, wobei, wenn Y Oxo oder Dichlor bedeutet, die Kondensation in Gegenwart eines Reduktionsmittels und unter vorübergehendem Schutz der Oxogruppe, die als Substituent X anwesend sein kann, durchgeführt wird, oder

d) in einer Verbindung der Formel

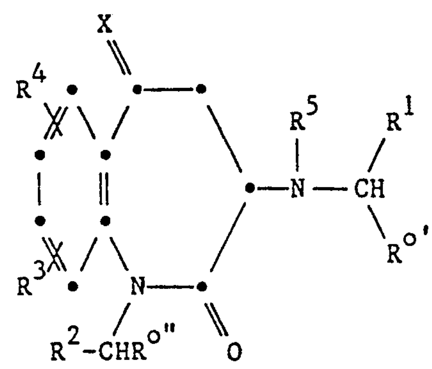

(VIII),

worin der carbocyclische Ring auch Hexahydro oder 6,7,8,9-Tetrahydro sein kann und worin X und $R^1$ bis $R^5$ die vorstehend angegebenen Bedeutungen besitzen, eines der Symbole $R^{o\prime}$ und $R^{o\prime\prime}$ Cyano ist und das andere Cyano oder $R^o$ wie vorstehend definiert ist, die Cyanogruppe(n) einer Solvolyse unterzieht oder

e) eine Verbindung der Formel

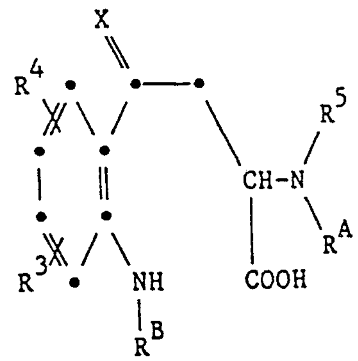

(IX),

worin der carbocyclische Ring auch Hexahydro oder 3,4,5,6-Tetrahydro sein kann und worin X, $R^A$, $R^B$, $R^3$, $R^4$ und $R^5$ die vorstehend angegebenen Bedeutungen besitzen, oder einen Ester hiervon cyclisiert oder

f) eine Verbindung, die strukturell identisch ist mit einer Verbindung der Formel I, wie oben definiert, ausser dass sie eine weitere Doppelbindung am C—3 oder zwischen dem Stickstoffatom und dem benachbarten Kohlenstoffatom innerhalb der Gruppe $R^A$ aufweist, mit einem Reduktionsmittel behandelt, um diese Doppelbindung zu sättigen, oder

g), um eine Verbindung der Formel I, wie vorstehend definiert, in der X Oxo bedeutet, herzustellen, eine Verbindung der Formel

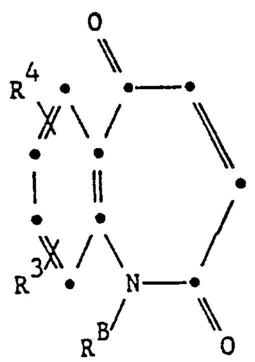

(X),

in der der Carbocyclus auch Hexahydro oder 6,7,8,9-Tetrahydro sein kann, und $R^B$, $R^3$ und $R^4$ die vorstehend angegebenen Bedeutungen haben, mit einem Amin der Formel

$$R^A—NH—R^5 \qquad (VII),$$

worin $R^A$ und $R^5$ die vorstehend angegebenen Bedeutungen haben, kondensiert und gewünschtenfalls eine erhaltene Verbindung der Formel I in eine andere Verbindung der Formel I umwandelt und/oder gewünschtenfalls eine erhaltene Verbindung der Formel I, die salzbildende Eigenschaften aufweist, in ihr Salz oder ein erhaltenes Salz in ein anderes Salz überführt oder eine freie Verbindung aus einem derartigen Salz freisetzt und/oder gewünschtenfalls ein optisches Isomeres, das eine spezifische Konfiguration im Hinblick auf zumindest ein Chiralitätszentrum besitzt, aus einer Mischung der stereoisomeren Formen einer erhaltenen Verbindung der Formel I anreichert.

15. Die nach dem Verfahren des Anspruchs 14 erhältlichen Verbindungen.

**Patentansprüche für den Vertragsstaat: AT**

1. Verfahren zur Herstellung von 3-Amino-l-benzazepin-2-on-l- alkansäuren der allgemeinen Formel

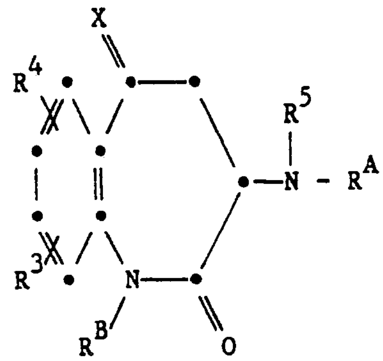

(I),

worin $R^A$ und $R^B$ Reste der Formel

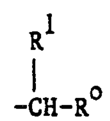 bzw. 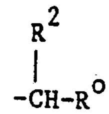

darstellen, worin R° Carboxy oder ein funktionell modifiziertes Carboxy darstellt; $R^1$ Wasserstoff, Niederalkyl, Amino-niederalkyl, Aryl, Aryl-niederalkyl, Cycloalkyl, Cycloalkyl-niederalkyl, Acylamino-niederalkyl, Mono- oder Di-niederalkylamino-niederalkyl, Niederalkylthio-niederalkyl, Carboxy-niederalkyl, verestertes Carboxv-niederalkyl, Carbamoyl-niederalkyl, N-substituiertes Carbamoyl-niederalkyl, Hydroxy-niederalkyl, verethertes oder acyliertes Hydroxy-niederalkyl, Aryloxy-niederalkyl, Aryl-(thio-, sulfinyl- oder sulfonyl-)-niederalkyl, Aryl-N-niederalkylamino-niederalkyl oder Arylamino-niederalkyl bedeutet; $R^2$ für Wasserstoff oder Niederalkyl steht; $R^3$ und $R^4$ unabhängig voneinander Wasserstoff, Niederalkyl, Niederalkoxy, Niederalkanoyloxy, Hydroxy, Halogen oder Trifluormethyl bedeuten, oder $R^3$ und $R^4$ gemeinsam für Niederalkylendioxy stehen; $R^5$ Wasserstoff oder Niederalkyl bedeutet; und X Oxo, zwei Wasserstoffatome oder Hydroxy oder acyliertes Hydroxy zusammen mit einem Wasserstoffatom bedeutet; und worin der carbocyclische Ring auch Hexahydro oder 6,7,8,9-Tetrahydro sein kann; mit der Massgabe, dass, wenn $R^1$ Wasserstoff, Niederalkyl, Amino-niederalkyl, Aryl, Aryl-niederalkyl, Cycloalkyl oder Cycloalkyl-niederalkyl ist, X acyliertes Hydroxy zusammen mit einem Wasserstoffatom bedeutet; Salze und Stereoisomere von allen diesen Verbindungen, dadurch gekennzeichnet, dass man

a) in eine Verbindung der Formel

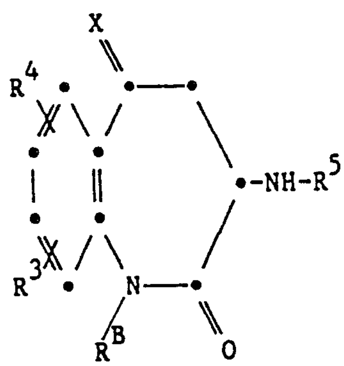

(II),

worin der carbocyclische Ring auch Hexahydro oder 6,7,8,9-Tetrahydro sein kann und worin X, $R^B$, $R^3$, $R^4$ und $R^5$ die vorstehend angegebenen Bedeutungen besitzen, $R^A$ durch Alkylierung mit einer Verbindung der Formel

$$R^A{-}Z \qquad (IIIA),$$

worin Z eine reaktive veresterte Hydroxygruppe bedeutet und $R^A$ die vorstehend angegebenen Bedeutungen besitzt, oder mit einer Verbindung der Formel

$$R^1{-}CO{-}R° \qquad (IV),$$

worin $R^1$ und $R^o$ die oben angegebenen Bedeutungen haben, in Gegenwart eines Reduktionsmittels, unter vorübergehendem Schutz etwaiger primärer und sekundärer Aminogruppen und/oder gegebenenfalls Hydroxy- und/oder Oxogruppen, die in irgendeinem der Reste X, $R^B$, $R^3$ und $R^4$ und/oder im Alkylierungsmittel anwesend sein können, einführt oder

b) eine Verbindung der Formel

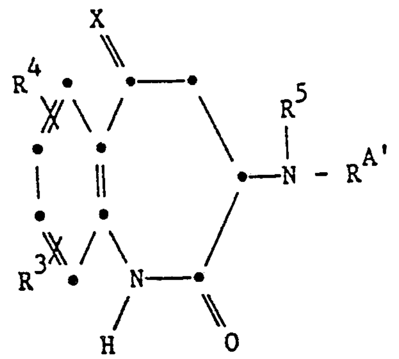

(V),

worin der carbocyclische Ring auch Hexahydro oder 6,7,8,9-Tetrahydro sein kann und worin X, $R^3$, $R^4$ und $R^5$ die vorstehend angegebenen Bedeutungen besitzen und $R^{A'}$ Wasserstoff oder $R^A$ wie vorstehend definiert ist, mit einer Verbindung der Formel

$$R^B\text{—}Z \qquad \text{(IIIB)},$$

worin Z eine reaktive veresterte Hydroxygruppe bedeutet und $R^B$ die vorstehend angegebenen Bedeutungen besitzt, alkyliert, wobei man vorübergehend etwaige primäre und sekundäre Aminogruppen und/oder gegebenenfalls Hydroxy- und/oder Oxogruppen, die in irgendeinem der Reste X, $R^{A'}$ $R^B$, $R^3$ und $R^4$ anwesend sein können, schützt oder

c) eine Verbindung der Formel

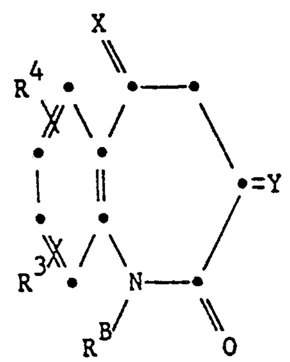

(VI),

worin der carbocyclische Ring auch Hexahydro oder 6,7,8,9-Tetrahydro sein kann und worin Y Oxo, Dichlor, oder eine reaktive veresterte Hydroxygruppe Z gemeinsam mit Wasserstoff bedeutet und X, $R^B$, $R^3$ und $R^4$ die vorstehend angegebenen Bedeutungen besitzen, mit einem Amin der Formel

$$R^A\text{—NH—}R^5 \qquad \text{(VII)},$$

worin $R^A$ und $R^5$ die vorstehend angegebenen Bedeutungen besitzen' kondensiert, wobei, wenn Y Oxo oder Dichlor bedeutet, die Kondensation in Gegenwart eines Reduktionsmittels und unter vorübergehendem Schutz der Oxogruppe, die als Substituent X anwesend sein kann, durchgeführt wird, oder

d) in einer Verbindung der Formel

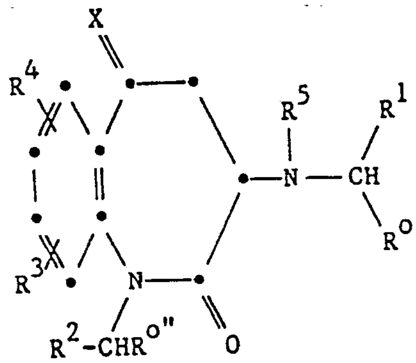

(VIII),

worin der carbocyclische Ring auch Hexahydro oder 6,7,8,9-Tetrahydro sein kann und worin X und $R^1$ bis $R^5$ die vorstehend angegebenen Bedeutungen besitzen, eines der Symbole $R^{o\prime}$ und $R^{o\prime\prime}$ Cyano ist und das andere Cyano oder $R^o$ wie vorstehend definiert ist, die Cyanogruppe(n) einer Solvolyse unterzieht oder

e) eine Verbindung der Formel

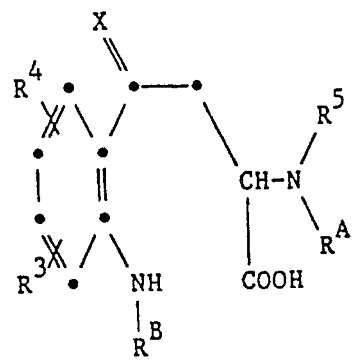

(IX),

worin der carbocyclische Ring auch Hexahydro oder 3,4,5,6-Tetrahydro sein kann und worin X, $R^A$, $R^B$, $R^3$, $R^4$ und $R^5$ die vorstehend angegebenen Bedeutungen besitzen, oder einen Ester hiervon cyclisiert oder

f) eine Verbindung, die strukturell identisch ist mit einer Verbindung der Formel I, wie oben definiert, ausser dass sie eine weitere Doppelbindung am C—3 oder zwischen dem Stickstoffatom und dem benachbarten Kohlenstoffatom innerhalb der Gruppe $R^A$ aufweist, mit einem Reduktionsmittel behandelt, um diese Doppelbindung zu sättigen, oder

g) um eine Verbindung der Formel I, wie vorstehend definiert, in der X Oxo bedeutet, herzustellen, eine Verbindung der Formel

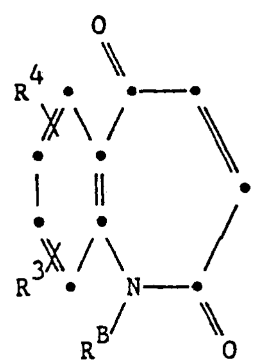

(X),

in der der Carbocyclus auch Hexahydro oder 6,7,8,9-Tetrahydro sein kann, und $R^B$, $R^3$ und $R^4$ die vorstehend angegebenen Bedeutungen haben, mit einem Amin der Formel

$$R^A—NH—R^5 \qquad (VII),$$

worin $R^A$ und $R^5$ die vorstehend angegebene, Bedeutungen haben, kondensiert und gewünschtenfalls eine erhaltene Verbindung der Formel I in eine andere Verbindung der Formel I umwandelt und/oder gewünschtenfalls eine erhaltene Verbindung der Formel I, die salzbildende Eigenschaften aufweist, in ihr Salz oder ein erhaltenes Salz in ein anderes Salz überführt oder eine freie Verbindung aus einem derartigen Salz freisetzt und/oder gewünschtenfalls ein optisches Isomeres, das eine spezifische Konfiguration im Hinblick auf zumindest ein Chiralitätszentrum besitzt, aus einer Mischung der stereoisomeren Formen einer erhaltenen Verbindung der Formel I anreichert.

2. Verfahren nach Anspruch 1, dadurch gekennzeichnet, dass man Verbindungen der Formel

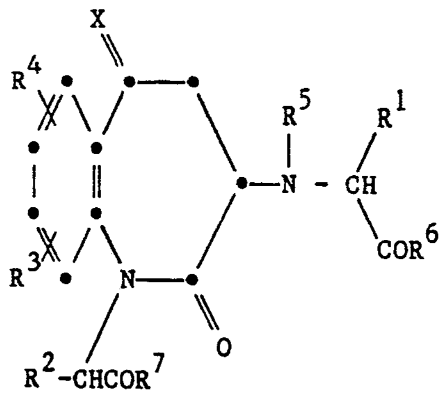

(IA),

worin der carbocyclische Ring auch Hexahydro sein kann; $R^1$ Wasserstoff, Niederalkyl, Amino-niederalkyl, Aryl, Aryl-niederalkyl, Cycloalkyl, Cycloalkyl-niederalkyl, Acylamino-niederalkyl, Mono-oder Di-niederalkylamino-niederalkyl, Niederalkylthio-niederalkyl, Carboxy-niederalkyl, verestertes Carboxy-niederalkyl, Carbamoyl-niederalkyl, N-substituiertes Carbamoyl-niederalkyl, Hydroxy-niederalkyl, verethertes oder acyliertes Hydroxy-niederalkyl, Aryloxy-niederalkyl, Arylthio-niederalkyl, Aryl-N-niederalkylamino-niederalkyl oder Arylamino-niederalkyl bedeutet; $R^2$ und $R^5$ Wasserstoff oder Niederalkyl bedeuten; $R^3$ und $R^4$ Wasserstoff, Niederalkyl, Niederalkoxy, Niederalkanoyloxy, Hydroxy, Halogen oder Trifluormethyl bedeuten oder $R^3$ und $R^4$ gemeinsam Niederalkylendioxy darstellen; X Oxo, zwei Wasserstoffatome, oder eine Hydroxygruppe oder acylierte Hydroxygruppe und ein Wasserstoff (unter Berücksichtigung der in Anspruch 1 angegebenen Massgabe) bedeutet; $R^6$ und $R^7$ unabhängig voneinander Hydroxy, Amino, Mono-oder Di-niederalkylamino, Niederalkoxy, Aryl-niederalkoxy, Niederalkanoyloxymethoxy, (Amino, Mono- oder Di-niederalkylamino, Carboxy oder Niederalkoxycarbonyl)-niederalkoxy bedeuten, oder Salze davon herstellt.

3. Verfahren nach Anspruch 1, dadurch gekennzeichnet, dass man Verbindungen der Formel IA, worin $R^1$ Wasserstoff, Niederalkyl, Amino-niederalkyl, Aryl-niederalkoxycarbonylamino-niederalkyl oder Aryl-niederalkyl bedeutet, worin Aryl für unsubstituiertes Phenyl oder durch Niederalkyl, Hydroxy, Niederalkoxy, Niederalkanoyloxy, Halogen oder Trifluormethyl monosubstituiertes Phenyl steht; $R^2$ und $R^5$ Wasserstoff oder Niederalkyl bedeuten; $R^3$ und $R^4$ für Wasserstoff, Niederalkyl, Niederalkoxy, Halogen oder Trifluormethyl stehen, oder $R^3$ und $R^4$ gemeinsam Niederalkylendioxy bedeuten; X für Oxo, zwei Wasserstoffatome, oder eine Hydroxy- oder Niederalkanoyloxygruppe und ein Wasserstoffatom steht (unter Berücksichtigung der in Anspruch 1 angegebenen Massgabe in einer Weise, die der hier für X genannten Bedeutung angepasst ist); $R^6$ und $R^7$ unabhängig voneinander für Hydroxy, Amino, Niederalkoxy, Phenyl-niederalkoxy oder Niederalkoxycarbonyl-niederalkoxy stehen; oder ihre Salze; oder die genannten Verbindungen, worin der carbocyclische Ring Hexahydro ist, herstellt.

4. Verfahren nach Anspruch 1, dadurch gekennzeichnet, dass man Verbindungen der Formel IA, worin $R^1$ Wasserstoff, Niederalkyl, ω-Amino-niederalkyl, ω-Arylmethoxycarbonylamino-niederalkyl oder Aryl-niederalkyl bedeutet, worin Aryl für unsubstituiertes Phenyl oder durch Niederalkyl, Hydroxy, Niederalkoxy, Niederalkanoyloxy, Halogen oder Trifluormethyl monosubstituiertes Phenyl steht; $R^2$ und $R^5$ Wasserstoff oder Niederalkyl bedeuten; $R^3$ für Wasserstoff steht; $R^4$ Wasserstoff, Niederalkyl, Niederalkoxy, Halogen oder Trifluormethyl bedeutet; X für Oxo, zwei Wasserstoffatome, oder eine Hydroxy- oder Niederalkanoyloxygruppe und ein Wasserstoffatom steht (unter Berücksichtigung der in Anspruch 1 angegebenen Massgabe in einer Weise, die der hier für X genannten Bedeutung angepasst ist); $R^6$ und $R^7$ unabhängig voneinander für Hydroxy, Amino, Niederalkoxy, Phenyl-niederalkoxy, Niederalkoxycarbonyl-niederalkoxy stehen; oder ihre Salze; oder die genannten Verbindungen, worin der carbocyclische Ring Hexahydro ist, herstellt.

5. Verfahren nach Anspruch 1, dadurch gekennzeichnet, dass man Verbindungen der Formel IA, worin $R^1$ die Bedeutung hat von Wasserstoff, Methyl, Ethyl, Isopropyl, ω-Aminopropyl, ω-Aminobutyl, ω-(Benzyloxycarbonylamino)propyl, ω-(Benzyloxycarbonylamino)butyl, Aryl-(methyl, ethyl, propyl), worin Aryl unsubstituiertes Phenyl oder durch Methyl, Hydroxy, Methoxy, Methylendioxy, Acetoxy, Chlor oder Trifluormethyl monosubstituiertes Phenyl bedeutet; $R^2$ und $R^5$ für Wasserstoff oder Methyl stehen; $R^3$ und $R^4$ Wasserstoff, Methoxy, Methyl, Chlor oder Trifluormethyl bedeuten; X für Oxo, zwei Wasserstoffatome, oder eine Hydroxy- oder Acetoxygruppe und ein Wasserstoffatom steht (unter Berücksichtigung der in Anspruch 1 angegebenen Massgabe in einer Weise, die der hier für X genannten Bedeutung angepasst ist); $R^6$ und $R^7$ unabhängig voneinander Hydroxy, Amino, Ethoxy, Methoxy, Benzyloxy, Ethoxycarbonylmethoxy oder Pivaloyloxymethoxy bedeuten; oder ihre Salze; oder die genannten Verbindungen, worin der carbocyclische Ring Hexahydro ist, herstellt.

6. Verfahren nach Anspruch 1, dadurch gekennzeichnet, dass man Verbindungen der Formel

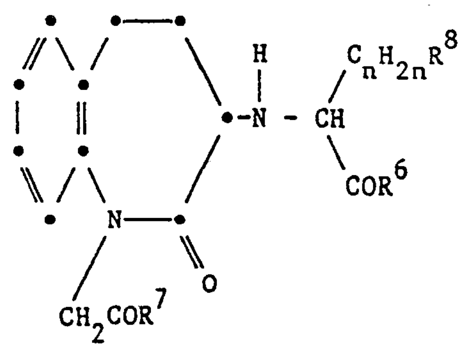

(IB),

worin n eine ganze Zahl von 1 bis 4 bedeutet, $R^8$ Benzyloxycarbonylamino bedeutet; $R^6$ und $R^7$ unabhängig voneinander Hydroxy, Niederalkoxy mit bis zu 4 Kohlenstoffatomen, Benzyloxy oder Amino bedeuten, oder ihre Salze; oder die genannten Verbindungen, worin der carbocyclische Ring Hexahydro ist, herstellt.

7. Verfahren nach Anspruch 1, dadurch gekennzeichnet, dass man Verbindungen der Formel

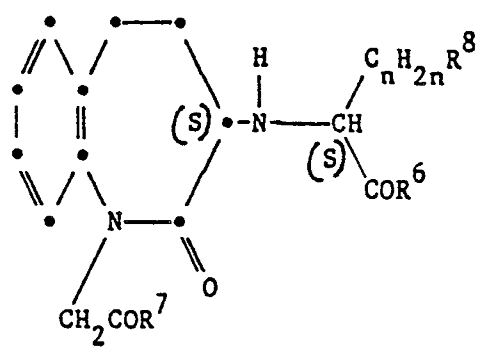

(IC),

worin S die Chiralität bedeutet, n eine ganze Zahl von 1 bis 4 bedeutet; $R^8$ Benzyloxycarbonylamino bedeutet; $R^6$ und $R^7$ unabhängig voneinander Hydroxy, Niederalkoxy mit bis zu 4 Kohlenstoffatomen, Benzyloxy oder Amino bedeuten; oder ihre Salze herstellt.

8. Verfahren nach Anspruch 1, dadurch gekennzeichnet, dass man 5-Acetoxy-1-carboxymethyl-3-(S)-(1-ethoxycarbonyl-3-phenylpropylamino)-2,3,4,5-tetrahydro-1H-1-benzazepin-2-on oder ein pharmazeutisch annehmbares Salz davon herstellt.

9. Verfahren nach Anspruch 1, dadurch gekennzeichnet, dass man 3-[(5-Benzyloxycarbonylamino-1-carboxy)-pentylamino]-1-carboxymethyl-2,3,4,5-tetrahydro-1H-1-benzazepin-2-on oder ein pharmazeutisch annehmbares Salz davon herstellt.

10. Verfahren zur Herstellung eines pharmazeutischen Präparates, gekennzeichnet durch die Verarbeitung eines nach einem der Ansprüche 1 bis 9 erfindungsgemäß hergestellten Wirkstoffs mit einem pharmazeutischen Trägermaterial.

**Claims for the Contracting States: BE CH DE FR GB IT LI LU NL SE**

1. Compounds of the general formula

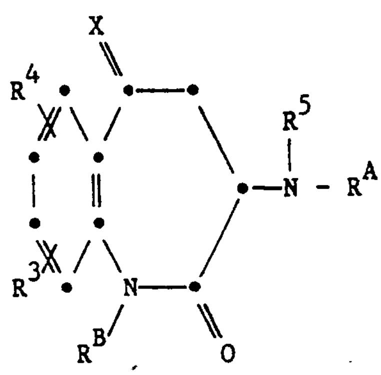

(I),

wherein $R^A$ and $R^B$ are radicals of the formulae

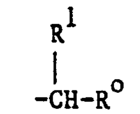 and 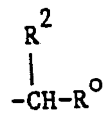 respectively,

in which R° is carboxy or functionally modified carboxy; $R^1$ is hydrogen, lower alkyl, amino-lower alkyl, aryl, aryl-lower alkyl, cycloalkyl, cycloalkyl-lower alkyl, acylamino-lower alkyl, mono- or di-lower alkylamino-lower alkyl, lower alkylthio-lower alkyl, carboxy-lower alkyl, esterified carboxy-lower alkyl, carbamoyl-lower alkyl, N-substituted carbamoyl-lower alkyl, hydroxy-lower alkyl, etherified or acylated hydroxy-lower alkyl, aryloxy-lower alkyl, aryl-(thio, sulfinyl or sulfonyl)-lower alkyl, aryl-N-lower alkylamino-lower alkyl or arylamino-lower alkyl; $R^2$ is hydrogen or lower alkyl; $R^3$ and $R^4$, each independently, represent hydrogen, lower alkyl, lower alkoxy, lower alkanoyloxy, hydroxy, halogen or trifluoromethyl, or $R^3$ and $R^4$ taken together represent lower alkylenedioxy; $R^5$ is hydrogen or lower alkyl; and X represents oxo, two hydrogen atoms, or hydroxy or acylated hydroxy together with one hydrogen atom; and wherein the carbocyclic ring may also be hexahydro or 6,7,8,9-tetrahydro; with the proviso that, if $R^1$ represents hydrogen, lower alkyl, amino-lower alkyl, aryl, aryl-lower alkyl, cycloalkyl or cycloalkyl-lower alkyl, X is acylated hydroxy together with one hydrogen atom; salts and stereoisomers of all these compounds.

2. Compounds of the formula

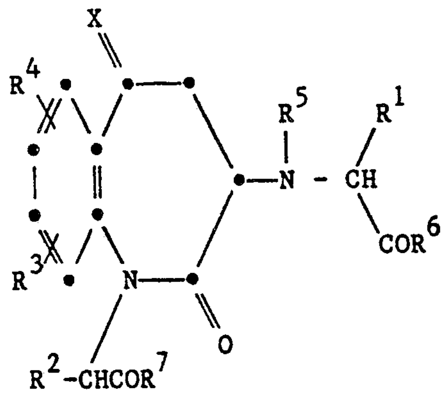 (IA),

wherein the carbocyclic ring may also be hexahydro; $R^1$ is hydrogen, lower alkyl, amino-lower alkyl, aryl, aryl-lower alkyl, cycloalkyl, cycloalkyl-lower alkyl, acylamino-lower alkyl, mono- or di-lower alkylamino-lower alkyl, lower alkylthio-lower alkyl, carboxy-lower alkyl, esterified carboxy-lower alkyl, carbamoyl-lower alkyl, N-substituted carbamoyl-lower alkyl, hydroxy-lower alkyl, etherified or acylated hydroxy-lower alkyl, aryloxy-lower alkyl, arylthio-lower alkyl, aryl-N-lower alkylamino-lower alkyl or arylamino-lower alkyl; $R^2$ and $R^5$ represent hydrogen or lower alkyl; $R^3$ and $R^4$ represent hydrogen, lower alkyl, lower alkoxy, lower alkanoyloxy, hydroxy, halogen or trifluoromethyl, or $R^3$ and $R^4$ taken together represent lower alkylenedioxy; X represents oxo, two hydrogen atoms, or one hydroxy or acylated hydroxy group and one hydrogen atom (with the proviso given in claim 1); $R^6$ and $R^7$, each independently, represent hydroxy, amino, mono- or di-lower alkylamino, lower alkoxy, aryl-lower alkoxy, lower alkanoyloxymethoxy, (amino, mono- or di-lower alkylamino, carboxy or lower alkoxycarbonyl)-lower alkoxy; or salts thereof.

3. Compounds of the formula IA shown in claim 2, wherein $R^1$ is hydrogen, lower alkyl, amino-lower alkyl, aryl-lower alkoxycarbonylamino-lower alkyl or aryl-lower alkyl wherein aryl represents unsubstituted phenyl or phenyl mono-substituted by lower alkyl, hydroxy, lower alkoxy, lower alkanoyloxy, halogen or by trifluoromethyl; $R^2$ and $R^5$ are hydrogen or lower alkyl; $R^3$ and $R^4$ are hydrogen, lower alkyl, lower alkoxy, halogen or trifluoromethyl, or $R^3$ and $R^4$ taken together represent lower alkylenedioxy; X represents oxo, two hydrogen atoms, or one hydroxy or lower alkanoyloxy group and one hydrogen atom (with the proviso of claim 1 in a form adapted to the meaning of X given here); $R^6$ and $R^7$, each independently, represent hydroxy, amino, lower alkoxy, phenyl-lower alkoxy or lower alkoxycarbonyl-lower alkoxy; or salts thereof; or said compounds wherein the carbocyclic ring is hexahydro.

4. Compounds of the formula IA shown in claim 2, wherein $R^1$ is hydrogen, lower alkyl, ω-amino-lower alkyl, ω-arylmethoxycarbonylamino-lower alkyl or aryl-lower alkyl wherein aryl represents unsubstituted phenyl or phenyl mono-substituted by lower alkyl, hydroxy, lower alkoxy, lower alkanoyloxy, halogen or by trifluoromethyl; $R^2$ and $R^5$ are hydrogen or lower alkyl; $R^3$ is hydrogen; $R^4$ is hydrogen, lower alkyl, lower alkoxy, halogen or trifluoromethyl; X represents oxo, two hydrogen atoms, or one hydroxy or lower alkanoyloxy group and one hydrogen atom (with the proviso of claim 1 in a form adapted to the meaning of X given here); $R^6$ and $R^7$, each independently, represent hydroxy, amino, lower alkoxy, phenyl-lower alkoxy, lower alkoxycarbonyl-lower alkoxy; or salts thereof; or said compounds wherein the carbocyclic ring is hexahydro.

5. Compounds of the formula IA shown in claim 2, wherein $R^1$ is hydrogen, methyl, ethyl, isopropyl, ω-aminopropyl, ω-aminobutyl, ω-(benzyloxycarbonylamino)propyl, ω-(benzyloxycarbonylamino)butyl, aryl-(methyl, ethyl, propyl) wherein aryl represents unsubstituted phenyl or phenyl mono-substituted by methyl, hydroxy, methoxy, methylenedioxy, acetoxy, chloro or by trifluoromethyl; $R^2$ and $R^5$ are hydrogen or methyl; $R^3$ and $R^4$ represent hydrogen, methoxy, methyl, chloro or trifluoromethyl; X represents oxo, two hydrogen atoms, or one hydroxy or acetoxy group and one hydrogen atom (with the proviso of claim 1 in a form adapted to the meaning of X given here); $R^6$ and $R^7$, each independently, represent hydroxy, amino, ethoxy, methoxy, benzyloxy, ethoxycarbonylmethoxy or pivaloyloxymethoxy; or salts thereof; or said compounds wherein the carbocyclic ring is hexahydro.

6. Compounds of the formula

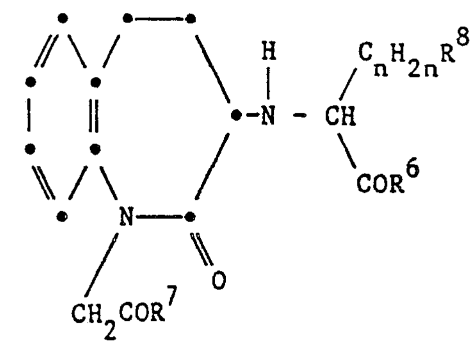

(IB),

wherein n represents an integer from 1 to 4; $R^8$ is benzyloxycarbonylamino; $R^6$ and $R^7$, each independently, represent hydroxy, lower alkoxy of up to 4 carbon atoms, benzyloxy or amino; or salts thereof; or said compounds wherein the carbocyclic ring is hexahydro.

7. Compounds of the formula

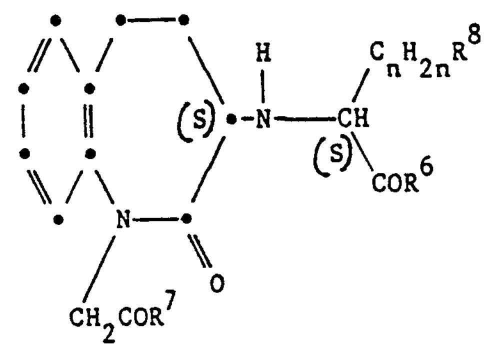

(IC),

wherein S represents the chirality, n represents an integer from 1 to 4; $R^8$ is benzyloxycarbonylamino; $R^6$ and $R^7$, each independently, represent hydroxy, lower alkoxy of up to 4 carbon atoms, benzyloxy or amino; or salts thereof.

8. 5 - Acetoxy - 1 - carboxymethyl - 3 - (S) - (1 - ethoxycarbonyl - 3 - phenylpropylamino) - 2,3,4,5 - tetrahydro - 1H - 1 - benzazepin - 2 - one or a salt thereof.

9. 3 - [5 - Benzyloxycarbonylamino - 1 - carboxy) - pentylamino] - 1 - carboxymethyl - 2,3,4,5 - tetrahydro - 1H - 1 - benzazepin - 2 - one or a salt thereof.

10. Pharmaceutical preparations containing a compound according to any one of claims 1 to 9, or a pharmaceutically acceptable salt thereof, together with a pharmaceutically suitable carrier.

11. The compounds named in any one of claims 1 to 9, or pharmaceutically acceptable salts thereof, for use in a method for the therapeutic treatment of the human or animal body.

12. The compounds named in any one of claims 1 to 9, or pharmaceutically acceptable salts thereof, as inhibitors of the angiotensin-converting enzyme.

13. The use of the compounds named in any one of claims 1 to 9, or pharmaceutically acceptable salts thereof, for the production of pharmaceutical preparations.

14. A process for the manufacture of the compounds of formula I mentioned in claim 1, in which formula all the symbols have the meanings given in claim 1, or salts thereof, characterised in that

a) in a compound of the formula

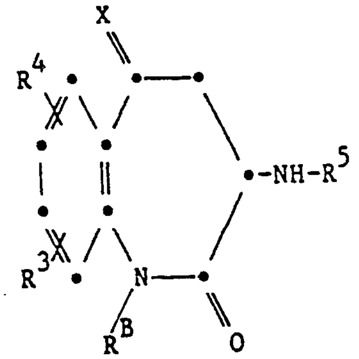

(II),

wherein the carbocyclic ring may also be hexahydro or 6,7,8,9-tetrahydro, and wherein X, $R^B$, $R^3$, $R^4$ and $R^5$ have the meanings given hereinabove, $R^A$ is introduced by alkylation with a compound of the formula

$$R^A—Z \qquad (IIIA),$$

wherein Z is a reactive esterified hydroxy group and $R^A$ has the meanings given hereinabove, or with a compound of the formula

$$R^1—CO—R^o \qquad (IV),$$

wherein $R^1$ and $R^o$ have the meanings given hereinabove, in the presence of a reducing agent with temporary protection of any primary and secondary amino groups and/or, optionally, hydroxy and/or oxo groups which may be present in any one of the radicals X, $R^B$, $R^3$ and $R^4$ and/or in the alkylating agent, or

b) a compound of the formula

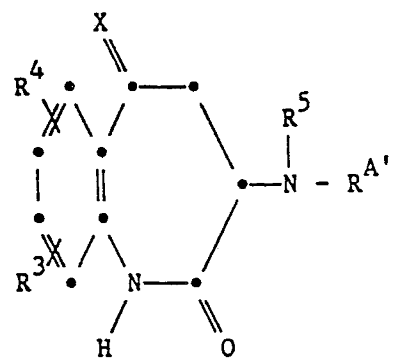

(V),

wherein the carbocyclic ring may also be hexahydro or 6,7,8,9-tetrahydro, and wherein X, $R^3$, $R^4$ and $R^5$ have the meanings given hereinabove and $R^{A'}$ is hydrogen or $R^A$ as defined hereinabove, is alkylated with a compound of the formula

$$R^B—Z \qquad (IIIB),$$

wherein Z is a reactive esterified hydroxy group and $R^B$ has the meanings given hereinabove, while temporarily protecting any primary and secondary amino groups and/or, optionally, hydroxy and/or oxo groups which may be present in any one of the radicals X, $R^{A'}$, $R^B$, $R^3$ and $R^4$, or

c) a compound of the formula

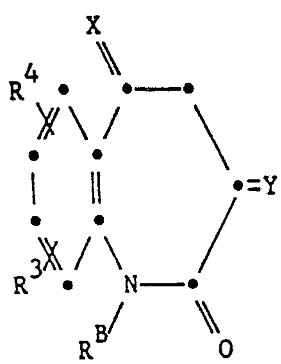

(VI),

wherein the carbocyclic ring may also be hexahydro or 6,7,8,9-tetrahydro, and wherein Y is oxo, dichloro, or a reactive esterified hydroxy group Z together with hydrogen, and X, $R^B$, $R^3$ and $R^4$ have the meanings given hereinabove, is condensed with an amine of the formula

$$R^A—NH—R^5 \qquad (VII),$$

wherein $R^A$ and $R^5$ have the meanings given hereinabove, with the proviso that, when Y is oxo or dichloro, the condensation is carried out in the presence of a reducing agent and with temporary protection of the oxo group which may be present as the substituent X, or

d) in a compound of the formula

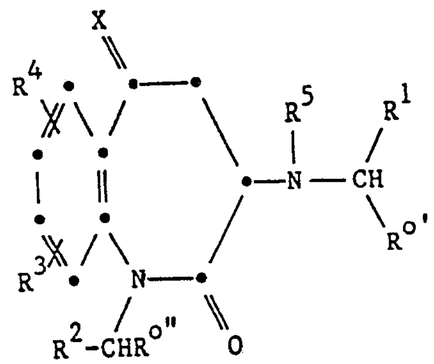

(VIII),

wherein the carbocyclic ring may also be hexahydro or 6,7,8,9-tetrahydro, and wherein X and $R^1$ to $R^5$ have the meanings given hereinabove, one of the symbols $R^{o\prime}$ and $R^{o\prime\prime}$ is cyano and the other one is cyano or $R^o$ as defined hereinabove, the cyano group(s) is (are) subjected to solvolysis, or

e) a compound of the formula

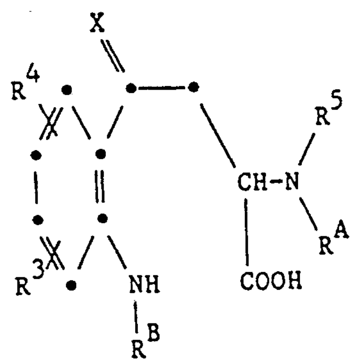

(IX),

wherein the carbocyclic ring may also be hexahydro or 3,4,5,6-tetrahydro, and wherein X, $R^A$, $R^B$, $R^3$, $R^4$ and $R^5$ have the meanings given hereinabove, or an ester thereof, is cyclised, or

f) a compound that is structurally identical with a compound of formula I as defined above, except for having an additional double bond located at C—3 or between the nitrogen atom and the adjacent carbon atom within the group $R^A$, is treated with a reducing agent in order to saturate this double bond, or

g) in order to produce a compound of formula I as defined hereinabove in which X is oxo, a compound of the formula

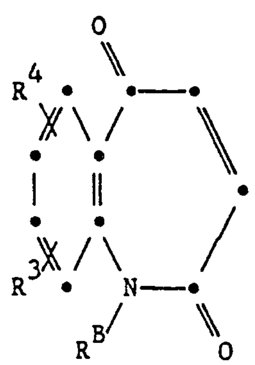

(X),

wherein the carbocyclic ring may also be hexahydro or 6,7,8,9-tetrahydro, and wherein $R^B$, $R^3$ and $R^4$ have the meanings given hereinabove, is condensed with an amine of the formula

$$R^A—NH—R^5 \qquad (VII),$$

wherein $R^A$ and $R^5$ have the meanings given hereinabove, and, if desired, a resulting compound of formula I is converted into another compound of formula I and/or, if desired, a resulting compound of formula I having salt-forming properties is converted into a salt thereof or a resulting salt is converted into another salt, or a free compound is liberated from such a salt and/or, if desired, an optical isomer that has a specific configuration with respect to at least one centre of chirality is enriched from a mixture of stereoisomeric forms of a resulting compound of formula I.

15. The compounds obtainable according to the process of claim 14.

**Claims for the Contracting State: AT**

1. A process for the manufacture of 3-amino-1-benzazepin-2-one-1-alkanoic acids of the general formula

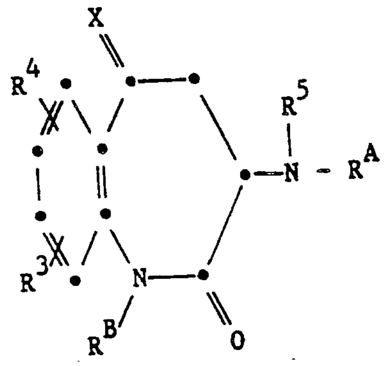

(I),

wherein $R^A$ and $R^B$ are radicals of the formulae

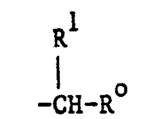

and

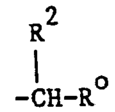

, respectively,

in which R° is carboxy or functionally modified carboxy; $R^1$ is hydrogen, lower alkyl, amino-lower alkyl, aryl, aryl-lower alkyl, cycloalkyl, cycloalkyl-lower alkyl, acylamino-lower alkyl, mono- or di-lower alkylamino-lower alkyl, lower alkylthio-lower alkyl, carboxy-lower alkyl, esterified carboxy-lower alkyl, carbamoyl-lower alkyl, N-substituted carbamoyl-lower alkyl, hydroxy-lower alkyl, etherified or acylated hydroxy-lower alkyl, aryloxy-lower alkyl, aryl-(thio, sulfinyl or sulfonyl)-lower alkyl, aryl-N-lower alkylamino-lower alkyl or arylamino-lower alkyl; $R^2$ is hydrogen or lower alkyl; $R^3$ and $R^4$, each independently, represent hydrogen, lower alkyl, lower alkoxy, lower alkanoyloxy, hydroxy, halogen or trifluoromethyl, or $R^3$ and $R^4$ taken together represent lower alkylenedioxy; $R^5$ is hydrogen or lower alkyl; and X represents oxo, two hydrogen atoms, or hydroxy or acylated hydroxy together with one hydrogen atom; and wherein the carbocyclic ring may also be hexahydro or 6,7,8,9-tetrahydro; with the proviso that, if $R^1$ represents hydrogen, lower alkyl, amino-lower alkyl, aryl, aryl-lower alkyl, cycloalkyl or cycloalkyl-lower alkyl, X is acylated hydroxy together with one hydrogen atom; salts and stereoisomers of all these compounds, characterised in that

a) in a compound of the formula

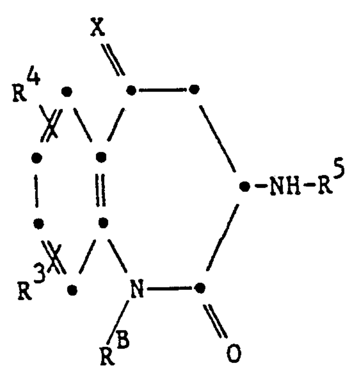

(II),

wherein the carbocyclic ring may also be hexahydro or 6,7,8,9-tetrahydro, and wherein X, $R^B$, $R^3$, $R^4$ and $R^5$ have the meanings given hereinabove, $R^A$ is introduced by alkylation with a compound of the formula

$R^A$—Z (IIIA),

wherein Z is a reactive esterified hydroxy group and $R^A$ has the meanings given hereinabove, or with a compound of the formula

$$R^1—CO—R^o \quad (IV),$$

wherein $R^1$ and $R^o$ have the meanings given hereinabove, in the presence of a reducing agent with temporary protection of any primary and secondary amino groups and/or, optionally, hydroxy and/or oxo groups which may be present in any one of the radicals X, $R^B$, $R^3$ and $R^4$ and/or in the alkylating agent, or

b) a compound of the formula

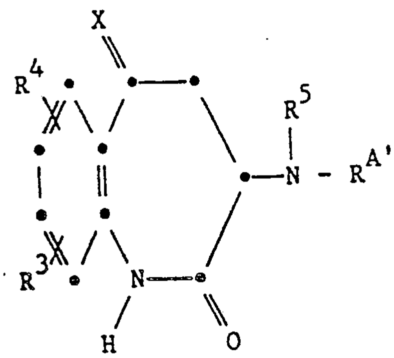

(V),

wherein the carbocyclic ring may also be hexahydro or 6,7,8,9-tetrahydro, and wherein X, $R^3$, $R^4$ and $R^5$ have the meanings given hereinabove and $R^{A'}$ is hydrogen or $R^A$ as defined hereinabove, is alkylated with a compound of the formula

$$R^B—Z \quad (IIIB),$$

wherein Z is a reactive esterified hydroxy group and $R^B$ has the meanings given hereinabove, while temporarily protecting any primary and secondary amino groups and/or, optionally, hydroxy and/or oxo groups which may be present in any one of the radicals X, $R^{A'}$, $R^B$, $R^3$ and $R^4$, or

c) a compound of the formula

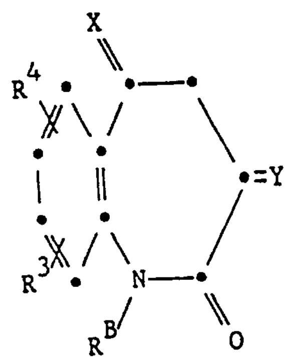

(VI),

wherein the carbocylic ring may also be hexahydro or 6,7,8,9-tetrahydro, and wherein Y is oxo, dichloro, or a reactive esterified hydroxy group Z together with hydrogen, and X, $R^B$, $R^3$ and $R^4$ have the meanings given hereinabove, is condensed with an amine of the formula

$$R^A—NH—R^5 \quad (VII),$$

wherein $R^A$ and $R^5$ have the meanings given hereinabove, with the proviso that, when Y is oxo or dichloro, the condensation is carried out in the presence of a reducing agent and with temporary protection of the

oxo group which may be present as the substituent X, or d) in a compound of the formula

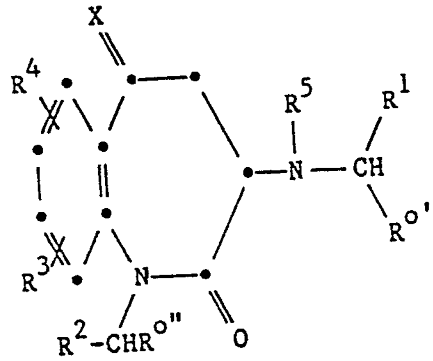

(VIII),

wherein the carbocyclic ring may also be hexahydro or 6,7,8,9-tetrahydro, and wherein X and $R^1$ to $R^5$ have the meanings given hereinabove, one of the symbols $R^{o\prime}$ and $R^{o\prime\prime}$ is cyano and the other one is cyano or $R^o$ as defined hereinabove, the cyano group(s) is (are) subjected to solvolysis, or

e) a compound of the formula

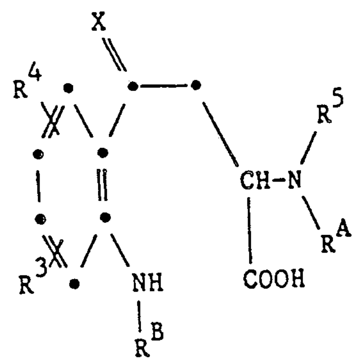

(IX),

wherein the carbocyclic ring may also be hexahydro or 3,4,5,6-tetrahydro, and wherein X, $R^A$, $R^B$, $R^3$, $R^4$ and $R^5$ have the meanings given hereinabove, or an ester thereof, is cyclised, or

f) a compound that is structurally identical with a compound of formula I as defined above, except for having an additional double bond located at C—3 or between the nitrogen atom and the adjacent carbon atom within the group $R^A$, is treated with a reducing agent in order to saturate this double bond, or

g) in order to produce a compound of formula I as defined hereinabove in which X is oxo, a compound of the formula

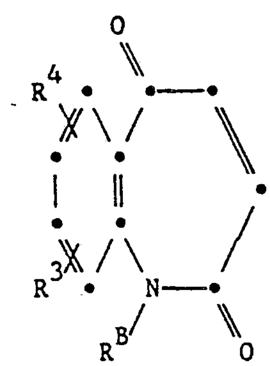

(X),

wherein the carbocyclic ring may also be hexahydro or 6,7,8,9-tetrahydro, and wherein $R^B$, $R^3$ and $R^4$ have the meanings given hereinabove, is condensed with an amine of the formula

$$R^A—NH—R^5 \qquad (VII),$$

wherein $R^A$ and $R^5$ have the meanings given hereinabove, and, if desired, a resulting compound of formula I is converted into another compound of formula I and/or, if desired, a resulting compound of formula I having salt-forming properties is converted into a salt thereof or a resulting salt is converted into another salt, or a free compound is liberated from such a salt and/or, if desired, an optical isomer that has a specific configuration with respect to at least one centre of chirality is enriched from a mixture of stereoisomeric forms of a resulting compound of formula I.

2. A process according to claim 1, characterised in that compounds of the formula

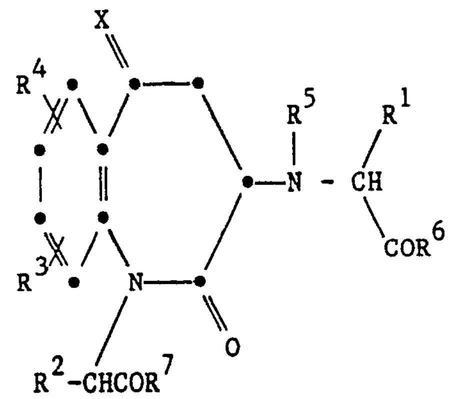

(IA),

wherein the carbocyclic ring may also be hexahydro; $R^1$ is hydrogen, lower alkyl, amino-lower alkyl, aryl, aryl-lower alkyl, cycloalkyl, cycloalkyl-lower alkyl, acylamino-lower alkyl, mono- or di-lower alkylamino-lower alkyl, lower alkylthio-lower alkyl, carboxy-lower alkyl, esterified carboxy-lower alkyl, carbamoyl-lower alkyl, N-substituted carbamoyl-lower alkyl, hydroxy-lower alkyl, etherified or acylated hydroxy-lower alkyl, aryloxy-lower alkyl, arylthio-lower alkyl, aryl-N-lower alkylamino-lower alkyl or arylamino-lower alkyl; $R^2$ and $R^5$ represent hydrogen or lower alkyl; $R^3$ and $R^4$ represent hydrogen, lower alkyl, lower alkoxy, lower alkanoyloxy, hydroxy, halogen or trifluoromethyl, or $R^3$ and $R^4$ taken together represent lower alkylenedioxy; X represents oxo, two hydrogen atoms, or one hydroxy or acylated hydroxy group and one hydrogen atom (with the proviso given in claim 1); $R^6$ and $R^7$, each independently, represent hydroxy, amino, mono- or di-lower alkylamino, lower alkoxy, aryl-lower alkoxy, lower alkanoyloxymethoxy, (amino, mono- or di-lower alkylamino, carboxy or lower alkoxycarbonyl)lower alkoxy; or salts thereof are manufactured.

3. A process according to claim 1, characterised in that compounds of the formula IA wherein $R^1$ is hydrogen, lower alkyl, amino-lower alkyl, aryl-lower alkoxycarbonylamino-lower alkyl or aryl-lower alkyl wherein aryl represents unsubstituted phenyl or phenyl mono-substituted by lower alkyl, hydroxy, lower alkoxy, lower alkanoyloxy, halogen or by trifluoromethyl; $R^2$ and $R^5$ are hydrogen or lower alkyl; $R^3$ and $R^4$ are hydrogen, lower alkyl, lower alkoxy, halogen or trifluoromethyl, or $R^3$ and $R^4$ taken together represent lower alkylenedioxy; X represents oxo, two hydrogen atoms, or one hydroxy or lower alkanoyloxy group and one hydrogen atom (with the proviso of claim 1 in a form adapted to the meaning of X given here); $R^6$ and $R^7$, each independently, represent hydroxy, amino, lower alkoxy, phenyl-lower alkoxy or lower alkoxycarbonyl-lower alkoxy; or salts thereof; or said compounds wherein the carbocyclic ring is hexahydro are manufactured.

4. A process according to claim 1, characterised in that compounds of the formula IA wherein $R^1$ is hydrogen, lower alkyl, ω-amino-lower alkyl, ω-arylmethoxycarbonylamino-lower alkyl or aryl-lower alkyl wherein aryl represents unsubstituted phenyl or phenyl mono-substituted by lower alkyl, hydroxy, lower alkoxy, lower alkanoyloxy, halogen or by trifluoromethyl; $R^2$ and $R^5$ are hydrogen or lower alkyl; $R^3$ is hydrogen; $R^4$ is hydrogen, lower alkyl, lower alkoxy, halogen or trifluoromethyl; X represents oxo, two hydrogen atoms, or one hydroxy or lower alkanoyloxy group and one hydrogen atom (with the proviso of claim 1 in a form adapted to the meaning of X given here); $R^6$ and $R^7$, each independently, represent hydroxy, amino, lower alkoxy, phenyl-lower alkoxy, lower alkoxycarbonyl-lower alkoxy; or salts thereof; or said compounds wherein the carbocyclic ring is hexahydro are manufactured.

5. A process according to claim 1, characterised in that compounds of the formula IA wherein $R^1$ is hydrogen, methyl, ethyl, isopropyl, ω-aminopropyl, ω-aminobutyl, ω-(benzyloxycarbonylamino)propyl, ω-(benzyloxycarbonylamino)butyl, aryl-(methyl, ethyl, propyl) wherein aryl represents unsubstituted phenyl or phenyl mono-substituted by methyl, hydroxy, methoxy, methylenedioxy, acetoxy, chloro or by trifluoromethyl; $R^2$ and $R^5$ are hydrogen or methyl; $R^3$ and $R^4$ represent hydrogen, methoxy, methyl, chloro or trifluoromethyl; X represents oxo, two hydrogen atoms, or one hydroxy or acetoxy group and one hydrogen atom (with the proviso of claim 1 in a form adapted to the meaning of X given here); $R^6$ and $R^7$, each independently, represent hydroxy, amino, ethoxy, methoxy, benzyloxy, ethoxycarbonylmethoxy or pivaloyloxymethoxy; or salts thereof; or said compounds wherein the carbocyclic ring is hexahydro are manufactured.

6. A process according to claim 1, characterised in that compounds of the formula

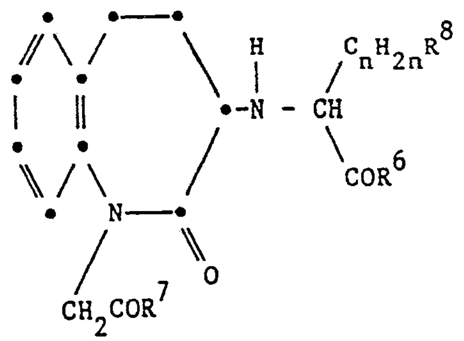

(IB),

wherein n represents an integer from 1 to 4; $R^8$ is benzyloxycarbonylamino; $R^6$ and $R^7$, each independently, represent hydroxy, lower alkoxy of up to 4 carbon atoms, benzyloxy or amino; or salts thereof; or said compounds wherein the carbocyclic ring is hexahydro are manufactured.

7. A process according to claim 1, characterised in that compounds of the formula

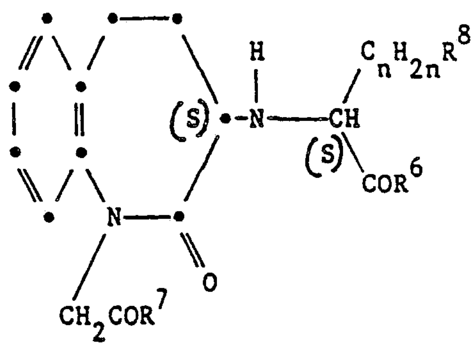

(IC),

wherein S represents the chirality, n represents an integer from 1 to 4; $R^8$ is benzyloxycarbonylamino; $R^6$ and $R^7$, each independently, represent hydroxy, lower alkoxy of up to 4 carbon atoms, benzyloxy or amino; or salts thereof are manufactured.

8. A process according to claim 1, characterised in that 5-acetoxy-1-carboxymethyl-3-(S)-(1-ethoxycarbonyl-3-phenylpropylamino)-2,3,4,5-tetrahydro-1H-1-benzazepin-2-one or a pharmaceutically acceptable salt thereof is manufactured.

9. A process according to claim 1, characterised in that 3-[5-benzyloxycarbonylamino-1-carboxy)-pentylamino]-1-carboxymethyl-2,3,4,5-tetrahydro-1H-1-benzazepin-2-one or a pharmaceutically acceptable salt thereof is manufactured.

10. A process for the manufacture of a pharmaceutical preparation, characterised by processing an active ingredient manufactured in accordance with the invention according to any one of claims 1 to 9 with a pharmaceutical carrier.

**Revendications pour les Etats contractants: BE CH LI DE FR GB IT LU NL SE**

1. Composés de formule générale

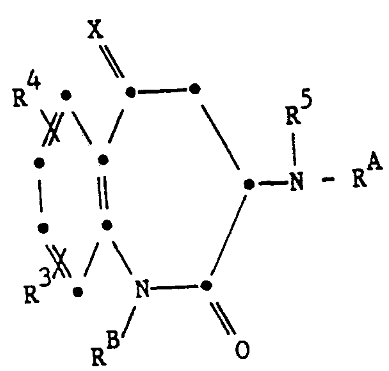

(I),

dans laquelle $R^A$ et $R^B$ représentent des groupes de formules respectives

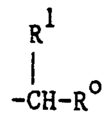

et

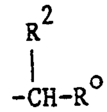

dans lesquelles R° représente un groupe carboxy ou un groupe carboxy qui a subi une modification fonctionnelle; $R^1$ représente l'hydrogène, un groupe alkyle inférieur, aminoalkyle inférieur, aryle, aryl-alkyle inférieur, cycloalkyle, cycloalkyl-alkyle inférieur, acylaminoalkyle inférieur, mono- ou di-(alkyle inférieur)-aminoalkyle inférieur, (alkyle inférieur)-thio-alkyle inférieur, carboxy-alkyle inférieur, (carboxy estérifié)alkyle inférieur, carbamoyl-alkyle inférieur, (carbamoyle substitué à l'azote)-alkyle inférieur, hydroxyalkyle inférieur, (hydroxy éthérifié ou acylé)-alkyle inférieur, aryloxy-alkyle inférieur, aryl-(thio-, sulfinyl- ou sulfonyl)-alkyle inférieur, aryl-(N-alkyle inférieur-amino-alkyle inférieur ou arylaminoalkyle inférieur; $R^2$ représente l'hydrogène ou un groupe alkyle inférieur; $R^3$ et $R^4$ représentent chacun, indépendamment l'un de l'autre, l'hydrogène, un groupe alkyle inférieur, alcoxy inférieur, alcanoyloxy inférieur, hydroxy, un halogène ou un groupe trifluorométhyle, ou bien $R^3$ et $R^4$ forment ensemble un groupe alkylène-dioxy inférieur; $R^5$ représente l'hydrogène ou un groupe alkyle inférieur; et X représente un groupe oxo, deux atomes d'hydrogène ou un groupe hydroxy ou hydroxy acylé et un atome d'hydrogène; et dans laquelle le noyau carbocyclique peut également être hexahydrogéné ou 6,7,8,9-tétrahydrogéné; sous réserve que, lorsque $R^1$ représente l'hydrogène, un groupe alkyle inférieur, aminoalkyle inférieur, aryle, aryl-alkyle inférieur, cycloalkyle ou cycloalkyl-alkyle inférieur, X représente un groupe hydroxy acylé et un atome d'hydrogène; les sels et stéréoisomères de tous ces composés.

2. Composés de formule

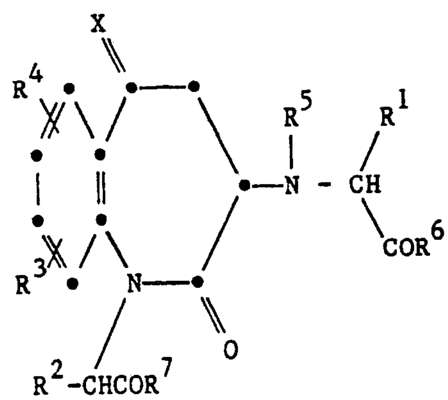

(IA),

dans laquelle le noyau carbocyclique peut également être hexahydrogéné; $R^1$ représente l'hydrogène, un groupe alkyle inférieur, aminoalkyle inférieur, aryle, aryl-alkyle inférieur, cycloalkyle, cycloalkyl-alkyle inférieur, acylamino-alkyle inférieur, mono- ou di(alkyle inférieur)-amino-alkyle inférieur, (alkyle inférieur)-thio-alkyle inférieur, carboxy-alkyle inférieur, (carboxy estérifié)-alkyle inférieur, carbamoylalkyle inférieur, (carbamoyle substitué à l'azote)alkyle inférieur, hydroxyalkyle inférieur, (hydroxy éthérifié ou acylé)-alkyle inférieur, aryloxy-alkyle inférieur, arylthio-alkyle inférieur, aryl-(N-alkyle inférieur)-amino-alkyle inférieur ou arylamino-alkyle inférieur; $R^2$ et $R^5$ représentent l'hydrogène ou des groupes alkyle inférieurs; $R^3$ et $R^4$ représentent l'hydrogène, des groupes alkyle inférieurs, alcoxy inférieurs, alcanoyloxy inférieurs, hydroxy, des halogènes ou des groupes trifluorométhyle ou bien $R^3$ et $R^4$ forment ensemble un groupe alkylène-dioxy inférieur; X représente un groupe oxo, deux atomes d'hydrogène ou un groupe hydroxy ou hydroxy acylé et un atome d'hydrogène (tenu compte des réserves indiquées dans la revendication 1); $R^6$ et $R^7$ représentent chacun, indépendamment l'un de l'autre, un groupe hydroxy, amino, mono- ou di-(alkyle inférieur)-amino, alcoxy inférieur, arylalcoxy inférieur, (alcanoyloxy inférieur)-méthoxy, [amino, mono- ou di-(alkyle inférieur)-amino, carboxy ou (alcoxy inférieur)-carbonyl]-alcoxy inférieur; ou leurs sels.

3. Composés de formule IA de la revendication 2, dans lesquels $R^1$ représente l'hydrogène, un groupe alkyle inférieur, aminoalkyle inférieur, aryl-(alcoxy inférieur)-carbonylamino-alkyle inférieur ou aryl-alkyle inférieur dans lesquels la partie aryle consiste en un groupe phényle non substitué ou en un groupe phényle mono-substitué par un groupe alkyle inférieur, hydroxy, alcoxy inférieur, alcanoyloxy inférieur, un halogène ou un groupe trifluorométhyle; $R^2$ et $R^5$ représentent l'hydrogène ou des groupes alkyle inférieurs; $R^3$ et $R^4$ représentent l'hydrogène, des groupes alkyle inférieurs, alcoxy inférieurs, des halogènes ou des groupes trifluorométhyle ou bien $R^3$ et $R^4$ forment ensemble un groupe alkylène-dioxy inférieur; X représente un groupe oxo, deux atomes d'hydrogène ou un groupe hydroxy ou alcanoyloxy inférieur et un atome d'hydrogène (tenu compte des réserves indiquées dans la revendication 1 en sorte que les significations données ici pour X conviennent); $R^6$ et $R^7$ représentent chacun, indépendamment l'un de l'autre, un groupe hydroxy, amino, alcoxy inférieur, phényl-alcoxy inférieur ou (alcoxy

inférieur)carbonyl-alcoxy inférieur; ou leurs sels; ou les composés correspondants dans lesquels le noyau carbocyclique est hexahydrogéné.

4. Composés de formule IA de la revendication 2, dans lesquels $R^1$ représente l'hydrogène, un groupe alkyle inférieur, oméga-aminoalkyle inférieur, omégaarylméthoxycarbonylamino-alkyle inférieur ou arylalkyle inférieur dans lesquels la partie aryle consiste en un groupe phényle non substitué ou en un groupe phényle monosubstitué par un groupe alkyle inférieur, hydroxy, alcoxy inférieur, alcanoyloxy inférieur, un halogène ou un groupe trifluorométhyle; $R^2$ et $R^5$ représentent l'hydrogène ou des groupes alkyle inférieurs; $R^3$ représente l'hydrogène; $R^4$ représente l'hydrogène, un groupe alkyle inférieur, alcoxy inférieur, un halogène ou un groupe trifluorométhyle; X représente un groupe oxo, deux atomes d'hydrogène ou un groupe hydroxy ou alcanoyloxy inférieur et un atome d'hydrogène (tenu compte des réserves indiquées dans la revendication 1 en sorte que les significations données ici pour X conviennent); $R^6$ et $R^7$ représentent chacun, indépendamment l'un de l'autre, un groupe hydroxy, amino, alcoxy inférieur, phényl-alcoxy inférieur, (alcoxy inférieur)-carbonyl-alcoxy inférieur; ou leurs sels; ou les composés correspondants dans lesquels le noyau carbocyclique est hexahydrogéné.

5. Composés de formule IA de la revendication 2, dans lesquels $R^1$ représente l'hydrogène, un groupe méthyle, éthyle, isopropyle, oméga-aminopropyle, omégaaminobutyle, oméga-(benzyloxycarbonyl-amino)-propyle, oméga-(benzyloxycarbonylamino)-butyle, aryl-(méthyle, éthyle, propyle) dans lesquels la partie aryle consiste en un groupe phényle non substitué en un groupe phényle monosubstitué par un groupe méthyle, hydroxy, méthoxy, méthylène-dioxy, acétoxy, le chlore ou un groupe trifluorométhyle; $R^2$ et $R^5$ représentent l'hydrogène ou des groupes méthyle; $R^3$ et $R^4$ représentent l'hydrogène, des groupes méthoxy, méthyle, le chlore ou des groupes trifluorométhyle; X représente un groupe oxo, deux atomes d'hydrogène ou un groupe hydroxy ou acétoxy et un atome d'hydrogène (tenu compte des réserves indiquées dans la revendication 1 en sorte que les définitions données ici pour X conviennent); $R^6$ et $R^7$ représentent chacun, indépendamment l'un de l'autre, un groupe hydroxy, amino, éthoxy, méthoxy, benzyloxy, éthoxycarbonylméthoxy ou pivaloyloxyméthoxy; ou leurs sels; ou les composés correspondants dans lesquels le noyau carbocyclique est hexahydrogéné.

6. Composés de formule

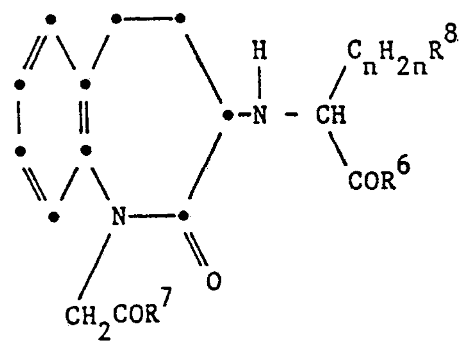

(IB),

dans laquelle n est un nombre entier allant de 1 à 4, $R^8$ représente un groupe benzyloxycarbonylamino; $R^6$ et $R^7$ représentent chacun, indépendamment l'un de l'autre, un groupe hydroxy, alcoxy inférieur contenant jusqu'à 4 atomes de carbone, benzyloxy ou amino, ou leurs sels; ou les composés correspondants dans lesquels le noyau carbocyclique est hexahydrogéné.

7. Composés de formule

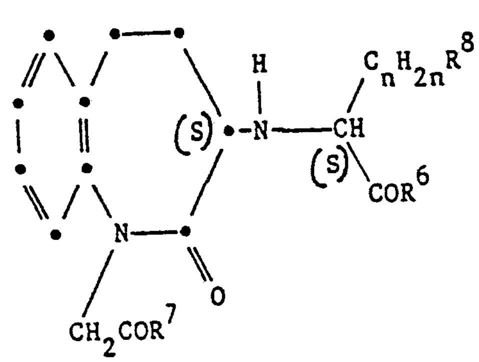

(IC),

dans laquelle S indique la chiralité, n est un nombre entier allant de 1 à 4; $R^8$ représente un groupe benzyloxycarbonylamino; $R^6$ et $R^7$ représentent chacun, indépendamment l'un de l'autre, un groupe hydroxy, alcoxy inférieur contenant jusqu'à 4 atomes de carbone, benzyloxy ou amino; ou leurs sels.

8. La 5 - acétoxy - 1 - carboxyméthyl - 3 - (S) - (1 - éthoxycarbonyl-3-phénylpropylamino) - 2,3,4,5 - tétrahydro - 1H - 1 - benzazépine - 2 - one ou un sel de ce composé.

9. La 3 - [(5 - benzyloxycarbonylamino - 1 - carboxy)pentylamino] - 1 - carboxyméthyl - 2,3,4,5 - tétrahydro - 1H - 1 - benzazépine-2-one ou un sel de ce composé.

10. Compositions pharmaceutiques contenant un composé selon l'une des revendications 1 à 9 ou un sel d'un tel composé acceptable pour l'usage pharmaceutique, avec un véhicule convenant pour l'usage pharmaceutique.

11. Les composés mentionnés dans l'une des revendications 1 à 9 ou leurs sels acceptables pour l'usage pharmaceutique, pour l'utilisation dans un procédé pour le traitement thérapeutique de l'organisme humain ou animal.

12. Les composés mentionnés dans l'une des revendications 1 à 9 ou leurs sels acceptables pour l'usage pharmaceutique, en tant qu'inhibiteurs de l'enzyme convertissant l'angiotensine.

13. Utilisation des composés mentionnés dans l'une des revendications 1 à 9 ou de leurs sels acceptables pour l'usage pharmaceutique, pour la préparation de compositions pharmaceutiques.

14. Procédé de préparation des composés de formule I définie dans la revendication 1 et dans laquelle tous les symboles ont les significations indiquées dans la revendication 1, ou de leurs sels, caractérisé en ce que:

a) dans un composé de formule

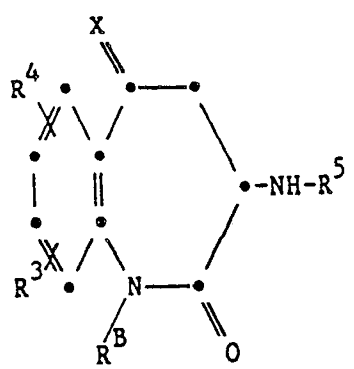

(II),

dans laquelle le noyau carbocyclique peut également être hexahydrogéné ou 6,7,8,9-tétrahydrogéné et X, $R^B$, $R^3$, $R^4$ et $R^5$ ont les significations indiquées précédemment, on introduit $R^A$ par alkylation à l'aide d'un composé de formule

$$R^A—Z \qquad \text{(IIIA)}$$

dans laquelle Z représente un groupe hydroxy à l'état d'ester réactif et $R^A$ a les significations indiquées ci-dessus, ou à l'aide d'un composé de formule

$$R^1—CO—R^o \qquad \text{(IV)}$$

dans laquelle $R^1$ et $R^o$ ont les significations indiquées ci-dessus, en présence d'un agent réducteur, en protégeant transitoirement des groupes amino primaires et secondaires éventuels et/ou le cas échéant des groupes hydroxy et/ou oxo qui peuvent être présents dans l'un quelconque des substituants X, $R^B$, $R^3$ et $R^4$ et/ou dans l'agent alkylant, ou bien

b) on alkyle un composé de formule

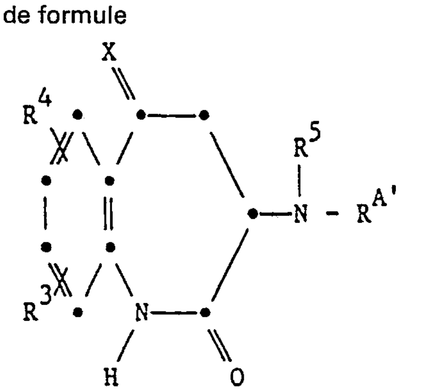

(V),

dans laquelle le noyau carbocyclique peut également être hexahydrogéné ou 6,7,8,9-tétrahydrogéné et X, $R^3$, $R^4$ et $R^5$ ont les significations indiquées ci-dessus, et $R^{A'}$ représente l'hydrogène ou a l'une des significations indiquées précédemment pour $R^A$, à l'aide d'un composé de formule

$$R^B—Z \qquad \text{(IIIB)}$$

dans laquelle Z représente un groupe hydroxy à l'état d'ester réactif et $R^B$ a les significations indiquées précédemment, en protégeant transitoirement les groupes amino primaires et secondaires éventuels et/ou le cas échéant des groupes hydroxy et/ou oxo qui peuvent être présents dans l'un quelconque des substituants X, $R^{A'}$, $R^B$, $R^3$ et $R^4$, ou bien

c) on condense un composé de formule

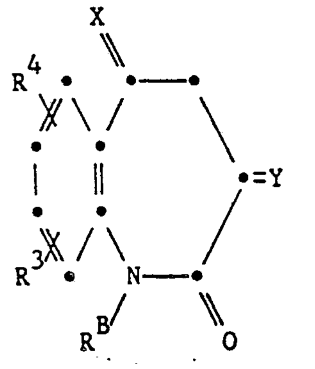

(VI),

dans laquelle le noyau carbocyclique peut également être hexahydrogéné ou 6,7,8,9-tétrahydrogéné et Y représente un substituant oxo, dichloro, ou un groupe hydroxy à l'état d'ester réactif Z et un atome d'hydrogène, et X, $R^B$, $R^3$ et $R^4$ ont les significations indiquées ci-dessus, avec une amine de formule

$$R^A—NH—R^5 \quad (VII)$$

dans laquelle $R^A$ et $R^5$ ont les significations indiquées ci-dessus, en opérant en présence d'un agent réducteur lorsque Y représente un substituant oxo ou dichloro, et en protégeant transitoirement le groupe oxo qui peut être présent en tant que substituant X, ou bien

d) dans un composé de formule

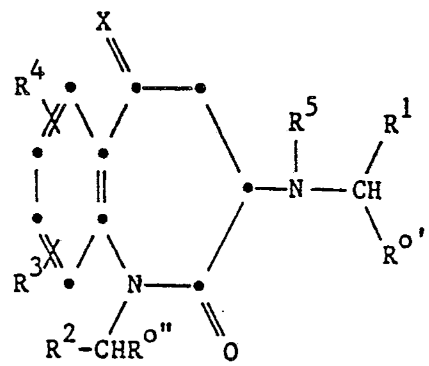

(VIII),

dans laquelle le noyau carbocyclique peut également être hexahydrogéné ou 6,7,8,9-tétrahydrogéné et X, $R^1$ à $R^5$ ont les significations indiquées ci-dessus, l'un des symboles $R^{o'}$ et $R^{o''}$ représente un groupe cyano et l'autre représente un groupe cyano ou a l'une des significations de $R^o$ données précédemment, on soumet le ou les groupes cyano à une solvolyse, ou bien

e) On cyclise un composé de formule

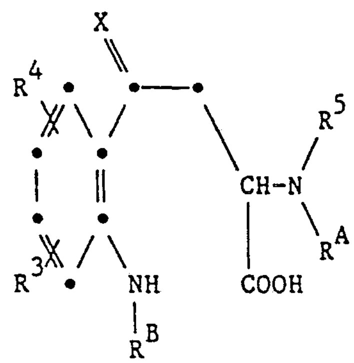

(IX),

dans laquelle le noyau carbocyclique peut également être hexahydrogéné ou 3,4,5,6-tétrahydrogéné et X, $R^A$, $R^B$, $R^3$, $R^4$ et $R^5$ ont les significations indiquées ci-dessus, ou un ester d'un tel composé,

f) on traite un composé ayant une structure identique à celle d'un composé de formule I telle que définie ci-dessus sauf qu'il porte une autre double liaison en C—3 ou entre l'atome d'azote et l'atome de carbone voisin à l'intérieur du groupe $R^A$, par un agent réducteur afin de saturer cette double liaison, ou bien

g) pour préparer un composé de formule I telle que définie ci-dessus et dans laquelle X représente un groupe oxo, on condense un composé de formule

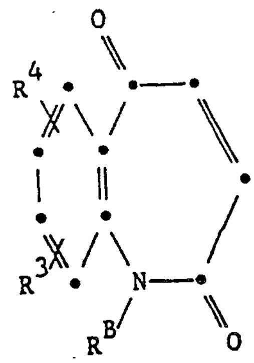

(X),

dans laquelle le noyau carbocyclique peut également être hexahydrogéné ou 6,7,8,9-tétrahydrogéné, et $R^B$, $R^3$ et $R^4$ ont les significations indiquées ci-dessus, avec une amine de formule

$$R^1—NH—R^5 \qquad (VII)$$

dans laquelle $R^A$ et $R^5$ ont les significations indiquées ci-dessus, et si on le désire, on convertit un composé obtenu répondant à la formule I en un autre composé de formule I et/ou, si on le désire, on convertit un composé obtenu répondant à la formule I et salifiable en un sel ou un sel obtenu en un autre sel ou bien, à partir d'un tel sel, on libère le composé et/ou si on le désire, on enrichit un mélange de formes stéréoisomères d'un composé obtenu répondant à la formule I en un isomère optique ayant une configuration spécifique en égard à au moins un centre de chiralité.

15. Les composés obtenus par le procédé de la revendication 14.

**Revendications pour l'Etat contractant: AT**

1. Procédé de préparation d'acides 3-amino-1-benzazépine-2-one-1-alcanoïques de formule générale

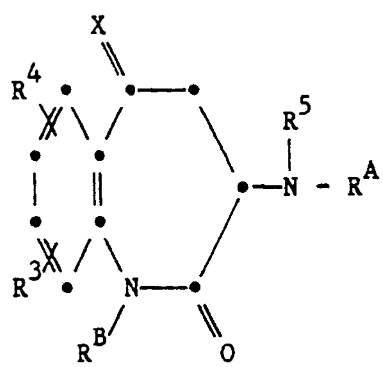

(I),

dans laquelle $R^A$ et $R^B$ représentent des groupes de formules respectives

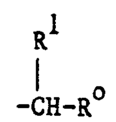

et

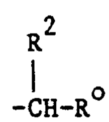

dans lesquelles R° représente un groupe carboxy ou un groupe carboxy ayant subi une modification fonctionnelle; $R^1$ représente l'hydrogène, un groupe alkyle inférieur, aminoalkyle inférieur, aryle, aryl-alkyle inférieur, cycloalkyle, cycloalkyl-alkyle inférieur, acylamino-alkyle inférieur, mono- ou di-(alkyle inférieur)amino-alkyle inférieur, (alkyle inférieur)-thio-alkyle inférieur, carboxy-alkyle inférieur, (carboxy estérifié)-alkyle inférieur, carbamoyl-alkyle inférieur, (carbamoyle substitué à l'azote)-alkyle inférieur, hydroxyalkyle inférieur, (hydroxy éthérifié ou acylé)alkyle inférieur, aryloxy-alkyle inférieur, aryl-(thio-, sulfinyl- ou sulfonyl-)-alkyle inférieur, aryl-(N-alkyle inférieur)-amino-alkyle inférieur ou arylamino-alkyle inférieur; $R^2$ représente l'hydrogène ou un groupe alkyle inférieur; $R^3$ et $R^4$ représentent chacun,

indépendamment l'un de l'autre, l'hydrogène, un groupe alkyle inférieur, alcoxy inférieur, alcanoyloxy inférieur, hydroxy, un halogène ou un groupe trifluorométhyle, ou bien $R^3$ et $R^4$ forment ensemble un groupe alkylène-dioxy inférieur; $R^5$ représente l'hydrogène ou un groupe alkyle inférieur; et X représente un groupe oxo, deux atomes d'hydrogène ou bien un groupe hydroxy ou hydroxy acylé et un atome d'hydrogène; et dans laquelle le noyau carbocyclique peut également être hexahydrogéné ou 6,7,8,9-tétrahydrogéné; sous réserve que lorsque $R^1$ représente l'hydrogène, un groupe alkyle inférieur, aminoalkyle inférieur, aryle, aryl-alkyle inférieur, cycloalkyle ou cycloalkyl-alkyle inférieur, X doit représenter un groupe hydroxy acylé et un atome d'hydrogène; des sels et stéréoisomères de tous ces composés, caractérisé en ce que:

a) dans un composé de formule

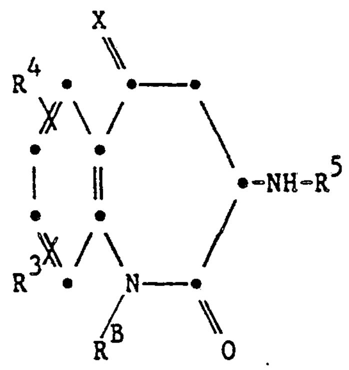

(II),

dans laquelle le noyau carbocyclique peut également être hexahydrogéné ou 6,7,8,9-tétrahydrogéné et X, $R^B$, $R^3$, $R^4$ et $R^5$ ont les significations indiquées précédemment, on introduit $R^A$ par alkylation à l'aide d'un composé de formule

$$R^A—Z \qquad (IIIA)$$

dans laquelle Z représente un groupe hydroxy à l'état d'ester réactif et $R^A$ a les significations indiquées ci-dessus, ou à l'aide d'un composé de formule

$$R^1—CO—R^o \qquad (IV)$$

dans laquelle $R^1$ et $R^o$ ont les significations indiquées ci-dessus, en présence d'un agent réducteur, en protégeant transitoirement des groupes amino primaires et secondaires éventuels et/ou le cas échéant des groupes hydroxy et/ou oxo qui peuvent être présents dans l'un quelconque des substituants X, $R^B$, $R^3$ et $R^4$ et/ou dans l'agent alkylant, ou bien

b) on alkyle un composé de formule

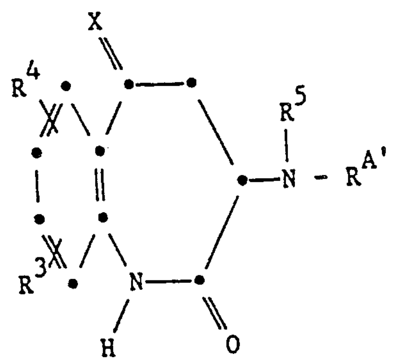

(V),

dans laquelle le noyau carbocyclique peut également être hexahydrogéné ou 6,7,8,9-tétrahydrogéné et X, $R^3$, $R^4$ et $R^5$ ont les significations indiquées ci-dessus, et $R^{A'}$ représente l'hydrogène ou a l'une des significations indiquées précédemment pour $R^A$, à l'aide d'un composé de formule

$$R^B—Z \qquad (IIIB)$$

dans laquelle Z représente un groupe hydroxy à l'état d'ester réactif et $R^B$ a les significations indiquées précédemment, en protégeant transitoirement les groupes amino primaires et secondaires éventuels et/ou le cas échéant des groupes hydroxy et/ou oxo qui peuvent être présents dans l'un quelconque des substituants X, $R^{A'}$, $R^B$, $R^3$ et $R^4$, ou bien

c) on condense un composé de formule

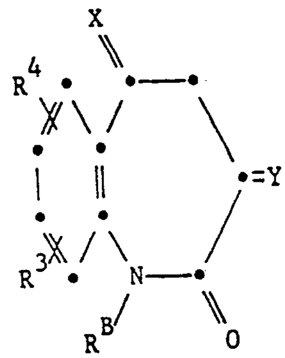

(VI),

dans laquelle le noyau carbocylique peut également être hexahydrogéné ou 6,7,8,9-tétrahydrogéné et Y représente un substituant oxo, dichloro, ou un groupe hydroxy à l'état d'ester réactif Z et un atome d'hydrogène, et X, $R^B$, $R^3$ et $R^4$ ont les significations indiquées ci-dessus, avec une amine de formule

$$R^A\text{—NH—}R^5 \qquad \text{(VII)}$$

dans laquelle $R^A$ et $R^5$ ont les significations indiquées ci-dessus, en opérant en présence d'un agent réducteur lorsque Y représente un substituant oxo ou dichloro, et en protégeant transitoirement le groupe oxo qui peut être présent en tant que substituant X, ou bien

d) dans un composé de formule

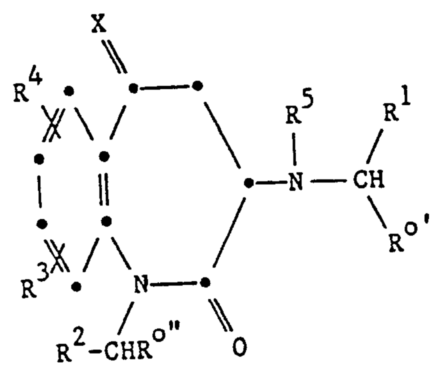

(VIII),

dans laquelle le noyau carbocylique peut également être hexahydrogéné ou 6,7,8,9-tétrahydrogéné et X, $R^1$ à $R^5$ ont les significations indiquées ci-dessus, l'un des symboles $R^{o\prime}$ et $R^{o\prime\prime}$ représente un groupe cyano et l'autre représente un groupe cyano ou a l'une des significations de $R^o$ données précédemment, on soumet le ou les groupes cyano à une solvolyse, ou bien

e) On cyclise un composé de formule

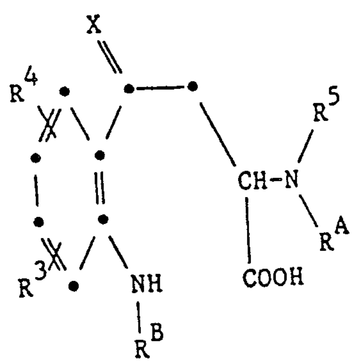

(IX),

dans laquelle le noyau carbocylique peut également être hexahydrogéné ou 3,4,5,6-tétrahydrogéné et X, $R^A$, $R^B$, $R^3$, $R^4$ et $R^5$ ont les significations indiquées ci-dessus, ou un ester d'un tel composé,

f) on traite un composé ayant une structure identique à celle d'un composé de formule I telle que définie ci-dessus sauf qu'il porte une autre double liaison en C—3 ou entre l'atome d'azote et l'atome de carbone voisin à l'intérieur du groupe $R^A$, par un agent réducteur afin de saturer cette double liaison, ou bien

g) pour préparer un composé de formule I telle que définie ci-dessus et dans laquelle X représente un groupe oxo, on condense un composé de formule

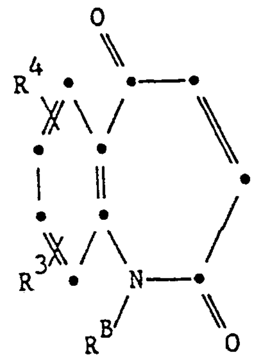

(X),

dans laquelle le noyau carbocyclique peut également être hexahydrogéné ou 6,7,8,9-tétrahydrogéné, et $R^B$, $R^3$ et $R^4$ ont les significations indiquées ci-dessus, avec une amine de formule

$$R^1—NH—R^5 \qquad (VII)$$

dans laquelle $R^A$ et $R^5$ ont les significations indiquées ci-dessus, et si on le désire, on convertit un composé obtenu répondant à la formule I en un autre composé de formule I et/ou, si on le désire, on convertit un composé obtenu répondant à la formule I et salifiable en un sel ou un sel obtenu en un autre sel ou bien, à partir d'un tel sel, on libère le composé et/ou si on le désire, on enrichit un mélange de formes stéréoisomères d'un composé obtenu répondant à la formule I en un isomère optique ayant une configuration spécifique en égard à au moins un centre de chiralité.

2. Procédé selon la revendication 1, caractérisé en ce que l'on prépare des composés de formule

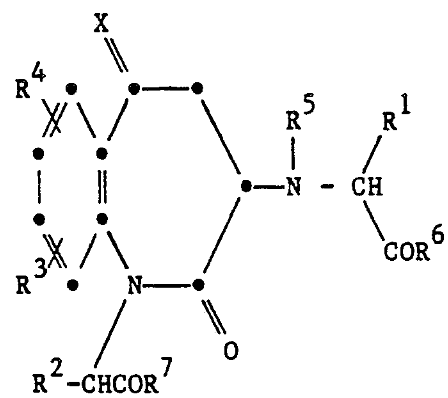

(IA),

dans laquelle le noyau carbocyclique peut également être hexahydrogéné; R représente l'hydrogène, un groupe alkyle inférieur, aminoalkyle inférieur, aryle, aryl-alkyle inférieur, cycloalkyle, cycloalkyl-alkyle inférieur, acylamino-alkyle inférieur, mono- ou di(alkyle inférieur)-amino-alkyle inférieur, (alkyle inférieur)-thio-alkyle inférieur, carboxy-alkyle inférieur, (carboxy estérifié)-alkyle inférieur, carbamoylalkyle inférieur, (carbamoyle substitué à l'azote)alkyle inférieur, hydroxyalkyle inférieur, (hydroxy éthérifié ou acylé)-alkyle inférieur, aryloxy-alkyle inférieur, arylthio-alkyle inférieur, aryl-N-alkyle inférieur)-amino-alkyle inférieur ou arylamino-alkyle inférieur; $R^2$ et $R^5$ représentent l'hydrogène ou des groupes alkyle inférieurs; $R^3$ et $R^4$ représentent l'hydrogène, des groupes alkyle inférieurs, alcoxy inférieurs, alcanoyloxy inférieurs, hydroxy, des halogènes ou des groupes trifluorométhyle ou bien $R^3$ et $R^4$ forment ensemble un groupe alkylène-dioxy inférieur; X représente un groupe oxo, deux atomes d'hydrogène ou un groupe hydroxy ou hydroxy acylé et un atome d'hydrogène (tenu compte des réserves indiquées dans la revendication 1); $R^6$ et $R^7$ représentent chacun, indépendamment l'un de l'autre, un groupe hydroxy, amino, mono- ou di-(alkyle inférieur)-amino, alcoxy inférieur, arylalcoxy inférieur, (alcanoyloxy inférieur)-méthoxy, [amino, mono- ou di-(alkyle inférieur)-amino, carboxy ou (alcoxy inférieur)-carbonyl]-alcoxy inférieur; ou leurs sels.

3. Procédé selon la revendication 1, caractérisé en ce que l'on prépare des composés de formule IA de la revendication 2, dans lesquels $R^1$ représente l'hydrogène, un groupe alkyle inférieur, aminoalkyle inférieur, aryl-(alcoxy inférieur)-carbonylamino-alkyle inférieur ou aryl-alkyle inférieur dans lesquels la partie aryle consiste en un groupe phényle non substitué ou en un groupe phényle mono-substitué par un groupe alkyle inférieur, hydroxy, alcoxy inférieur, alcanoyloxy inférieur, un halogène ou un groupe trifluorométhyle; $R^2$ et $R^5$ représentent l'hydrogène ou des groupes alkyle inférieurs; $R^3$ et $R^4$ représentent

l'hydrogène, des groupes alkyle inférieurs, alcoxy inférieurs, des halogènes ou des groupes trifluorométhyle ou bien $R^3$ et $R^4$ forment ensemble un groupe alkylène-dioxy inférieur; X représente un groupe oxo, deux atomes d'hydrogène ou un groupe hydroxy ou alcanoyloxy inférieur et un atome d'hydrogène (tenu compte des réserves indiquées dans la revendication 1 en sorte que les significations données ici pour X conviennent); $R^6$ et $R^7$ représentent chacun, indépendamment l'un de l'autre, un groupe hydroxy, amino, alcoxy inférieur, phényl-alcoxy inférieur ou (alcoxy inférieur)-carbonyl-alcoxy inférieur; ou leurs sels; ou les composés correspondants dans lesquels le noyau carbocyclique est hexahydrogéné.

4. Procédé selon la revendication 1, caractérisé en ce que l'on prépare des composés de formule IA de la revendication 2, dans lesquels $R^1$ représente l'hydrogène, un groupe alkyle inférieur, oméga-aminoalkyle inférieur, oméga-arylméthoxycarbonylamino-alkyle inférieur ou aryl-alkyle inférieur dans lesquels la partie aryle consiste en un groupe phényle non substitué ou en un groupe phényle monosubstitué par un groupe alkyle inférieur, hydroxy, alcoxy inférieur, alcanoyloxy inférieur, un halogène ou un groupe trifluorométhyle; $R^2$ et $R^5$ représentent l'hydrogène ou des groupes alkyle inférieurs; $R^3$ représente l'hydrogène; $R^4$ représente l'hydrogène, un groupe alkyle inférieur, alcoxy inférieur, un halogène ou un groupe trifluorométhyle; X représente un groupe oxo, deux atomes d'hydrogène ou un groupe hydroxy ou alcanoyloxy inférieur et un atome d'hydrogène (tenu compte des réserves indiquées dans la revendication 1 en sorte que les significations données ici pour X conviennent); $R^6$ et $R^7$ représentent chacun, indépendamment l'un de l'autre, un groupe hydroxy, amino, alcoxy inférieur, phényl-alcoxy inférieur, (alcoxy inférieur)-carbonyl-alcoxy inférieur; ou leurs sels; ou les composés correspondants dans lesquels le noyau carbocyclique est hexahydrogéné.

5. Procédé selon la revendication 1, caractérisé en ce que l'on prépare des composés de formule IA de la revendication 2, dans lesquels $R^1$ représente l'hydrogène, un groupe méthyle, éthyle, isopropyle, oméga-aminopropyle, oméga-aminobutyle, oméga-(benzyloxycarbonylamino)-propyle, oméga-(benzyloxycarbonylamino)butyle, aryl-(méthyle, éthyle, propyle) dans lesquels la partie aryle consiste en un groupe phényle non substitué en un groupe phényle monosubstitué par un groupe méthyle, hydroxy, méthoxy, méthylène-dioxy, acétoxy, le chlore ou un groupe trifluorométhyle; $R^2$ et $R^5$ représentent l'hydrogène ou des groupes méthyle; $R^3$ et $R^4$ représentent l'hydrogène, des groupes méthoxy, méthyle, le chlore ou des groupes trifluorométhyle; X représente un groupe oxo, deux atomes d'hydrogène ou un groupe hydroxy ou acétoxy et un atome d'hydrogène (tenu compte des réserves indiquées dans la revendication 1 en sorte que les définitions données ici pour X conviennent); $R^6$ et $R^7$ représentent chacun, indépendamment l'un de l'autre, un groupe hydroxy, amino, éthoxy, méthoxy, benzyloxy, éthoxycarbonylméthoxy ou pivaloyloxyméthoxy; ou leurs sels; ou les composés correspondants dans lesquels le noyau carbocyclique est hexahydrogéné.

6. Procédé selon la revendication 1, caractérisé en ce que l'on prépare des composés de formule

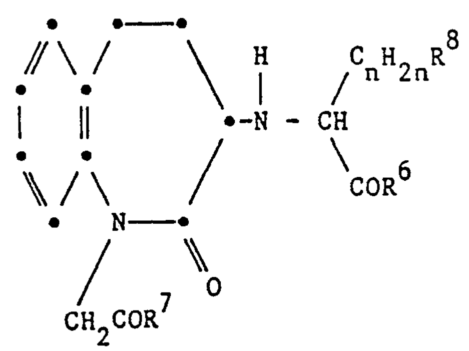

(IB),

dans laquelle n est un nombre entier allant de 1 à 4, $R^8$ représente un groupe benzyloxycarbonylamino; $R^6$ et $R^7$ représentent chacun, indépendamment l'un de l'autre, un groupe hydroxy, alcoxy inférieur contenant jusqu'à 4 atomes de carbone, benzyloxy ou amino, ou leurs sels; ou les composés correspondants dans lesquels le noyau carbocyclique est hexahydrogéné.

7. Procédé selon la revendication 1, caractérisé en ce que l'on prépare des composés de formule

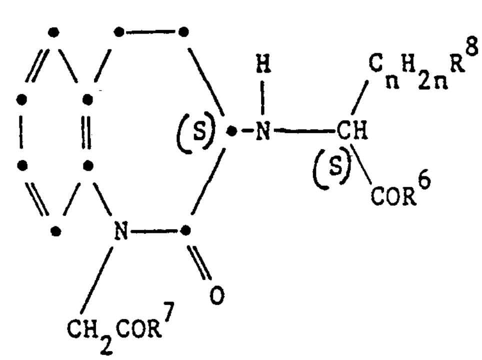

(IC),

dans laquelle S indique la chiralité, n est un nombre entier allant de 1 à 4; $R^8$ représente un groupe benzyloxycarbonylamino; $R^6$ et $R^7$ représentent chacun, indépendamment l'un de l'autre, un groupe hydroxy, alcoxy inférieur contenant jusqu'à 4 atomes de carbone, benzyloxy ou amino; ou leurs sels.

8. Procédé selon la revendication 1, caractérisé en ce que l'on prépare la 5-acétoxy-1-carboxyméthyl-3-(S)-(1-éthoxycarbonyl-3-phénylpropylamino)-2,3,4,5-tétrahydro-1H-1-benzazépine-2-one ou un sel de ce composé acceptable pour l'usage pharmaceutique.

9. Procédé selon la revendication 1, caractérisé en ce que l'on prépare la 3 - [(5 - benzyloxycarbonylamino - 1 - carboxy) - pentylamino] - 1 - carboxyméthyl - 2,3,4,5 - tétrahydro - 1H - 1 - benzazépine - 2 - one ou un sel de ce composé acceptable pour l'usage pharmaceutique.

10. Procédé de préparation d'une composition pharmaceutique, caractérisé en ce que l'on combine une substance active préparée conformément à l'invention selon l'une des revendications 1 à 9, avec un véhicule pharmaceutique.